# EUROPEAN PATENT APPLICATION

(11) **EP 4 667 483 A1**
(43) Date of publication of application: **24.12.2025**
(21) Application number: 24757005.4
(22) Date of filing: 19.02.2024
(51) Int. Cl.: C07K 7/54, A61K 38/10, A61K 47/51, A61K 47/60, A61P 25/00

(54) **HUMAN TRANSFERRIN RECEPTOR-BINDING PEPTIDE**

(30) Priority: 17.02.2023 JP 2023023798
(71) Applicant: JCR Pharmaceuticals Co., Ltd., Ashiya-shi, Hyogo 659-0021 (JP); Peptidream Inc, Kawasaki-ku Kawasaki-shi, Kanagawa 210-0821 (JP)
(72) Inventor: TAKUWA Masatoshi, Kawasaki-shi, Kanagawa 210-0821 (JP); YAMAKOSHI Shuhei, Kawasaki-shi, Kanagawa 210-0821 (JP); JITSUOKA Makoto, Kawasaki-shi, Kanagawa 210-0821 (JP); OHUCHI Masaki, Kawasaki-shi, Kanagawa 210-0821 (JP); TAKAHASHI Kenichi, Kobe-shi, Hyogo 651-2241 (JP); YODEN Eiji, Kobe-shi, Hyogo 651-2241 (JP); HASHIMOTO Hidehiko, Kobe-shi, Hyogo 651-2241 (JP); ONOUCHI Takashi, Kobe-shi, Hyogo 651-2241 (JP); FUJIYAMA Saki, Kobe-shi, Hyogo 651-2241 (JP)
(74) Representative: Mathys & Squire
(86) International application number: PCT/JP2024/005714
(87) International publication number: WO 2024/172166

(57) **Abstract**

[Problem] To provide: a novel peptide that binds to a human transferrin receptor (hTfR); and various uses of the novel peptide.

[Solution] A peptide consisting of an amino acid sequence disclosed in Ala-Val-MeF3C-Val-W7N-Asn-3Py6NH2-F4OMe-Ile-Ile-Arg-Arg-4Py-MeTyr-Cys (SEQ ID NO: 1) or a pharmaceutically acceptable salt thereof; a composite comprising said peptide or pharmaceutically acceptable salt thereof, and a material bound to a linker; a composition comprising said peptide or said composite; and a method for producing a pharmaceutical or diagnostic composition.

## Description

### Technical Field

This invention relates to a peptide capable of binding to a human transferrin receptor (hTfR).

### Background Art

The capillaries that supply the blood to most of the brain tissues, except some regions including the circumventricular organs (the pineal gland, the pituitary gland, the area postrema, etc.), are different from the capillaries present in other tissues, such as muscles, in that the endothelial cells forming the endothelium of the capillaries join together tightly through strong intercellular junctions. Thus, the passive transfer of substances from the blood to the brain is prevented, and although there are some exceptions, substances rarely migrate from the capillaries to the brain, except highly fat-soluble substances or substances with a small molecular weight (200 to 500 daltons or less) and that are electrically neutral at close to a physiological pH. This mechanism, which restricts the exchange of substances between the blood and the tissue fluid of the brain through the capillary endothelium in the brain, is called the blood-brain barrier (BBB). Further, the blood-brain barrier restricts the exchange of substances between not only the blood and the brain, but also the blood and the tissue fluids of the central nervous system, including the brain and the spinal cord. Due to the presence of the blood-brain barrier, most of the cells in the central nervous system can maintain their biochemical homeostasis without being influenced by changes in concentration of substances in the blood, such as hormones and lymphokines.

Various methods have been reported (Patent Literature 1 to 3) for delivering high-molecular-weight substances into the brain across the blood-brain barrier by modifying these substances to have affinity for the transferrin receptor, a membrane protein present on the endothelial cells of brain capillaries. For example, as described in Patent Literature 1, which describes a blood-brain barrier shuttle that has affinity to the transferrin receptor and can bind to the receptor, it is known that a substance that can bind to the transferrin receptor will probably pass through the blood-brain barrier.

### Citation List

### Patent Literature

Patent Literature 1: JP-T-2015-528452
Patent Literature 2: JP-A-H06-228199
Patent Literature 3: WO2016/208695
Patent Literature 4: WO2019/151539

### Non Patent Literature

Non Patent Literature 1: PLOS ONE 2014, 9, e96340

### Summary of Invention

### Technical Problem

One aspect of the invention described in this specification has an object of providing a novel peptide that binds to a human transferrin receptor (hTfR).

Another aspect of the invention has an object of providing various applications of the above-mentioned novel peptides.

### Means for solving the technical problem

A certain invention described in this specification relates to a peptide or a pharmaceutically acceptable salt thereof (hereinafter, these are also sometimes collectively referred to simply as "peptide") that binds to the transferrin receptor.

The peptide is a peptide that has an amino acid sequence set forth in Ala-Val-MeF3C-Val-W7N-Asn-3Py6NH2-F4OMe-Ile-Ile-Arg-Arg-4Py-MeTyr-Cys (SEQ ID NO: 1), or a peptide that includes the amino acid sequence set forth in SEQ ID NO: 1 which includes one or more substitutions selected from the following groups:
(I) substitution of the valine residue at position 2 of SEQ ID NO: 1 for a polar amino acid;
(II) substitution of the MeF3C residue at position 3 of SEQ ID NO: 1 for an N-methyl amino acid having an aromatic ring or a heterocyclic ring in a side chain;
(III) substitution of the W7N residue at position 5 of SEQ ID NO: 1 for tryptophan optionally having a substituent, in which a C on the indole ring is optionally substituted for N, or N-methyltryptophan;
(IV) substitution of the asparagine residue at position 6 of SEQ ID NO: 1 for Nmm;
(V) substitution of the 3Py6NH2 residue at position 7 of SEQ ID NO: 1 for an amino acid having in a side chain an aromatic ring or heterocyclic ring optionally having a substituent;
(VI) substitution of the F4OMe residue at position 8 of SEQ ID NO: 1 for an amino acid having in a side chain an aromatic ring or heterocyclic ring optionally having a substituent;
(VII) substitution of the isoleucine residue at position 9 of SEQ ID NO: 1 for Eva;
(VIII) substitution of the isoleucine residue at position 10 of SEQ ID NO: 1 for any amino acid;
(IX) substitution of the arginine residue at position 11 of SEQ ID NO: 1 for lysine optionally having a substituent in a side chain or glutamine optionally having a substituent in a side chain;
(X) substitution of the arginine residue at position 12 of SEQ ID NO: 1 for lysine, histidine, or glutamine optionally having a substituent in a side chain.
(XI) substitution of the 4Py residue at position 13 of SEQ ID NO: 1 for an amino acid having in a side chain an aromatic ring or heterocyclic ring optionally having a substituent, or glutamine optionally having a substituent in a side chain; and
(XII) substitution of the MeTyr residue at position 14 of SEQ ID NO: 1 for an N-methyl amino acid having in a side chain an aromatic ring or heterocyclic ring optionally having a substituent.

### Advantageous Effects of Invention

According to the invention described in this specification, as proved in the Examples, it is possible to provide a peptide that binds to the human transferrin receptor (hTfR) and the like.

### Description of Embodiments

Hereinafter, embodiments of the present invention will be described using the drawings. The present invention is not limited to the embodiments described below and includes modifications made as appropriate based on the following embodiments within a scope obvious to those skilled in the art.

One aspect of the invention described in this specification relates to a peptide that binds to a transferrin receptor.

### Transferrin receptor

Transferrin receptor refers to a receptor that binds to transferrin, a protein present in blood plasma that binds to an iron ion, and that has a function of taking the transferrin into cells. The transferrin receptor is expressed on the surface of various cells, such as reticulocytes, trophoblasts of the placenta, and lymphocytes, and it is suggested that the transferrin receptor is particularly expressed in tumor cells. Further, since the transferrin receptor has a property of triggering endocytosis of cells upon stimulation due to binding to iron ions in the blood plasma, studies have been conducted on allowing substances to pass through the BBB using an antibody or the like that binds to the transferrin receptor as a DDS. Further, although transferrin receptors of type I and type II are known, the transferrin receptor in the present invention is preferably the type I transferrin receptor (Gene ID: 7037). In the specification of the present application, a human-derived transferrin receptor is referred to as human TfR, hTfR, or simply TfR, unless otherwise specified.

### Peptides binding to transferrin receptor

Binding to the transferrin receptor (also referred to as having binding activity or having affinity) means specifical binding to the transferrin receptor.

Affinity is represented by an Equilibrium constant (KD) of dissociation between the transferrin receptor and a binding peptide. The Equilibrium constant (KD) is an index representing a binding strength between the transferrin receptor and an antigen-binding site on the binding peptide. As a KD value becomes smaller, the binding strength between the transferrin receptor and the binding peptide becomes stronger (alternately, the affinity can also be represented as an affinity constant (KA) defined by 1/KD). As is obvious to those skilled in the art, the affinity can be determined by a publicly known method (e.g., based on the further disclosure in the present specification) in accordance with particular target antigens. The binding activity is an index for the binding strength between the transferrin receptor and the binding peptide. The binding activity is related to both the affinity between the transferrin receptor and a binding site thereof on the binding peptide and a number of the relevant binding sites present on the binding molecule.

The specific binding between the transferrin receptor and the binding peptide can be determined by any appropriate known method, including, for example, surface plasmon resonance (SPR) assays described in this specification, Scatchard analysis, and/or competitive binding assays such as radioimmunoassay (RIA), enzyme immunoassay (EIA), and sandwich competition assays, as well as various other variants known per se in the art. Preferably, the affinity (KD) between the peptide of the present invention and the transferrin receptor is less than 100 nM, more preferably less than 50 nM, even more preferably less than 20 nM, and still more preferably less than 10 nM. However, it is not limited thereto, and may be in the range of about 10⁻⁵ M to about 10⁻⁹ M, or alternatively, about 10⁻⁷ M or less, for example from about 10⁻⁷ M to 10⁻¹³ M, or about 10⁻⁹ M to 10⁻¹³ M. In this specification, peptides having binding ability to the transferrin receptor within the above range, ranging from low to high affinity, are provided.

### Peptides

A peptide refers to a structure of multiple consecutive amino acids, with polypeptides and proteins also included in that definition. Note that amino acids in the present application not only include naturally present amino acids (natural amino acids) taken into a peptide chain when mRNA is translated in a cell, but also amino acids not present in nature (non-natural amino acids) that can constitute a part of a peptide chain through a peptide bond. The amino acid may be artificially synthesized, or it may be one that is present in the nature world.

Further, in the present application, peptides with a circular part formed by cyclization after synthesis (also referred to as a cyclic peptide), peptides obtained by further chemically modifying the peptide are also included in the peptides of the present invention.

In the present specification, cyclic peptide refers to a peptide in which two amino acids separated from each other via one or more amino acid residues are bound to each other, causing the amino acid sequence to be wholly or partially cyclic. Note that a type of bonding between the two amino acids is not particularly limited, and examples of the cyclic peptide include those in which a cyclic structure is formed by an amide bond between a carboxyl group of one amino acid and an amino group of another amino acid, a thioether bond between a carboxyl group of one amino acid and a thiol group of another amino acid, a thiol bond between a thiol group of one amino acid and a thiol group of another amino acid, or lactam ring formation or a macrocyclization reaction, those having a lasso peptide-like structure, and the like. However, when the two amino acids are bonded to each other by an amide bond, the amide bond is not limited to the one formed by the bond of a carboxyl group of one amino acid and an amino group of another amino acid, but may be bound by an amide bond resulting from a synthesis reaction. The same also applies to other types of bonds. That is, in the present application, a cyclic peptide may have only a portion forming a cyclic structure and may include a linear segment. In addition, the cyclic peptide may have a complex cyclic structure, such as a bicyclic structure in which two amino acids within a single cyclic structure are further connected.

In this specification, some amino acids may be modified for the purpose of peptide cyclization. Such partially modified amino acids are also included in the amino acids of the present application. For example, a case where a chloroacetyl group is added to an amino acid positioned at the N-terminus and binds to a cysteine residue within the peptide to achieve cyclization can be cited. Various (natural/non-natural) amino acids with an added chloroacetyl group are also included in the amino acids of the present application.

Non-natural amino acids refer to compounds that have the characteristics of amino acids, other than the natural amino acids. Examples of the non-natural amino acids include, but are not limited to, β-amino acid, γ-amino acid, and L-amino acid; D-amino acid (also referred to as D-configuration amino acid); a chemically modified amino acid such as an amino acid variant or an amino acid derivative; an amino acid that is not a constituent material of protein in vivo such as norleucine, β-alanine, or ornithine; and the like. N-methyl amino acid, N-ethyl amino acid, D-amino acid, a histidine-like amino acid, an amino acid having a structure in which additional methylene or an aromatic ring is added to a side chain, an amino acid derivative having a structure in which a carboxylic acid functional group in the side chain is replaced with a sulfonic acid group, and the like are also included.

Examples of the non-natural amino acids and abbreviations thereof are indicated in the present specification as follows. The CAS reference number or supplier company name is indicated in parentheses, and newly synthesized amino acids are indicated by the synthesis example number. Note that while the CAS number represents the non-natural amino acid alone or the non-natural amino acid to which a protective group is bound, special amino acids are not limited to these, and examples of the special amino acids include those having a structure in which one or a plurality of hydrogen atoms in these molecules are replaced with an alkyl group. When the hydrogen atom is replaced with an alkyl group, the alkyl group is preferably a methyl group or an ethyl group, and more preferably a methyl group. Note that in the present specification, "Me" or "N-Me-" denoted at the front of an amino acid name represents N-methyl amino acid, unless otherwise specified. For example, N-methylated amino acid of alanine (Ala or A) is represented by MeAla, N-MeAla, MeA, or N-MeA. Further, a single letter code of an amino acid designation with a "d" denoted in front represents D-amino acid. For example, D-amino acid of alanine (Ala or A) is represented by "da". Reagents without a CAS number or supplier listed can be purchased as general reagents. Note that the following amino acids can be used for peptide synthesis by protecting their alpha amino groups with Fmoc using a known method.
W7N: L-7-azatryptophan (CAS No. 49758-35-2)
KCOpipzaa: N⁶-(4-(carboxymethyl)piperazin-1-carbonyl) -L-lysine (Kishida Chemical Co.)
4Py: L-4-pyridylalanine (CAS No. 37535-49-2)
PeG: N-phenethylglycine (CAS No. 7738-38-7)
3Py: 3- (3-pyridyl) -L-alanine (CAS No. 64090-98-8)
3Py6NH₂: (S)-2-amino-3-(6-aminopyridin-3-yl) propanoic acid (CAS No.1269968-61-7)
A4paa: (S)-2-amino-3-(1-(carboxymethyl)piperidin-4-yl) propanoic acid (Kishida Chemical Co.)
dkCOmegenine: N⁶-(methyl(((2S,3R,4R,5R) -2,3,4,5,6-pentahydroxyhexyl) carbamoyl)) - D-lysine (see Synthetic Example 1-7)
F3COO: (S)-3-(2-amino-2-carboxyethyl) benzoic acid (CAS No. 13861-02-4)
F3COO (allyl): (S)-3-((allyloxy)carbonyl) phenyl) -2-aminopropanoic acid (see Synthetic Example 1-1)
F4aao: (S)-2-amino-3-(4-(carboxymethoxy) phenyl) propanoic acid (CAS No.24558-63-2)
F4COO (allyl): (S)-3-((allyloxy) carbonyl) phenyl) -2-aminopropanoic acid (see Synthetic Example 1-2)
F4OMe: (S)-2-amino-3-(4-methoxyphenyl) propanoic acid (CAS No. 6230-11-1) s
KdMe: N⁶, N⁶-dimethyl-L-lysine (CAS No. 2259-86-1)
Kmor: (S)-2-amino-6-morpholinohexanoic acid (CAS No. 960135-14-2; see Synthetic Example 1-10)
KN₃: N⁶-diazo-L-lysine (CAS No. 159610-92-1)
Me3Py: (S)-2-(methylamino)-3-(pyridin-3-yl) propanoic acid (CAS No. 2651172-69-7)
Me4Py: (S)-2-(methylamino)-3-(pyridin-4-yl) propanoic acid
MeA: methyl-L-alanine (CAS No. 3913-67-5)
MeF: methyl-L-phenylalanine (CAS No. 2566-30-5)
MeF3C: (S)-3-(3-chlorophenyl) -2-(methylamino)propanoic acid (CAS No.2255324-91-3)
MeF3COO: (S)-3-(2-carboxy-2-(methylamino) ethyl) benzoic acid (CAS No.1499826-56-0)
MeF3COO (allyl): (S)-3-((allyloxy)carbonyl) phenyl-2-(methylamino)propanoic acid (see Synthetic Example1-3)
MeF4F: (S)-3-(4-fluorophenyl) -2-(methylamino)propanoic acid (CAS No.347851-71-2) MeR: methyl-L-arginine (CAS No. 2480-28-6)
MeW: methyl-L-tryptophan (CAS No. 526-31-8)
MeY: methyl-L-tyrosine (CAS No. 537-49-5)
QhEt: N⁵-(2-hydroxyethyl) -L-glutamine (CAS No.2650-74-0; see Synthetic Example 1-4)
Qpipzaa: (S)-2-amino-5-(4-(carboxymethyl)piperazin-1-yl) -5-oxopentanoic acid (see Synthetic Example 1-5)
W1aa: 1-(carboxymethyl)-L-tryptophan (CAS No. 773823-50-0)
W1aa(allyl): 1-(2-((allyloxy)carbonyl)) -L-tryptophan (see Synthetic Example 1-6)
W5C: (S)-2-amino-3-(5-chloro-1H-indol-3-yl) propanoic acid (CAS No. 52448-15-4)
Ndm: N⁴, N⁴-dimethyl-L-asparagine (CAS No. 62937-43-3)
F4C: N-α-chloroacetyl-4-chloro-L-phenylalanine (CAS No. 14173-39-8)
dr: D-arginine
dk: D-lysine
Aib: α-methylalanine (CAS No. 62-57-7)
Ahp/Alahp: (S)-2-aminoheptanoic acid (CAS No. 1115-90-8)
MeF3COO (PEG4Me): (S)-3-(3-((2,5,8,11-tetraoxatridecan-13-yl) carbamoyl) phenyl) -2-(methylamino)propanoic acid (see Synthetic Example 1-11)
MeF4COO (PEG4Me): (S)-3-(4-((2,5,8,11-tetraoxatridecan-13-yl) carbamoyl) phenyl) -2-(methylamino)propanoic acid (see Synthetic Example 1-8)
F3COO (PEG4Me): (S)-3-(3-((2,5,8,11-tetraoxatridecan-13-yl) carbamoyl) phenyl) -2-aminopropanoic acid (see Synthetic Example 1-12)
K (Mecar): N⁶-(methoxycarbonyl)-L-lysine (CAS No.74761-45-8)
K (MePEG4c): (S)-20-amino-14-oxo-2,5,8,11-tetraoxa-15-azahenicosanoic acid (CAS No.1188295-19-3)
E (PEG1Me): N⁵-(2-methoxyethyl) -L-glutamine (CAS No. 132432-96-3)
E (NHdPEG1Me): N⁵-(1,3-dimethoxypropan-2-yl) -L-glutamine
E (PEG4Me): (S)-18-amino-15-oxo-2,5,8,11-tetraoxa-14-azanonadecan-19-oic acid (CAS No. 2234872-12-7)
E (PEG8Me):
   (S)-30-amino-27-oxo-2,5,8,11,14,17,20,23-octaoxa-26-azahentriacontan-31-oic acid E (Glucamine):
   N⁵-[(2S,3R,4R,5R)-2,3,4,5,6-pentahydroxyhexyl]-L-glutamine (CAS No. 1956384-48-7)
   MeF4COO (PEG8Me): (S)-3-(4-((2,5,8,11,14,17,20,23-octaoxapentacosan-25-yl) carbamoyl) phenyl) -2-(methylamino)propanoic acid (see Synthetic Example1-13)
   F3COO (PEG8Me): (S)-3-(3-((2,5,8,11,14,17,20,23-octaoxapentacosan-25-yl) carbamoyl) phenyl) -2-aminopropanoic acid (see Synthetic Example 1-9)
   F4aao (PEG8Me): (S)-2-amino-3-(4-((27-oxo-2,5,8,11,14,17,20,23-octaoxa-26-aza-octacosan-28-yl) oxy) phenyl) propanoic acid
   aI: L-allo-isoleucine (CAS No.1509-34-8)
   da: D-alanine
   de: D-glutamic acid
   ds: D-serine
   PEG4c or PEG3: 1-amino-3,6,9,12-tetraoxapentadecan-15-oic acid (CAS No.663921-15-1)
   PEG8c: 1-amino-3,6,9,12,15,18,21,24-octaoxaheptacontan-27-oic acid (CAS No.756526-04-2)
   PEG8Me: 2,5,8,11-tetraoxapentacosan-25-amine (CAS No.869718-81-0)
   PEG12c/PEG11/PEG12: 1-amino-3,6,...,36-dodecaoxanonatriacontan-39-oic acid (CAS No.1415408-69-3)
   MePEG4c: 2,5,8,11-tetraoxatetradecan-14-oic acid (CAS No.67319-28-2)
   PEG1Me: 2-methoxyethan-1-amine (CAS No.109-85-3)
   PEG4Me: 2,5,8,11-tetraoxotridecan-13-amine (CAS No.85030-56-4)
   NHdPEG1Me: 1,3-dimethoxypropan-2-amine (CAS No.78531-29-0)
   BCNOCO: ((1R,8S,9s) -bicyclo[6.1.0] non-4-en-9-yl) methyl(2,5-dioxopyrrolidin-1-yl) carboxylate (CAS No. 1426827-79-3)
   K (BCNOCO): N⁶-(methoxycarbonyl((2S,3R,4R,5R) -2,3,4,5,6-pentahydroxyhexyl)) -((1R,8S,9s) -bicyclo [6.1.0] non-4-en-9-yl) methoxycarbonyl) -L-lysine
   C (AcNMe): S-(2-(methylamino)-2-oxoethyl) -L-cysteine
   Cit: L-citrulline (CAS No.372-75-8)
   K (Ac): N⁶-acetyl-L-lysine (CAS No.692-04-6)
   K (C5Mal): N⁶-(6-(2,5-dioxo-2,5-dihydro-1H-pyrrol-1-yl) hexanoyl) -L-lysine
   PEG24c: 1-amino-3,6,9,...,72-tetraoxapentadecan-75-oic acid (CAS No. 2563873-76-5) K(Maleimide): N⁶-(4-((2,5-dioxo-2,5-dihydro-1H-pyrrol-1-yl) methyl) cyclohexan-1-carbonyl) -L-lysine
   F4aao (Glucamine): (S)-2-amino-3-(4-(2-oxo-((2S,3R,4R,5R) -2,3,4,5,6-pentahydroxyhexylamino) ethoxy) phenyl) propanoic acid
   F4aao (pipzaa): (S)-2-amino-3-(4-(2-((4-(carboxymethyl)piperazin-1-yl) -2-oxoethoxy) phenyl) propanoic acid
   YaeCOpipzaa: (S)-2-amino-3-(4-(2-((4-(carboxymethyl)piperazin-1-yl) -carboxyamido) ethoxy) phenyl) propanoic acid
   Har: N⁶-carbamimidoyl-L-lysine (CAS No.156-86-5)
   W1EtOH: 1-(2-hydroxyethyl) -L-tryptophan
   Nmm: N⁴-methyl-L-asparagine (CAS No.7175-34-0)
   Eva: (S)-2-amino-3-ethylpentanoic acid (CAS No.14328-49-5)
   Mor: (S)-morpholine-3-carboxylic acid (CAS No. 106825-79-0)
   Hpr: (S)-piperidine-2-carboxylic acid (CAS No.3105-95-1)
   Y26dF: (S)-2-amino-3-(2,6-difluoro-4-hydroxyphenyl) propanoic acid (CAS No.182756-57-6)
   W1Me: 1-methyl-L-tryptophan (CAS No. 21339-55-9)
   MeF4COO: (S)-4-(2-carboxy-2-(methylamino)ethyl) benzoic acid
   Y3Me: (S)-2-amino-3-(4-hydroxy-3-methylphenyl) propanoic acid (CAS No.17028-03-4)
   3Imp: (S)-2-amino-3-(imidazo[1,2-a] pyridin-3-yl) propanoic acid (CAS No.2276942-95-9)
   3Py6OMe: (S)-2-amino-3-(6-methoxypyridin-3-yl) propanoic acid (CAS No.1270178-24-9)
   3Py6NHaa: (S)-2-amino-3-(6-((carboxymethyl)amino) pyridin-3-yl) propanoic acid
   MeF4C: (S)-3-(4-chlorophenyl) -2-(methylamino)propanoic acid (CAS No.347851-70-1)
   MeF4OMe: (S)-3-(4-methoxyphenyl) -2-(methylamino)propanoic acid (CAS No.52939-33-0)
   MeF3OMe: (S)-3-(3-methoxyphenyl) -2-(methylamino)propanoic acid
   3Py6Me: (S)-2-amino-3-(6-methylpyridin-3-yl) propanoic acid (CAS No.1270288-73-7)
   4Py2Me: (S)-2-amino-3-(2-methylpyridin-4-yl) propanoic acid (CAS No.1269969-41-6)
   4Py2OMe: (S)-2-amino-3-(2-methoxypyridin-4-yl) propanoic acid (CAS No. 1269920-00-4)
   4Py2NH₂: (S)-2-amino-3-(2-aminopyridin-4-yl) propanoic acid (CAS No.1269969-46-1)
   E(PEG8c):
      (S)-32-amino-29-oxo-4,7,10,13,16,19,22,25-octaoxa-28-azatriacontan-dioxoic acid
      F3COO(PEG8c): (S)-1-(3-(2-amino-2-carboxyethyl) phenyl) -1-oxo-5,8,11,14,17,20,23,26-octaoxa-2-azanonacosan-29-oic acid
      E(PEG4c): (S)-20-amino-17-oxo-4,7,10,13-tetraoxa-16-azaheptadecan-19-oic acid
      F3COO(PEG4c) or F3CONPEG4c: (S)-1-(3-(2-amino-2-carboxyethyl) phenyl) -1-oxo-5,8,11,14-tetraoxa-2-azaheptadecan-17-oic acid
      E (apa): N⁵-(3-aminopropyl) -L-glutamine
      MeF4COO(PEG4c):(S)-1-(4-(2-amino-2-carboxyethyl)
      phenyl-1-oxo-5,8,11,14-tetraoxa-2-azaheptadecan-17-oic acid
      Note that newly synthesized amino acids are useful as they have the potential to add new functions to various peptides when various peptide derivatives are produced.

The peptide of the present invention is preferably the following peptide A.

The peptide having an amino acid sequence set forth in Ala-Val-MeF3C-Val-W7N-Asn-3Py6NH2-F4OMe-Ile-Ile-Arg-Arg-4Py-MeTyr-Cys (SEQ ID NO: 1), or a peptide that includes the amino acid sequence set forth in SEQ ID NO: 1 which includes one or more substitutions selected from the following groups:
(I) substitution of the valine residue at position 2 of SEQ ID NO: 1 for a polar amino acid;
(II) substitution of the MeF3C residue at position 3 of SEQ ID NO: 1 for an N-methyl amino acid having an aromatic ring or a heterocyclic ring in a side chain;
(III) substitution of the W7N residue at position 5 of SEQ ID NO: 1 for tryptophan optionally having a substituent, in which a C on the indole ring is optionally substituted for N, or N-methyltryptophan;
(IV) substitution of the asparagine residue at position 6 of SEQ ID NO: 1 for Nmm;
(V) substitution of the 3Py6NH2 residue at position 7 of SEQ ID NO: 1 for an amino acid having in a side chain an aromatic ring or heterocyclic ring optionally having a substituent;
(VI) substitution of the F4OMe residue at position 8 of SEQ ID NO: 1 for an amino acid having in a side chain an aromatic ring or heterocyclic ring optionally having a substituent;
(VII) substitution of the isoleucine residue at position 9 of SEQ ID NO: 1 for Eva;
(VIII) substitution of the isoleucine residue at position 10 of SEQ ID NO: 1 for any amino acid;
(IX) substitution of the arginine residue at position 11 of SEQ ID NO: 1 for lysine optionally having a substituent in a side chain or glutamine optionally having a substituent in a side chain;
(X) substitution of the arginine residue at position 12 of SEQ ID NO: 1 for lysine, histidine, or glutamine optionally having a substituent in a side chain.
(XI) substitution of the 4Py residue at position 13 of SEQ ID NO: 1 for an amino acid having in a side chain an aromatic ring or heterocyclic ring optionally having a substituent, or glutamine optionally having a substituent in a side chain; and
(XII) substitution of the MeTyr residue at position 14 of SEQ ID NO: 1 for an N-methyl amino acid having in a side chain an aromatic ring or heterocyclic ring optionally having a substituent.

Each of the above options (I) through (XII) may be selected in any combination.

The peptide of the present invention may also be a peptide having an amino acid sequence in which 1 to 3 amino acid residues are substituted, deleted, added, or inserted from any of the above sequences. In these cases, the peptide is preferably a peptide that has the ability to bind to hTfR. The peptide may be the peptide itself, or may be a pharmaceutically acceptable salt (e.g., sodium salt or potassium salt) thereof.

### Conservative amino acid substitution

Conservative amino acid substitution refers to substitution with a functionally Equivalent or similar amino acid. The conservative amino acid substitution in a peptide causes a static change in the amino acid sequence of the peptide. For example, one or two or more amino acids having a same polarity act functionally Equivalently in causing a static change in the amino acid sequence of the peptide. In general, a substitution within a certain group can be considered to be conservative in terms of structure and function. However, as is obvious to those skilled in the art, a role of a particular amino acid residue can be determined in the context of a three-dimensional structure of a molecule containing the particular amino acid. For example, a cysteine residue can take an oxidized (disulfide) form having lower polarity than a reduced (thiol) form. A long aliphatic moiety in an arginine side chain can constitute structurally and functionally important features. Further, an aromatic ring-containing side chain (tryptophan, tyrosine, and phenylalanine) can contribute to ion-aromatic interaction or cation-pi interaction. In such a case, substitution of the amino acids having these side chains with amino acids belonging to an acidic or non-polar group can be structurally and functionally conservative. Residues of proline, glycine, cysteine (disulfide form), and the like may have a direct effect on a three-dimensional structure of the main chain, which often makes it not possible to substitute these amino acids without causing structural distortion.

The conservative amino acid substitution includes, as shown below, specific substitution based on side chain similarity (L. Lehninger, Biochemistry, 2nd edition, pp. 73-75, Worth Publisher, New York (1975)) and typical substitution.

Preferable examples of this peptide include the amino acid sequence having one or more substitutions selected from the following groups:
(1) Hydrophobic (also called nonpolar) amino acids: Amino acids that exhibit hydrophobic (also called nonpolar) and include alanine ("Ala" or simply "A"), glycine ("Gly" or simply "G"), valine ("Val" or simply "V"), leucine ("Leu" or simply "L"), isoleucine ("Ie" or simply "I"), proline ("Pro" or simply "P"), phenylalanine ("Phe" or simply "F"), tryptophan ("Trp" or simply "W"), tyrosine ("Tyr" or simply "Y"), methionine ("Met" or simply "M").

The hydrophobic amino acids can be further divided into the following groups.

Aliphatic amino acids: Amino acids having a fatty acid or hydrogen in a side chain, including Ala, Gly, Val, Ile and Leu.

Aliphatic and branched-chain amino acids: Amino acids having a branched fatty acid in a side chain, including Val, Ile and Leu.

Aromatic amino acids: Amino acids having an aromatic ring in a side chain, including Trp, Tyr and Phe.

(2) Hydrophilic (also called polar) amino acids: Amino acids that exhibit hydrophilicity (polarity) and include serine ("Ser" or simply "S"), threonine ("Thr" or simply "T"), cysteine ("Cys" or simply "C"), asparagine ("Asn" or simply "N"), glutamine ("GIn" or simply "Q"), aspartic acid ("Asp" or simply "D"), glutamic acid ("Glu" or simply "E"), lysine ("Lys" or simply "K"), arginine ("Arg" or simply "R"), and histidine ("His" or simply "H").

The hydrophilic amino acids can be further divided into the following groups.

Acidic amino acids: Amino acids having an acidic side chain, including Asp and Glu.

Basic amino acids: Amino acids having a basic side chain, including Lys, Arg and His.

Neutral amino acids: Amino acids having a neutral side chain, including Ser, Thr, Asn, GIn and Cys.

Gly and Pro can be divided into "amino acids affecting the direction of the main chain", and amino acids containing a sulfur molecule in a side chain, Cys and Met, can also be divided into "sulfur-containing amino acids".

Examples of groups having an aromatic in a side chain include Trp, Tyr and Phe.

As used herein, "amino acid" includes not only natural amino acids but also unnatural amino acids. Unnatural amino acids include, for example, N-alkyl amino acids in which a natural amino acid described above is N-alkylated; and those modified with lower alkyl groups (for example, of C1 to C5, preferably C1 to C3, and more preferably Cl) in which the nitrogen forming a peptide bond is branched or not branched. Among the N-alkyl amino acids, N-ethyl amino acid, N-butyl amino acid, or N-methyl amino acid is preferable, and N-methyl amino acid is more preferable. C4 or more alkyl group means a linear or branched alkyl group having 4 or more carbons. Examples of C4 or more alkyl groups are C4-10 alkyl groups, which may be C4-6 alkyl groups or C6-10 alkyl groups. Further, unnatural amino acids also include D-type amino acids (also referred to as D-amino acids), chemically modified amino acids such as β-amino acids, 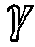 -amino acids, amino acid variants, amino acid derivatives, and the like; amino acids that are not constituent materials for proteins in vivo, such as norleucine, ornithine, and the like; and the like. Also included are amino acids to which a functional group is further added to the side chain of a natural amino acid or substituted for another functional group (for example, an amino acid having a substitution or an addition in a part such as an arylene group, an alkylene group, and the like of the side chain; an amino acid wherein the arylene group, alkylene group or the alkyl group of the side chain has an increased C-number; an amino acid having a substitution in the aromatic ring of the side chain; an amino acid derivative with the structure in which the carboxylic acid functional group in the side chain is replaced by a sulfonic acid group; a heterocyclic or condensed cyclic amino acid; or the like).

Note that by adding or substituting a structure such as a functional group in the side chain of a natural amino acid, a property different from that of the natural amino acid may be imparted. Namely, the group described above, obtained by dividing natural amino acids based on the properties of their common side chains, may include unnatural amino acids having the same side chain property. For example, N-methylalanine (MeA), which is an amino acid in which the nitrogen atom in the main chain of alanine belonging to an aliphatic amino acid is methylated, is an unnatural amino acid, but it may be classified as a hydrophobic amino acid because it exhibits a hydrophobic (nonpolar) property. Thus, an unnatural amino acid exhibiting the same side chain property as that of a certain amino acid may also be included as the subject of a conservative amino acid substitution.

As used herein, "in a non-limiting manner" and "in one aspect" may be used interchangeably.

Non-limiting examples of conservative amino acid substitutions of unnatural amino acids include the following.
MeA: N-methylated amino acid, hydrophobic amino acid group and aliphatic amino acid group
MeF: N-methylated amino acid, hydrophobic amino acid group and aromatic amino acid group
MeW: N-methylated amino acid, hydrophobic amino acid group and aromatic amino acid group
MeY: N-methylated amino acid, hydrophobic amino acid group and aromatic amino acid group
MeR: N-methylated amino acid, hydrophilic amino acid group and basic amino acid group
da: D-type amino acid, hydrophobic amino acid group and aliphatic amino acid group
de: D-type amino acids, hydrophilic amino acid group and acidic amino acid group
dr: D-type amino acid, hydrophilic amino acid group and basic amino acid group
ds: D-type amino acid, hydrophilic amino acid group and neutral amino acid group
4Py: Basic amino acid group and aromatic amino acid group
3Py: Basic amino acid group and aromatic amino acid group
F4C: Hydrophobic amino acid group and aromatic amino acid group
F40Me: Hydrophobic amino acid group and aromatic amino acid group
F4aao: Hydrophobic amino acid group and aromatic amino acid group
F3COO: Hydrophobic amino acid group and aromatic amino acid group
MeF3C: N-methylated amino acid, hydrophobic amino acid group and aromatic amino acid group
MeF4F: N-methylated amino acid, hydrophobic amino acid group and aromatic amino acid group
Me3Py: N-methylated amino acid, hydrophobic amino acid group and aromatic amino acid group
Me4Py: N-methylated amino acid, hydrophobic amino acid group and aromatic amino acid group
MeF3COO: N-methylated amino acid, hydrophobic amino acid group and aromatic amino acid group
MeF4COO: N-methylated amino acid, hydrophobic amino acid group and aromatic amino acid group
W5C: Hydrophobic amino acid group and aromatic amino acid group
W1aa: Hydrophobic amino acid group and aromatic amino acid group
W7N: Basic amino acid group and aromatic amino acid group
W5C: Hydrophobic amino acid group and aromatic amino acid group
3Py6NH2: Basic amino acid group and aromatic amino acid group
all: Hydrophobic amino acid group and aliphatic amino acid group
Aib: Hydrophobic amino acid group and aliphatic amino acid group
Ahp: Hydrophobic amino acid group and aliphatic amino acid group
A4paa: Hydrophobic amino acid group and aliphatic amino acid group
Kmor: Hydrophilic amino acid group
Kdme: Hydrophilic amino acid group
KCOpipzaa: Hydrophilic amino acid group
QhEt: Hydrophilic amino acid group
W1EtOH: Hydrophobic amino acid group and aromatic amino acid group
W1Me: Hydrophobic amino acid group and aromatic amino acid group
3Imp: Basic amino acid group and aromatic amino acid group
Nmm: Hydrophilic amino acid group
Y26dF: Hydrophobic amino acid group and aromatic amino acid group
3Py6NHaa: Hydrophilic amino acid group and aromatic amino acid group
Y3Me: Hydrophobic amino acid group and aromatic amino acid group
F4aao (Glucamine): Hydrophilic amino acid group and aromatic amino acid group
F4aao (pipzaa): Hydrophilic amino acid group and aromatic amino acid group
YaeCOpipzaa: Hydrophilic amino acid group and aromatic amino acid group
3Py6OMe: Basic amino acid group and aromatic amino acid group
Eva: Hydrophobic group and aliphatic/branched amino acid group
Har: Hydrophilic amino acid group
Mor: Hydrophilic amino acid group
Hpr: Hydrophobic amino acid group
E (Glucamine): Hydrophilic amino acid group
E (PEG8Me): Hydrophilic amino acid group
QPEG8Me: Hydrophilic amino acid group
E (PEG8c): Hydrophilic amino acid group
E (PEG4c): Hydrophilic amino acid group
F3CONPEG4c: Hydrophilic amino acid group and aromatic amino acid group
3Py6Me: Basic amino acid group and aromatic amino acid group
4Py2Me: Basic amino acid group and aromatic amino acid group
4Py2OMe: Basic amino acid group and aromatic amino acid group
4Py2NH2: Basic amino acid group and aromatic amino acid group
F3COO (PEG4c): Hydrophilic amino acid group and aromatic amino acid group
F3COO (PEG8c): Hydrophilic amino acid group and aromatic amino acid group
MeF4C: N-methylated amino acid, hydrophobic amino acid group and aromatic amino acid group
MeF4OMe: N-methylated amino acid, hydrophobic amino acid group and aromatic amino acid group
MeF3OMe: N-methylated amino acid, hydrophobic amino acid group and aromatic amino acid group
F4aao (PEG8Me): Hydrophilic amino acid group and aromatic amino acid group
F3COO (PEG8Me): Hydrophilic amino acid group and aromatic amino acid group
D-amino acids may be classified as D-amino acids, but they may also be classified according to the properties of their side chains, and N-methyl amino acids may be classified as N-alkyl amino acids and may also be classified according to the property of the side chain of the original amino acid that has not undergone N-methylation.
As used herein, a "positively charged amino acid" may be any natural or unnatural amino acid, and examples of natural positively charged amino acids include lysine, arginine and histidine. The "positively charged amino acid" may be any amino acid having a positive charge (e.g. NH³⁺) and may have various substituents.

Preferable examples of this peptide include the amino acid sequence having one or more substitutions selected from the following groups:
(I) the residue at position 1 of SEQ ID NO: 1 is an alanine residue;
(II) the residue at position 4 of SEQ ID NO: 1 is a valine residue;
(III) the residue at position 6 of SEQ ID NO: 1 is an asparagine residue;
(IV) the residue at position 7 of SEQ ID NO: 1 is a tyrosine residue or a 3Py6NH2 residue; and
(V) the residue at position 9 of SEQ ID NO: 1 is an isoleucine residue.

Here, the alanine residue of (I) may be a derivative thereof, the valine residue of (II) may be a derivative thereof, the asparagine residue of (III) may be a derivative thereof, the tyrosine residue or 3Py6NH2 residue of (IV) may be a derivative thereof, and the isoleucine residue of (V) may be a derivative thereof.

Further, each of the options (I) to (V) above may be selected in any combination; however, the peptide may comprise an amino acid sequence that satisfies at least one of (I) to (V).

According to Example 3, (I) to (V) are considered to be amino acid residues that significantly affect binding to TfR1.

Therefore, when the amino acid residues of SEQ ID NO: 1 satisfy at least one, and preferably all, of the conditions (I) to (V), strong binding to TfR1 can be achieved.

The affinity of these peptides for the transferrin receptor is not limited thereto, but may have a KD of less than 50 nM, more preferably less than 20 nM, and even more preferably less than 10 nM.

Note that, in one embodiment of the peptide provided in this specification, the fourth amino acid residue of SEQ ID NO: 1 is a valine residue, which is also supported by the examples as an important residue for strong binding to TfR1.

In the peptide having the amino acid sequence set forth in SEQ ID NO: 1, the peptide may also have an amino acid sequence in which any one of the amino acid residues at the 2nd, 3rd, 5th, 6th, 7th, 8th, 9th, 10th, 11th, 12th, 13th and 14th positions of SEQ ID NO: 1 are replaced.

"Amino acid residue is substituted" means that a specific amino acid residue is replaced by another amino acid residue that may be modified. Herein, substitution of an amino acid residue is preferably a conservative amino acid substitution.

"May be modified" means that publicly known amino acid modifications or alterations may be applied. Examples of such modifications include N-methylation, amino acid modifications denoted by the aforementioned abbreviations, conversion to D-form, or transformation into known derivatives of the respective amino acids."

"May have a linker" means that a linker may be bound to the amino acid residue. Examples of amino acids with a linker include K (MePEG4c), in which MePEG4c is bound to the amino group of the side chain of a lysine residue, E (PEG1Me), in which PEG1Me is bound to the carboxy group of the side chain of a glutamic acid residue; F4aao (PEG8Me), in which PEG8Me is bound to the carboxy group of the side chain of F4aoo, E( PEG8Me), in which PEG8Me is bound to the carboxy group of the side chain of a glutamic acid residue; E (PEG8c), in which PEG8c is bound to the carboxy group of the side chain of a glutamic acid residue; E (PEG4c), in which PEG4c is bound to the carboxy group of the side chain of a glutamic acid residue; QPEG8Me, in which PEG8Me is bound to the amide group of the side chain of the glutamine residue, F3CONPEG4c, in which PEG4c is bound to the amide group of the side chain of F3CON, F3COO (PEG4Me), in which PEG4Me is bound to the carboxy group of the side chain of F3COO, F3COO (PEG4c), in which PEG4c is bound to the carboxy group of the side chain of F3COO, F3COO (PEG8c), in which PEG8c is bound to the carboxy group of the side chain of F3COO, F3COO (PEG8Me), in which PEG8Me is bound to the carboxy group of the side chain of F3COO, MeF3COO (PEG4Me), in which PEG4Me is bound to the carboxy group of the side chain of MeF3COO, and a cysteine residue, in which PEG4c bound to the carboxyl group of the C-terminal.

In one aspect of the present invention, this peptide may be a peptide (peptide B) having the following amino acid sequence.

That is, this peptide (peptide B) may be the above-mentioned peptide including an amino acid sequence which includes one or more substitutions selected from:
(I) substitution of the valine residue at position 2 of SEQ ID NO: 1 for one amino acid selected from the group consisting of T and Kmor;
(II) substitution of the MeF3C residue at position 3 of SEQ ID NO: 1 for one amino acid selected from the group consisting of MeF and Me3Py;
(III) substitution of the W7N residue at position 5 of SEQ ID NO: 1 for one amino acid selected from the group consisting of W1aa, W, W1EtOH, W1Me, 3Imp, and MeW;
(IV) substitution of the 3Py6NH2 residue at position 7 of SEQ ID NO: 1 for one amino acid selected from the group consisting of Y, Y26dF, 3Py6NHaa, Y3Me, and R;
(V) substitution of the F4OMe residue at position 8 of SEQ ID NO: 1 for one amino acid selected from the group consisting of F4aao, F4aao (Glucamine), F4aao (pipzaa), YaeCOpipzaa, 3Py6OMe, 3Py6NHaa, 3Imp, Y, and F4aao (PEG8Me);
(VI) substitution of the isoleucine residue at position 10 of SEQ ID NO:1 for one amino acid selected from the group consisting of Har, Kmor, T, Mor, Hpr, and S;
(VII) substitution of the arginine residue at position 11 of SEQ ID NO: 1 for one amino acid selected from the group consisting of Kmor, KCOpipzaa, K, E (Glucamine), Q, a Q optionally having a linker in a side chain, and E and E (apa) optionally having a linker in a side chain;
(VIII) substitution of the arginine residue at position 12 of SEQ ID NO: 1 for one amino acid selected from the group consisting of Kmor, KCOpipzaa, Q, and H;
(IX) substitution of the 4Py residue at position 13 of SEQ ID NO: 1 for one amino acid selected from the group consisting of F3COO, F, F3CONPEG4c, Y3Me, 3Py6Me, 3Py6OMe, 3Py6NH2, 4Py2Me, 4Py2OMe, 4Py2NH2, 3Py, Y, F3COO optionally having a linker, and E optionally having a linker in a side chain; and
(X) substitution of the MeTyr residue at position 14 of SEQ ID NO: 1 for one amino acid selected from the group consisting of MeF4COO, MeF3COO, MeF4C, MeF4OMe, MeF3C, MeF3OMe, Me4Py, Me3Py, and MeF4COO (PEG4c).

Each of the above options (I) through (X) may be selected in any combination.

The peptide of the present invention may also be a peptide having an amino acid sequence in which 1 to 3 amino acid residues are substituted, deleted, added, or inserted from any of the above sequences. In these cases, the peptide is preferably a peptide that has the ability to bind to hTfR.

In another aspect of the present invention, the peptide has the following amino acid sequence.

That is, this peptide may be a peptide in which:
(I) the amino acid residue at position 2 of SEQ ID NO: 1 is V, T, or Kmor;
(II) the amino acid residue at position 3 of SEQ ID NO: 1 is MeF3C, MeF, or Me3Py;
(III) the amino acid residue at position 5 of SEQ ID NO: 1 is W7N, W1aa, W, W1EtOH, W1Me, 3Imp, or MeW;
(IV) the amino acid residue at position 6 of SEQ ID NO: 1 is N or Nmm;
(V) the amino acid residue at position 7 of SEQ ID NO: 1 is 3Py6NH2, Y, Y26dF, 3Py6NHaa, or Y3Me;
(VI) the amino acid residue at position 8 of SEQ ID NO: 1 is F4OMe, F4aao, F4aao (Glucamine), F4aao (pipzaa), YaeCOpipzaa, 3Py6OMe, 3Py6NHaa, 3Imp, Y, or F4aao (PEG8Me);
(VII) the amino acid residue at position 9 of SEQ ID NO: 1 is I or Eva;
(VIII) the amino acid residue at position 10 of SEQ ID NO: 1 is I, Har, Kmor, T, Mor, Hpr, or S;
(IX) the amino acid residue at position 11 of SEQ ID NO: 1 is R, Kmor, KCOpipzaa, K, E (Glucamine), E (PEG8Me), Q, QPEG8Me, E (PEG8c), or E (PEG4c);
(X) the amino acid residue at position 12 of SEQ ID NO: 1 is R, Kmor, KCOpipzaa, Q, or H;
(XI) the amino acid residue at position 13 of SEQ ID NO: 1 is 4Py, F3COO, F, F3CONPEG4c, Y3Me, 3Py6Me, 3Py6OMe, 3Py6NH2, 4Py2Me, 4Py2OMe, 4Py2NH2, 3Py, F3COO (PEG4c), Y, E (PEG8c), F3COO (PEG8c), E (PEG4c), or F3COO (PEG8Me); and
(XII) the amino acid residue at position 14 of SEQ ID NO: 1 is MeY, MeF4COO, MeF3COO, MeF4C, MeF4OMe, MeF3C, MeF3OMe, Me4Py, or Me3Py.

Each of the above options (I) to (XII) may be selected in any combination.

The invention described in this specification also provides a peptide that binds to hTfR and has a binding ability that changes depending on pH.

As used herein, although not being limited thereto, the fact that the peptide has a binding ability that changes depending on pH (in this specification, also referred to as "having pH-dependent binding ability") means that the binding ability of the peptide to hTfR at one of at least two different pH values is at least two times or more, five times or more, and preferably ten times or more higher than the binding ability at the other value. Here, the binding ability may be the affinity (which can be expressed by KD) or dissociation rate (which can be expressed by dissociation rate constant: koff). The temperature at this time may be, for example, 25°C or 37°C.

Further, the two different pHs may be, but are not limited to, an acidic pH and a neutral or physiological pH. The acidic pH is preferably pH 4 to 6.8, and more preferably pH 5.5 to 6.8. The neutral pH is preferably pH 7 to 8, and more preferably pH 7.3 to 7.5. The physiological pH means a pH under normal physiological conditions in humans, and may be pH 7.3 to 7.4, pH 7.35 to 7.45, or pH 7.4.

Substances that bind to the transferrin receptor can pass through the blood-brain barrier and reach the brain by transcytosis. Factors that affect transcytosis include the binding ability of the substance to the transferrin receptor, as well as the acidification of endosomes. It has been reported that in acidified endosomes, the transcytosis of antibodies with reduced binding ability to the transferrin receptor is promoted (Non-Patent Literature 1). In other words, it is suggested that transcytosis is promoted when a substance with pH-dependent binding ability is used, such as a substance that binds strongly to the transferrin receptor at neutral pH and has a binding ability that decreases at acidic pH.

The peptide of the present invention includes, but is not limited to, a peptide that has pH-dependent binding ability such that the dissociation rate at acidic pH is at least two times or more, five times or more, and preferably ten times or more than the dissociation rate at neutral or physiological pH. It is believed that peptides having this property bind strongly to the transferrin receptor in blood and are taken up into endosomes, and then, in acidified endosomes, their binding ability to transferrin receptors is reduced, promoting transcytosis, thereby enabling the peptide to pass through the blood-brain barrier and reach the brain more efficiently.

The peptide having such a pH-dependent binding ability is preferably a peptide having the amino acid sequence of the above-mentioned peptide A and/or peptide B, and further, in the amino acid sequence set forth in SEQ ID NO: 1, the peptide preferably satisfies at least one of the following (I) to (V) and includes an amino acid sequence corresponding to at least either the following A or B.
(I) the residue at position 1 of SEQ ID NO: 1 is an alanine residue;
(II) the residue at position 4 of SEQ ID NO: 1 is a valine residue;
(III) the residue at position 6 of SEQ ID NO: 1 is an asparagine residue;
(IV) the residue at position 7 of SEQ ID NO: 1 is a tyrosine residue or a 3Py6NH2 residue; and
(V) the residue at position 9 of SEQ ID NO: 1 is an isoleucine residue.

A: An amino acid sequence that satisfies at least two of:
   (a) the residue at position 5 of SEQ ID NO: 1 is a W7N residue or a 3Imp residue;
   (b) the residue at position 7 of SEQ ID NO: 1 is a 3Py6NH2 residue;
   (c) the residue at position 8 of SEQ ID NO: 1 is a F4aao (pipzaa) residue; and
   (d) the residue at position 13 of SEQ ID NO: 1 is a 4Py residue.
B: An amino acid sequence in which (i) the residue at position 8 of SEQ ID NO: 1 is an F4aao (pipzaa) residue, an F4aao (Glucamine) residue, an F4aao (PEG8Me) residue, or a YaeCOpipzaa residue.

Here, the alanine residue of (I) may be a derivative thereof, the valine residue of (II) may be a derivative thereof, the asparagine residue of (III) may be a derivative thereof, the tyrosine residue or 3Py6NH2 residue of (IV) may be a derivative thereof, and the isoleucine residue of (V) may be a derivative thereof.

Further, the (a) W7N residue or 3Imp residue may be a derivative thereof, the (b) 3Py6NH2 residue may be a derivative thereof, the (c) F4aao (pipzaa) residue may be a derivative thereof, and the (d) 4Py residue may be a derivative thereof.

In addition, the F4aao (pipzaa) residue, F4aao (Glucamine) residue, F4aao (PEG8Me) residue, or YaeCOpipzaa residue in (i) may each be a derivative thereof.

Each of the above options (I) to (V), (a) to (d), and (i) may be selected in any combination.

However, preferably, peptides comprising the amino acid sequence listed as SEQ ID No. 162 among the above-mentioned peptides are excluded.

A preferred example of the peptide of the present invention is a peptide comprising an amino acid sequence described in any of SEQ ID No: 1 to 202, preferably SEQ ID No: 1 to 161 and 168 to 202, more preferably SEQ ID No: 1 to 149 and 168 to 197, and most preferably SEQ ID No: 1 to 68, or a conjugate of an amino acid sequence described in any of SEQ ID No: 1 to 202, preferably SEQ ID No: 1 to 161 and 168 to 202, more preferably SEQ ID No: 1 to 149 and 168 to 197, and most preferably SEQ ID No: 1 to 68, with a linker, wherein the peptide comprises the 1st to 15th amino acid residues of an amino acid sequence portion, and the amino acid sequence portion has a cyclic structure.

A preferred example of this peptide is any one of the above-mentioned peptides, having a cyclic structure.

A preferred example of this peptide is a peptide having a cyclic structure at the above 1-15th amino acid sequence site of any one of the above-mentioned peptides.

A preferred example of this peptide is any one of the above-mentioned peptides, which consists of an amino acid sequence in which the N-terminal amino acid, namely the 1st amino acid, is chloroacetylated and the acetyl group and the C-terminal amino acid, cysteine, are cyclized.

### In respect of cyclic peptides

Cyclic peptides refer to a peptide in which two amino acids are bound to each other in the peptide, thereby causing the peptide to be wholly or partially cyclized. Note that in the present application, cyclic peptides include those in which amino acids form a cross-linking structure in the peptide, those in which a cyclic structure is formed by lactam ring formation or a macrocyclization reaction, those having a lasso peptide-like structure, and the like. That is, in the present application, the cyclic peptide may be one in which a part thereof forms a cyclic structure, and it may also have a linear portion.

Peptides have problems such as general poor metabolic stability in vivo and difficulty in penetrating cell membranes due to their large size. In response to such problems, a method of cyclizing the peptide has been adopted. It has been suggested that peptide cyclization improves protease resistance and metabolic stability, in addition to also restricting conformational change, thereby increasing rigidity and improving a membrane penetrating property and affinity to a target protein.

### Cyclization method

Peptide cyclization can be performed according to a publicly known method.

For example, a peptide designed to comprise two or more cysteine residues can form a cyclic structure via a disulfide bond after translation, although the invention is not limited thereto. Further, cyclization can also be achieved by synthesizing a peptide having a chloroacetyl group at the N-terminus and placing a cysteine residue in the peptide using a genetic code reprogramming technique according to the method by Goto et al. (Y. Goto et al., Acss Chem. Biol. 3 120-129 (2008)). In this method, a mercapto group spontaneously performs a nucleophilic attack on the chloroacetyl group after translation, leading to cyclization of the peptide via a thioether bond. Cyclization may also be achieved by placing, in the peptide, a combination of other amino acids that are bonded to form a cycle by the genetic code reprogramming technique. Further, cyclization can also be achieved by synthesizing a peptide having cycloamide at the N-terminus and placing an Hgl residue in the peptide. As described above, as long as a publicly known cyclization method is used, cyclization can be performed without any particular limitations.

The peptide has a cyclic structure in which the N-terminal amino acid (the first amino acid residue) is bound to a cysteine residue contained in the peptide. In one embodiment, the peptide has a cyclic structure in which the N-terminal amino acid (the first amino acid residue) is bound to the 15th cysteine residue contained in the peptide. In one embodiment, the peptide has a cyclic structure in which a chloroacetylated N-terminal amino acid (the first amino acid residue) is bound to the 15th cysteine residue contained in the peptide. "Chloroacetylation" may also be "haloacetylation" using another halogen. Furthermore, "acetylation" may also be "acylation" using an acyl group other than an acetyl group.

In this specification, some amino acids may be modified for cyclization of the peptide. Amino acids with such partial modifications are also included in the present invention. For example, as mentioned above, a chloroacetyl group may be added to the amino acid located at the N-terminal, which is combined with a cysteine residue in the peptide for cyclization, and various (natural/unnatural) amino acids to which such a chloroacetyl group is added are also included as amino acids in this application.

A preferable example of this peptide is any of the above-mentioned peptides consisting of 15 amino acid residues.

A preferred example of this peptide is any one of the above-mentioned peptides, wherein a linker is bound to the 8th, 11th, 13th, or 15th amino acid residue of the amino acid sequence. More preferably, the peptide is a peptide set forth in any one of SEQ ID NOs: 1-167, preferably a peptide set forth in SEQ ID NOs: 1-161, more preferably a peptide set forth in SEQ ID NOs: 1-149, and most preferably a peptide set forth in SEQ ID NOs: 1-68.

### Peptide length

While a number of amide bonds (a number/length of amino acids) in the peptide and the peptide site is not particularly limited, a total of the amino acid residues (when a substance bound to the peptide or a linker through which the substance is bound to the peptide comprises amino acids, these amino acids are not comprised) is preferably 20 residues or less. As for the amino acids, it is preferable that there are 10 or more, or 11 or more, 12 or more, 13 or more, 14 or more and preferable that there are 19 or less, 18 or less, 17 or less, 16 or less.

Another embodiment described in this specification relates to composite (conjugate). The composite is a composite including any of the above-mentioned peptides, a linker bound to the peptide and a substance bound to the linker. Preferably, the composite is a composite that is at least capable of binding to TfR.

The above-mentioned composite may be a composite made by bonding the C-terminal of a cysteine (Cys) of any one of the above-mentioned peptides to the above-mentioned substance, or a composite made by bonding a linker attached to the above-mentioned C-terminal to the substance. Further the composite may be made by bonding a functional group of a side chain end contained in any one of the above-mentioned amino acid residues of the peptide to the substance, or a composite made by bonding a linker attached to the functional group to the substance.

An example of the linker is one having an amino acid length of 1 or more and 15 or less and comprising one or more of glycine (Gly) or serine (Ser).

A preferable example of this linker is cysteine (Cys) of which N-terminus may be modified or lysine (Lys) that may be modified.

Another example of the linker is one having an amino acid length of 1 or more and 5 or less and comprising either or both of D-configuration glutamic acid (de) and methylated glycine (MeG).

A preferable example of this linker is cysteine (Cys) of which N-terminus may be modified or lysine (Lys) that may be modified.

Another example of the linker is a PEG linker containing polyethylene glycol (PEG) or a derivative of polyethylene glycol. The derivative of polyethylene glycol includes all publicly known PEG linkers. A portion of the linker may be partially substituted for a functional group or functionalized with a functional group. Examples include, but are not limited to, a methyl group, an amino group, an azide group, and the like.

Preferably, the PEG linker is PEG4c, PEG8c, PEG12c, PEG8Me, MePEG4c, PEG1Me, PEG4Me or NHdPEG1Me.

In addition, the linker may further contain a reactive functional group to bind a desired substance. Examples of the reactive functional group include maleimide and hydrazide.

As another example, the linker be sequence described in the Linker section of SEQ ID NOs: 1 to 68 shown in Table 1-1. For example, in SEQ ID NO. 1, PEG4c is the linker, and in SEQ ID NO. 64, the [G-KN3] sequence starting from glycine, which is the amino acid residue listed in the Linker row, is the linker.

Preferred examples of the peptide or composite to which this linker is bound are those in which the linker are:
a G linker, which is a peptide linker consisting of polyethylene glycol (PEG), Gly, or MeG, a GS linker, which is a peptide linker consisting of Gly or MeG and Ser, and a GKN3 linker, which is a peptide linker consisting of Gly and KN3.

"Linker" (also called crosslinker) herein refers to a structure that is additionally bound to an amino acid residue in the cyclic amino acid sequence of a peptide that binds to the transferrin receptor, or an intermolecular linkage between the peptide and the substance to be delivered to the TfR, and the linker may be any linker known or described herein. In a specific embodiment, the above-mentioned linker is, for example, a chemical linker, a fatty acid linker, or a peptide linker (a polypeptide linker). Further, the linker may be a composite of, for example, a chemical linker, a peptide linker, and the like.

The linker may, for example, be dissociated or separated depending on the environment or conditions, or it may maintain a stable structure.

Chemical linker: in several embodiments, the linker may be a chemical linker. Examples of the chemical linker include, but are not limited to, substituted or unsubstituted alkylene, substituted or unsubstituted heteroalkylene, substituted or unsubstituted cycloalkylene, substituted or unsubstituted heterocycloalkylene, substituted or unsubstituted arylene, and/or substituted or unsubstituted heteroarylene. Further, the peptide and the linker can be conjugated via a sulfhydryl group, an amino group (amine), and/or a carbohydrate, or any suitable reactive group. Homobifunctional and heterobifunctional crosslinkers (conjugation agents) are available from many commercial sources. The crosslinker may contain a flexible arm, for example, 2, 3, 4, 5, 6, 7, 8, 9, 10, 11, 12, 13, 14 or 15 carbon atoms. Examples of the crosslinker include BSS3 ([bis(sulfosuccinimidyl)suberate]), NHSS/EDC (N-hydroxysuccinimide and N-ethyl(dimethylaminopropyl)carbodiimide, sulfo-EMCSS ([N-e-maleimidocaproic acid] hydrazide, hydrazide, and SSATA (N-succinimidyl-SS-acetylthioacetate).

A preferable example of the chemical linker is a PEG polyethylene glycol) linker. For example, the PEG linker may be a PEG linker consisting of 1 to 24 ethylene glycol units.

Fatty acid linker: the linker may be a fatty acid linker comprising a divalent chemical moiety derived from a fatty acid. For example, the fatty acid linker may be a linker comprising 12-aminododecanoic acid.

Peptide linker: a peptide linker comprising at least one amino acid (e.g., a peptide having at least 2, 3, 4, 5, 6, 7, 10, 15, 20, 25, 40 or 50 amino acids). In a specific embodiment, the linker is a single amino acid (e.g., any natural amino acid such as Cys). In another embodiment, a glycine-rich peptide such as a peptide having a sequence of [Gly-Gly-Gly-Gly-Ser] n (in the formula, n is 1, 2, 3, 4, 5, or 6) such as is described in U.S. Patent No. 7,271,149 or the like is used. In another embodiment, a serine-rich peptide linker such as is described in U.S. Patent No. 5,525,491 is used. Examples of the serine-rich peptide linker include those represented by a formula [X-X-X-X-Gly] y (in the formula, 2 Xs at most are Thr, with the rest of the Xs being Ser; y is 1 to 5) (e.g., Ser-Ser-Ser-Ser-Gly (in the formula, y is 2 or more)). In several cases, the linker is a single amino acid (e.g., any amino acid such as Gly or Ala).

Another embodiment disclosed in this specification includes the peptide to which the above-mentioned linker is bound or the above-mentioned composite, and the above-mentioned substance is the active ingredient. Another embodiment disclosed in this specification may be a composite including a substance bound to a peptide or a linker. The "bound substance" may be any substance bound to a peptide or a linker, and an example of the bound substance is a pharmaceutical or an active ingredient of a pharmaceutical.

The substance may be any substance desired by a person having ordinary skill in the art as long as it is a substance the skilled person desires to be delivered to the TfR. Further, when the above-mentioned pH-dependent peptide of the present invention is used, the substance may be a substance desired to be delivered into the brain. Examples of the substance include, but are not limited to, the following:
Compound: the compound may not only be small molecule compounds and medium molecule compounds but may also be any compound that can be introduced by a cell cytosis mechanism. Examples include a publicly known small molecule drug.

Peptide: the peptide may bind to an in vivo target to exhibit some kind of effect, and it may, for example, be a cyclic peptide.

RI: any may be used, as long as the RI is a compound that can be labeled with a radioisotope, such as a small or medium molecule compound, an antibody, or the like labeled with the radioisotope. Examples include a compound for PET examination.

Protein: any may be used, as long as the protein exhibits a useful function in vivo, such as an antibody, an enzyme, or the like. Examples include enzymes used for enzyme replacement therapy.

Nucleic acid: any may be used, as long as the nucleic acid contains a base sequence, such as DNA, RNA, or the like. Examples include a nucleic acid therapeutic.

DDS: a DDS molecule such as a liposome or a micelle may be used. The DDS molecule may also further comprise a compound, such as a pharmaceutical, therewithin.

Further, the substance may also be a composite of these above-mentioned substances.

Another embodiment disclosed in this specification relates to a composition that includes any of the above-mentioned peptides, or a composition that includes any of the above-mentioned composites. The composition may include known materials (e.g., a carrier or a solvent) in addition to the peptide or composite. Another embodiment disclosed in this specification relates to a method for producing a pharmaceutical or diagnostic composition. This method is a method for producing a prophylactic, therapeutic, or diagnostic agent, which includes the step of obtaining the above-mentioned composite.

In a preferred example of this method, the linker is polyethylene glycol (PEG), a G linker, a GS linker, or a GKN3 linker. Alternatively, the linker may be a sequence in the Linker section of SEQ ID NOs: 1-68 shown in Table 1-1. Alternatively, the linker may be a sequence in the Linker section of SEQ ID NOs: 168-197 shown in Table 1-3.

Another embodiment disclosed in this specification relates to a pharmaceutical or diagnostic composition including the above-mentioned peptide or a composite that includes a substance further bound to a conjugate of the peptide and a linker. Preferably, the composition is for use in a pharmaceutical or diagnostic composition for a TfR-related disease.

The peptide of the present invention can be produced by a publicly known peptide production method including a chemical synthesis method such as a liquid phase method, a solid phase method, a hybrid method that combines the liquid phase method and the solid phase method, and the like; a genetic recombination method; and the like.

In the solid phase method, for example, a hydroxyl group of a resin having the hydroxyl group and a carboxyl group of a first amino acid (normally the C-terminus amino acid of the target peptide) in which an α-amino group is protected by a protective group are subjected to an esterification reaction. A publicly known dehydration condensing agent such as 1-mesitylenesulfonyl-3-nitro-1,2,4-triazole (MSNT), dicyclohexylcarbodiimide (DCC), or diisopropyl carbodiimide (DIPCDI) can be used as an esterifying catalyst.

Next, the protective group of the α-amino group of the first amino acid is removed and a second amino acid in which all the functional groups other than a main chain carboxyl group are protected is added, followed by activation of the carboxyl group to allow the first amino acid and the second amino acid to bind to each other. Further, an α-amino group of the second amino acid is deprotected and a third amino acid in which all the functional groups other than a main chain carboxyl group are protected is added, followed by activation of the carboxyl group to allow the second amino acid and the third amino acid to bind to each other. This process is repeated, and when a peptide with an intended length has been synthesized, all the functional groups are deprotected.

Examples of the resin for solid phase synthesis include Merrifield resin, MBHA resin, Cl-Trt resin, SASRIN resin, Wang resin, Rink amide resin, HMFS resin, Amino-PEGAresin (Merck), HMPA-PEGA resin (Merck), and the like. These resins can be used after washing with a solvent (dimethylformamide (DMF), 2-propanol, methylene chloride, etc.).

Examples of the protective group of the α-amino group include a benzyloxycarbonyl (Cbz or Z) group, a tert-butoxycarbonyl (Boc) group, a fluorenylmethoxycarbonyl (Fmoc) group, a benzyl group, an allyl group, an allyloxycarbonyl (Alloc) group, and the like. The Cbz group can be deprotected by hydrofluoric acid, hydrogenation, or the like, the Boc group can be deprotected by trifluoroacetic acid (TFA), and the Fmoc group can be deprotected by a piperidine treatment.

A methyl ester, an ethyl ester, a benzyl ester, a tert-butyl ester, a cyclohexyl ester, or the like can be used to protect the α-carboxyl group.

As for other functional groups of the amino acid, a hydroxyl group of serine or threonine can be protected by a benzyl group or a tert-butyl group, and a hydroxyl group of tyrosine is protected by a 2-bromobenzyloxycarbonyl group or a tert-butyl group. An amino group of a lysine side chain and a carboxyl group of glutamic acid or aspartic acid can be protected in the same manner as that of the α-amino group and the α-carboxyl group.

The carboxyl group can be activated using a condensing agent. Examples of the condensing agent include dicyclohexylcarbodiimide (DCC), diisopropylcarbodiimide (DIPCDI), 1-ethyl-3-(3-dimethylaminopropyl) carbodiimide (EDC or WSC), (1H-benzotriazol-1-yloxy) tris (dimethylamino) phosphonium hexafluorophosphate (BOP), 1-[bis(dimethylamino)methyl]-1H-benzotriazolium-3-oxide hexafluorophosphate (HBTU), or the like.

A peptide chain can be cleaved from the resin by treating with an acid such as TFA, hydrogen fluoride (HF), or the like.

Peptide production by a gene recombination method (a translation synthesis system) can be performed using nucleic acids that encode the peptide of the present invention. The nucleic acids that encode the peptide of the present invention may be DNA or RNA.

The nucleic acids that encode the peptide of the present invention can be prepared by a publicly known method, or a method based thereon. For example, synthesis can be performed by an automatic synthesizer. A restriction enzyme recognition site for inserting the obtained DNA into a vector may be added, and a base sequence encoding an amino acid sequence for cleaving the resulting peptide chain with an enzyme or the like may also be incorporated.

As described above, when the peptide of the present invention is fused to a membrane-penetrating peptide or the like, the above-mentioned nucleic acids also include nucleic acids that encode a membrane-penetrating peptide.

To suppress degradation by a host-derived protease, a chimeric protein expression method in which the target peptide is expressed as a chimeric peptide with other peptides can be used. In this case, nucleic acids that encode the target peptide and a peptide to be bound hereto are used as the above-mentioned nucleic acids.

Next, an expression vector is prepared using the nucleic acids that encode the peptide of the present invention. The nucleic acids can be inserted downstream of the promoter of the expression vector as they are, by digestion with a restriction enzyme, by addition of a linker, or the like. Examples of the vector include an Escherichia coli-derived plasmid (pBR322, pBR325, pUC12, pUC13, pUC18, pUC19, pUC118, pBluescript II, etc.), a Bacillus subtilis-derived plasmid (pUB110, pTP5, pC1912, pTP4, pE194, pC194, etc.), a yeast-derived plasmid (pSH19, pSH15, YEp, YRp, YIp, YAC, etc.), a bacteriophage (e phage, M13 phage, etc.), a virus (retrovirus, vaccinia virus, adenovirus, adeno-associated virus (AAV), cauliflower mosaic virus, tobacco mosaic virus, baculovirus, etc.), a cosmid, and the like.

The promoter can be selected as appropriate according to the host type. When the host is an animal cell, for example, an SV40 (simian virus 40)-derived promoter or a CMV (cytomegalovirus)-derived promoter can be used. When the host is Escherichia coli, a trp promoter, a T7 promoter, a lac promoter, or the like can be used.

Nucleic acids encoding a DNA replication origin (ori), a selection marker (antibiotic resistance, auxotrophy, etc.), an enhancer, a splicing signal, a poly A addition signal, or a tag (FLAG, HA, GST, GFP, etc.), or the like can be incorporated in the expression vector.

Next, suitable host cells are transformed with the above-mentioned expression vector. The host can be selected as appropriate in relation to the vector, and, for example, Escherichia coli, Bacillus subtilis, Bacillus spp., yeast, an insect or an insect cell, an animal cell, or the like is used. For example, HEK293T cells, CHO cells, COS cells, myeloma cells, HeLa cells, or Vero cells can be used as the animal cell. Transformation can be performed according to a publicly known method such as a lipofection method, a calcium phosphate method, an electroporation method, a microinjection method, a particle gun method, or the like, depending on the host type. A transformant is cultured according to a conventional method to express the target peptide.

The peptide is purified from a culture product of the transformant as follows. After cultured cells are collected and suspended in a suitable buffer liquid, the suspension is subjected to a method such as an ultrasonic treatment, freezing and thawing, or the like to crush the cells, followed by centrifugation separation or filtration to obtain a crude extract. When the peptide is secreted in the culture liquid, a supernatant is collected.

Purification from the crude extract or the culture supernatant can also be performed by a publicly known method, or a method based thereon (e.g., salting out, a dialysis method, an ultrafiltration method, a gel filtration method, an SDS-PAGE method, ion exchange chromatography, affinity chromatography, reverse-phase high-performance liquid chromatography, etc.).

The obtained peptide may be converted from a free form to a salt or from a salt to a free form by a publicly known method or a method based thereon.

The translation synthesis system may be a cell-free translation system. Cell-free translation systems include, for example, a ribosomal protein, an aminoacyl-tRNA synthetase (ARS), a ribosomal RNA, an amino acid, rRNA, GTP, ATP, translation initiation factor (IF), elongation factor (EF), and release factor (RF), ribosome recycling factor (RRF), and other factors necessary for translation. An Escherichia coli extract liquid or a wheat germ extract liquid may also be added to increase expression efficiency. In addition, a rabbit red blood cell extract liquid or an insect cell extract liquid may also be added.

By continuously supplying a system including the above-mentioned components with energy using dialysis, it is possible to produce several hundred micrograms per milliliter to several milligrams per milliliter of proteins. The system may include an RNA polymerase for performing transcription from gene DNA at the same time. Examples of the commercially available cell-free translation system that can be used include an Escherichia coli-derived system such as "RTS-100" (registered trademark) available from Roche Diagnostics "PURE system" available from Gene Frontier, "PURExpress In Vitro Protein Synthesis Kit" available from New England Biolabs; a system using a wheat germ extract liquid such as is available from ZoeGene or CellFree Sciences; and the like.

According to the cell-free translation system, an expression product can be obtained in a highly pure form without purification.

In the cell-free translation system, an artificial aminoacyl-tRNA in which a desired amino acid or hydroxy acid is linked (acylated) to tRNA may be used instead of an aminoacyl-tRNA synthesized into a natural aminoacyl-tRNA synthase. Such aminoacyl-tRNA can be synthesized using an artificial ribozyme.

Examples of such a ribozyme include a flexizyme (H. Murakami, H. Saito, and H. Suga, (2003), Chemistry & Biology, Vol. 10, 655-662; H. Murakami, D. Kourouklis, and H. Suga, (2003), Chemistry & Biology, Vol. 10, 1077-1084; H. Murakami, A. Ohta, H. Ashigai, H. Suga (2006) Nature Methods 3, 357-359, "The flexizyme system: a highly flexible tRNA aminoacylation tool for the synthesis of nonnatural peptides"; N. Niwa, Y. Yamagishi, H. Murakami, H. Suga (2009) Bioorganic & Medicinal Chemistry Letters 19, 3892-3894 "A flexizyme that selectively charges amino acids activated by a water-friendly leaving group"; WO2007/066627; etc.). The flexizyme is also known as original flexizyme (Fx), and dinitrobenzyl flexizyme (dFx), enhanced flexizyme (eFx), amino flexizyme (aFx), and the like, which are modified therefrom.

Using a tRNA linked to a desired amino acid or hydroxy acid produced by the flexizyme makes it possible to translate a desired codon in association with the desired amino acid or hydroxy acid. A special amino acid may be used as the desired amino acid. For example, the above-mentioned non-natural amino acid required for cyclization can also be introduced into the binding peptide by this method.

Chemical synthesis of a macrocyclic peptide of the present invention and an analog thereof can be performed using various methods commonly used in the technical field, including stepwise solid phase synthesis, semisynthesis of a peptide fragment through conformationally-assisted re-ligation, and chemical ligation. The synthesis of the peptide and the analog thereof described in the present specification is chemical synthesis using various solid phase techniques, such as is described in, for example, K. J. Jensen, P. T. Shelton, S. L. Pedersen, Peptide Synthesis and Applications, 2nd Edition, Springer, 2013. A preferable strategy is based on a combination of an Fmoc group capable of temporarily protecting an α-amino group and selectively removable by a base, and a protective group that temporarily protects a side chain functional group and is stable under Fmoc removal conditions. Selection of such a general peptide side chain is known in the above-mentioned Peptide Synthesis and Applications, 2nd edition and G. B. Fields, R. L. Noble, Solid phase Peptide Synthesis Utilizing 9-Fluorenylmethoxycarbonyl Amino Acids, Int. J. Peptide Protein Res. 35, 1990, 161-214, however examples of the preferable peptide side chain protective group include a Boc group and an Mtt group for lysine and other amino groups, a tert-butyl group for a carboxyl group of glutamic acid and aspartic acid, and a Trt group and an Mmt group for a thiol group of cysteine.

The peptide and the analog thereof described in the present invention can be synthesized on the above-mentioned solid phase resin using a stepwise method. The α-amino protective groups of the C-terminus amino acid in use and all amino acids and peptides used in synthesis need to be selectively removed during the synthetic process. It is preferable that the synthetic process is performed using the above-mentioned solid phase resin and initiated by converting a C-terminus carboxyl group of a peptide in which the N-terminus is suitably protected by Fmoc or the like, or a C-terminus carboxyl group of an amino acid protected by Fmoc to an activated ester using a suitable reagent and adding the activated ester to an amino group on the solid phase resin. Subsequent elongation of the peptide chain can be achieved by sequentially repeating the removal of the N-terminus protective group (the Fmoc group) and the following condensation of the protected amino acid derivative according to the amino acid sequence of the target peptide. Note that these target peptides can be released in the final stage. For example, releasing can be performed in a TFA solution containing water/silyl hydride/thiol as a scavenger in TFA, as described as a releasing condition in Teixeira, W. E. Benckhuijsen, P. E. de Koning, A. R. P. M. Valentijn, J. W. Drijfhout, Protein Pept. Lett., 2002, 9, 379-385 and the like. TFA/water/TIS/DODT (a volume ratio of 92.5:2.5:2.5:2.5) is mentioned as a typical example thereof.

The synthesis of the peptide analog described in the present specification can be performed using a single or multi-channel peptide synthesizer, such as the Liberty Blue synthesizer available from CEM or the Syro I synthesizer available from Biotage.

Activation of the carboxy group can be performed using a condensing agent. Examples of the condensing agent include dicyclohexylcarbodiimide (DCC), diisopropylcarbodiimide (DIPCDI), 1-ethyl-3-(3-dimethylaminopropyl) carbodiimide (EDC or WSC), (1H-benzotriazol-1-yloxy) tris (dimethylamino) phosphonium hexafluorophosphate (BOP), 1- [bis (dimethyl amino) methyl]-1H-benzotriazolium-3-oxide hexafluorophosphate (HBTU), and the like.

Another embodiment disclosed in this specification relates to a pharmaceutical. This pharmaceutical includes the above-mentioned peptide and a pharmaceutically acceptable salt or solvate thereof (for simplicity, hereinafter these may be simply referred to as "peptides"). This pharmaceutical preferably includes an effective amount of the above-mentioned peptide as an active ingredient.

In the present specification, a route of administration of the pharmaceutical composition is not particularly limited, and the pharmaceutical composition may be administered either orally or parenterally. Examples of parenteral administration include administration by injection such as intramuscular injection, intravenous injection, or subcutaneous injection, transdermal administration, transmucosal administration (nasal, buccal, ocular, pulmonary, vaginal, or rectal administration), and the like.

Considering that polypeptides are an easily metabolizable and excretable substance, the above-mentioned pharmaceutical composition can be subjected to various modifications. For example, adding polyethylene glycol (PEG) or a sugar chain to the polypeptide can prolong the residence time of the peptide in the blood and reduce antigenicity of the peptide. Further, an emulsion, a nanoparticle, a nanosphere, or the like prepared using a biodegradable polymer compound such as polylactic acid-glycol (PLGA), porous hydroxyapatite, a liposome, a surface-modified liposome, or an unsaturated fatty acid may be used as a sustained-release base in which to encapsulate the polypeptide. In the case of transdermal administration, a weak electric current can be applied to the skin surface to allow the pharmaceutical composition to penetrate the stratum corneum (an iontophoresis method).

For the above-mentioned pharmaceutical composition, the active ingredient may be used as it is, or the pharmaceutical composition may be formulated by adding a pharmaceutically acceptable carrier, excipient, additive, or the like to the active ingredient. Examples of the dosage form include a liquid agent (e.g., an injected liquid), a dispersant, a suspension, a tablet, a pill, a powder, a suppository, a powdered drug, a fine granule, a granule, a capsule, a syrup, a troche, an inhalant, an ointment, an eye drop, a nasal drop, an ear drop, a cataplasm, and the like.

The formulation can be performed by a conventional method by using, for example, an excipient, a binder, a disintegrant, a lubricant, a solubilizing agent, a dissolution adjuvant, a colorant, a flavoring agent, a stabilizer, an emulsifier, an absorption promoter, a surfactant, a pH adjuster, an antiseptic, an antioxidant, or the like as appropriate.

Examples of components to be used in the formulation include, but are not limited to, purified water, saline, a phosphate buffer, dextrose, glycerol, a pharmaceutically acceptable organic solvent such as ethanol, animal or vegetable oil, lactose, mannitol, glucose, sorbitol, crystalline cellulose, hydroxypropyl cellulose, starch, corn starch, silicic anhydride, magnesium aluminum silicate, collagen, polyvinyl alcohol, polyvinylpyrrolidone, a carboxyvinyl polymer, carboxymethylcellulose sodium, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerol, glycerol, propylene glycol, vaseline, paraffin, octyldodecyl myristate, isopropyl myristate, a higher alcohol, stearyl alcohol, stearic acid, human serum albumin, and the like.

Considering that peptides are not easily absorbed through the mucosa, the above-mentioned pharmaceutical composition can include an absorption promoter to improve the absorption of a poorly absorbed pharmaceutical. Examples of a substance that can be used as such an absorption promoter include a surfactant such as a polyoxyethylene lauryl ether, sodium lauryl sulfate, or a saponin; a bile acid salt such as glycocholic acid, deoxycholic acid, or taurocholic acid; a chelating agent such as EDTA or a salicylic acid; a fatty acid such as caproic acid, capric acid, lauric acid, oleic acid, linoleic acid, or a mixed micelle; an enamine derivative; an N-acyl collagen peptide; an N-acyl amino acid; a cyclodextrin; a chitosan; a nitric oxide donor; and the like.

The pill or the tablet can also be coated with sugar or a gastro-soluble or enteric substance.

The injection liquid can comprise distilled water for injection, normal saline, propylene glycol, polyethylene glycol, vegetable oil, an alcohol, or the like. Further, a wetting agent, an emulsifier, a dispersant, a stabilizer, a solubilizing agent, a dissolution adjuvant, an antiseptic, or the like can be added.

When administered to a mammal (e.g., a human, a mouse, a rat, a guinea pig, a rabbit, a dog, a horse, a monkey, a pig, etc.), particularly a human, the dose to be administered of the pharmaceutical composition of the present invention is not particularly limited, and varies depending on a symptom, age, sex, weight, and sensitivity of a patient, an administration method, a dosing interval, a type of active ingredient, and a type of formulation. For example, the pharmaceutical composition can be administered once or split over several times at a dose of 30 µg to 100 g, 100 µg to 500 mg, or 100 µg to 100 mg. When administering by injection, the pharmaceutical composition may be administered once or split over several times at a dose of 1 µg/kg to 3000 µg/kg or 3 µg/kg to 1000 µg/kg, depending on a weight of a patient.

"TfR-related disease" refers to diseases in which TfR is a therapeutic target protein and diseases caused in cells or tissues expressing TfR. Further, since binding to TfR allows the blood-brain barrier to be crossed, "TfR-related disease" includes brain diseases (diseases caused by some abnormality in the brain, such as central nervous system (CNS) diseases) and neuromuscular diseases (diseases in which muscle weakness is caused by lesions of nerves or muscles, such as the brain, spinal cord, and peripheral nerves), because TfR is expressed in large numbers in muscle tissue.

### Detection agent for TfR-related diseases and detection kit

The present invention also includes the TfR-related disease detection agent including the peptide of the present invention. When used as the detection agent, the peptide of the present invention may be labelled to enable detection. Examples of substances used for peptide labelling include an enzyme such as peroxidase or alkaline phosphatase, a radioactive substance such as 1251, 1311, 35S, or 3H, a fluorescent substance such as fluorescein isothiocyanate, rhodamine, dansyl chloride, phycoerythrin, tetramethylrhodamine isothiocyanate, or a near-infrared fluorescent material, an antibody labeled with a luminescent substance such as luciferase, luciferin, aequorin, or the like. In addition, an antibody labeled with a nanoparticle such as colloidal gold or a quantum dot can also be detected. For example, a TfR -related disease can be detected by creating a composite of an antibody that binds to a specific target related to the brain-related disease and the peptide of the present invention, labeling the antibody or the peptide of the present invention in the composite, and administering and detecting the resulting composite.

Further, in an immunoassay, the peptide of the present invention can be labeled with biotin and detected by allowing avidin or streptavidin labeled with an enzyme or the like to bind to the biotin.

Among immunoassays, an ELISA method using enzyme labeling is preferable since antigens can be measured easily and quickly. For example, an antibody is immobilized on a solid phase carrier and after a sample is added to the carrier to cause a reaction, the labeled peptide of the present invention is added to the carrier mixture to cause a reaction. After washing, the carrier mixture is reacted with an enzyme substrate for color development, and by measuring an absorbance, it is possible to detect the TfR-related disease. After reacting the sample with the antibody immobilized on the solid phase carrier, the unlabeled peptide of the present invention may be added to the carrier mixture, then an antibody against the peptide of the present invention may be labeled with an enzyme and further added to the carrier mixture.

When the enzyme is a peroxidase, 3,3'-diaminobenzidine (DAB), 3,3',5,5'-tetramethylbenzidine (TMB), o-phenylenediamine (OPD), or the like can be used as the enzyme substrate, and when the enzyme is an alkaline phosphatase, p-nitropheny phosphate (NPP) or the like can be used.

In the present specification, the "solid phase carrier" is not particularly limited, as long as the carrier can immobilize antibodies, and examples include a microtiter plate made of glass, metal, resin, or the like, a substrate, a bead, a nitrocellulose membrane, a nylon membrane, a PVDF membrane, and the like. A target substance can be immobilized on these solid phase carriers in accordance with a publicly known method.

The detection kit according to the present invention includes a reagent and an instrument required for the above-mentioned detection (including, but not limited to, the peptide of the present invention, an antibody, a solid phase carrier, a buffer, an enzyme reaction terminator liquid, a microplate reader, etc.).

In another embodiment disclosed in this specification, it can also be considered that the invention is used as a detection kit including the above-mentioned TfR-related detection agent or a tool for elucidating a TfR-related disease and various cellular functions and biological phenomena associated with the TfR-related disease.

This specification also provides use of a peptide for the preparation of a pharmaceutical or diagnostic composition.

This specification also provides a preventative or therapeutic method for a TfR disease that includes a step of administering an effective amount of the peptide, the pharmaceutically acceptable salt or solvate thereof, or the composite thereof to a subject, which is a human, a non-human mammal, or a bird. The above-mentioned peptide can be used as appropriate as the peptide. Examples of the non-human mammal are a non-human primate, a pig, a cow, a dog, a cat, a horse, sheep, a rat, and a mouse.

This specification also provides a preventative or therapeutic method for a TfR-related disease that includes a step of administering an effective amount of the peptide, the pharmaceutically acceptable salt or solvate thereof, or the composite thereof to a subject, which is a human, a non-human mammal, or a bird.

This specification provides a method for testing a peptide and/or a composite, wherein the peptide and/or a composite including a substance further bound to a conjugate of the peptide and a linker is tested for at least one of:
a) solubility in a solvent;
b) binding ability to hTfR;
c) toxicity to a cell and/or a tissue; and
d) toxicity to an experimental animal, and
wherein the peptide is a peptide having an amino acid sequence in which one to three amino acid residues are deleted, substituted, inserted and/or added in the amino acid sequence of the peptide of the present invention.

In a specific embodiment of the method, the b) binding ability to hTfR may be a pH-dependent binding ability to hTfR.

The peptide of the invention maybe any of the peptides described herein, but as a preferred aspect of the peptide, the peptide may be a peptide that has the amino acid sequence of peptide A and/or peptide B, and further includes at least one of, or satisfies all of, the following in the amino acid sequence set forth in SEQ ID NO: 1:
(I) the residue at position 1 of SEQ ID NO: 1 is an alanine residue;
(II) the residue at position 4 of SEQ ID NO: 1 is a valine residue;
(III) the residue at position 6 of SEQ ID NO: 1 is an asparagine residue;
(IV) the residue at position 7 of SEQ ID NO:1 is a tyrosine residue or a 3Py6NH2 residue; and
(V) the residue at position 9 of SEQ ID NO: 1 is an isoleucine residue.

In the above, the alanine of (I) may be a derivative thereof, the valine of (II) may be a derivative thereof, the asparagine of (III) may be a derivative thereof, the tyrosine or 3Py6NH of (IV) may be a derivative thereof, and the isoleucine of (V) may be a derivative thereof.

Also, each of the options (I) to (V) above may be selected in any combination.

Further, the peptide of the present invention is not limited thereto, but in one aspect, it includes or consists of an amino acid sequence set forth in any one of SEQ ID NOs: 1 to 202, preferably any one of SEQ ID NOs: 1 to 161 and 168 to 202, more preferably any one of SEQ ID NOs: 1 to 149 and 168 to 197, and most preferably any one of SEQ ID NOs: 1 to 68.

In one aspect, the peptide is a cyclic peptide including or consisting of an amino acid sequence set forth in any one of SEQ ID NOs: 1 to 202, preferably any one of SEQ ID NOs: 1 to 161 and 168 to 202, more preferably any one of SEQ ID NOs: 1 to 149 and 168 to 197, and most preferably any one of SEQ ID NOs: 1 to 68.

Although not limited thereto, one skilled in the art may appropriately select one to three amino acids in the amino acid sequence set forth in any one of SEQ ID NOs: 1 to 202, preferably any one of SEQ ID NOs: 1 to 161 and 168 to 202, and create a sequence with deletion, substitution, insertion, and/or addition of amino acids.

In the above method, the number of amino acids to be deleted, substituted, inserted, and/or added is from 1 to 3, preferably from 1 to 2, and more preferably 1.

Deletion, substitution, insertion and/or addition of amino acid residues is not limited, but substitution is preferred, and conservative amino acid substitution is more preferred.

The peptide may contain or consist of an amino acid sequence having at least 80% or more, preferably 85% or more, 86% or more, 87% or more, 88% or more, 90% or more, 91% or more, 92% or more, 93% or more, 94% or more amino acid identity to the amino acid sequence of the peptide of the present invention.

The percent identity of two amino acid sequences may be determined by visual inspection and mathematical calculation. Alternatively, the percent identity of two protein sequences may be determined by comparing sequence information based on the algorithm of Needleman, S. B. and Wunsch, C. D. (J. Mol. Biol., 48: 443-453, 1970) and using the GAP computer program available from the University of Wisconsin Genetics Computer Group (UWGCG). Preferred default parameters of the GAP program include: (1) the scoring matrix, blosum 62, as described in Henikoff, S. and Henikoff, J. G. (Proc. Nat. Acad. Sci. USA, 89: 10915-10919, 1992); (2) 12 gap weights; (3) 4 gap length weights; and (4) no penalty for the terminal gap.

Other programs for sequence comparison, used by those skilled in the art, may also be used. Percent identity can be determined by comparison with sequence information using, for example, the BLAST program described in Altschul et al. (Nucl. Acids. Res., 25, p. 3389-3402, 1997). The program is available on the Internet at the websites of the National Center for Biotechnology Information (NCBI) or the DNA Data Bank of Japan (DDBJ). Various conditions (parameters) for identity searches by using the BLAST program are described in detail on the above-mentioned website, and although some settings can be changed as necessary, searches are usually performed using default values. Alternatively, the % identity of two amino acid sequences may be determined using a program such as the genetic information processing software GENETYX Ver. 7 (GENETYX CORPORATION), or the FASTA algorithm. In such cases, default values may be used for the search.

Based on the amino acid sequence prepared in this way, a person skilled in the art can prepare peptides to be used in the test method of the present invention by known peptide production methods, such as chemical synthesis methods, e.g. the above-mentioned liquid phase method, solid phase method, and hybrid method combining liquid phase method and solid phase method, and genetic recombination method and the like.

The peptide having the amino acid sequence in which 1 to 3 amino acid residues are deleted, substituted, inserted and/or added (i.e., a peptide to be tested described in the test method) may be, but is not limited to, a peptide that has the amino acid sequence of peptide A and/or peptide B, and further includes at least one of, or satisfies all of, the following in the amino acid sequence set forth in SEQ ID NO: 1:
(I) the residue at position 1 of SEQ ID NO: 1 is an alanine residue;
(II) the residue at position 4 of SEQ ID NO: 1 is a valine residue;
(III) the residue at position 6 of SEQ ID NO: 1 is an asparagine residue;
(IV) the residue at position 7 of SEQ ID NO:1 is a tyrosine residue or a 3Py6NH2 residue; and
(V) the residue at position 9 of SEQ ID NO: 1 is an isoleucine residue. Such peptides are considered to have high binding affinity to hTfR.

In the above, the alanine of (I) may be a derivative thereof, the valine of (II) may be a derivative thereof, the asparagine of (III) may be a derivative thereof, the tyrosine or 3Py6NH of (IV) may be a derivative thereof, and the isoleucine of (V) may be a derivative thereof.

Each of the above options (I) through (V) may be selected in any combination.

The peptide to be tested is preferably, but not limited to, a peptide having the amino acid sequence of the above-mentioned peptide A and/or peptide B, and further, in the amino acid sequence set forth in SEQ ID NO: 1, the peptide satisfies at least one of the above (I) to (V) and includes an amino acid sequence corresponding to at least either the following A or B.
A: An amino acid sequence that satisfies at least two of:
   (a) the residue at position 5 of SEQ ID NO: 1 is a W7N residue or a 3Imp residue;
   (b) the residue at position 7 of SEQ ID NO: 1 is a 3Py6NH2 residue;
   (c) the residue at position 8 of SEQ ID NO: 1 is a F4aao (pipzaa) residue; and
   (d) the residue at position 13 of SEQ ID NO: 1 is a 4Py residue.
B: An amino acid sequence in which (i) the residue at position 8 of SEQ ID NO: 1 is an F4aao (pipzaa) residue, an F4aao (Glucamine) residue, an F4aao (PEG8Me) residue, or a YaeCOpipzaa residue.

Such peptides are considered to have high binding ability to hTfR and pH-dependent binding ability.

In the above, the (a) W7N residue or 3Imp residue may be a derivative thereof, the (b) 3Py6NH2 residue may be a derivative thereof, the (c) F4aao (pipzaa) residue may be a derivative thereof, and the (d) 4Py residue may be a derivative thereof.

In addition, the F4aao (pipzaa) residue, F4aao (Glucamine) residue, F4aao (PEG8Me) residue or YaeCOpipzaa residue in (i) may each be a derivative thereof.

Each of the above options (I) to (V), (a) to (d) and (i) may be selected in any combination.

However, preferably, peptides comprising the amino acid sequence listed as Sequence ID No. 162 among the above-mentioned peptides are excluded.

Regarding the test method, the solubility of a peptide in a solvent may be tested by measuring the solubility of the peptide in the solvent. In the measurement of solubility, the solvent is not limited, and may be freely selected according to the purpose. For the method of solubility measurement, a known method can be appropriately selected according to the type of solvent.

The test for binding ability to hTfR may be a measurement of binding ability to hTfR, and tests that may preferably be used include, but are not limited to, known methods such as the surface plasmon resonance (SPR) assay described above, Scatchard analysis and/or radioimmunoassay (RIA), and competitive binding assay such as enzyme immunoassay (EIA) and sandwich competition assay, and the like. In addition, the test for pH-dependent binding ability to hTfR may be performed at the various pH values described above to measure the binding ability to hTfR.

The test of toxicity to cells and/or tissues may be any known toxicity evaluation test using cells and/or tissues, e.g., in-vitro. Cells and tissues may be, but are not limited to, cells and/or tissues for which toxicity evaluation studies are usually performed for pharmaceutical products.

The test method for toxicity in experimental animals may be any known toxicity evaluation test using experimental animals. The experimental animals may be any animal that is normally used, and examples thereof include, but are not limited to, mouse, rat, guinea pig, gerbil, hamster, ferret, rabbit, dog, cat, pig, goat, horse, cow, bird (e.g., chicken, quail, etc.), monkey, primate other than human (e.g., capuchin monkey, marmoset, rhesus monkey, etc.), and the like.

The above toxicity assessment test can be, but is not limited to, a safety test that is usually conducted in non-clinical test of pharmaceuticals, including, for example, a general toxicity test (single-dose toxicity test/repeated-dose toxicity test), a genotoxicity test (Ames test/chromosome aberration test/in-vitro micronucleus test), an oncogenicity test, reproductive toxicity test (ICH-1,II, I1), local irritation test (eye irritation test, skin irritation test, etc.), other toxicity test (skin sensitization test, phototoxicity test, antigenicity test), chemical analysis and bioanalysis (TK/PK), and the like.

The abbreviations used in the present specification, especially in the below-mentioned representative examples, are well-known to those skilled in the art. Several of the abbreviations used are as follows:
AF647 as Alexa Fluor (registered trademark) 647;
Å as angstrom (unit);
CIAc as chloroacetyl;
Cy5SAlk as Sulfo-Cy5-alkyne;
DCM as dichloromethane;
TIPS as triisopropylsilyl;
tBu as tert-butyl;
DTT as dithiothreitol;
DMSO as dimethyl sulfoxide;
Trt as trityl;
Boc as tert-butoxycarbonyl;
DMF as N, N-dimethylformamide;
DIEA or DIPEA as N, N-diisopropylethylamine;
DIPCI as N, N'-diisopropylcarbodiimide;
Oxyma Pure as ethyl cyano(hydroxyimino)acetate;
DODT as 3,6-dioxer-1,8-octane-dithiol;
Fmoc as 9-fluorenylmethyloxycarbonyl;
g as gram (unit);
HATU as O-(7-azabenzotriazol-1-yl)-N, N, N', N'-tetramethyluronium hexafluorophosphate;
HOSu as N-hydroxysuccinimide;
HPLC as high performance liquid chromatography;
LC-MS or LC/MS as liquid chromatography mass spectrometer;
mL as millilitre (unit);
M as molar (unit);
µL as microliter (unit);
mM as millimolar (unit);
µM as micromolar (unit);
mmol as millimole (unit);
mg as milligram (unit);
MeCN or CH3CN as acetonitrile;
min as minute (unit);
mm as millimeter (unit);
µm as micrometer (unit);
nm as nanometer (unit);
nM as nanomolar (units);
OSu as succinimide;
PEG as polyethylene glycol;
rpm as revolutions per minute (unit);
tBu as tert-butyl;
TFA as trifluoroacetic acid;
TIS as triisopropylsilane;
Trt or Tr as trityl group;
H-PEG4Me as 2,5,8,11-tetraoxatridecan-13-amine;
H-PEG8Me as 2,5,8,11,14,17,20,23-octaoxapentacosan-25-amine;
AA as amino acid;
PyAOP as 7-((azabenzotriazol-1-yloxy) tripyrrolidinophosphonium hexafluorophosphate;
CSA as 10-camphorsulfonic acid;
Fmoc-OSu as N-(9-fluorenylmethoxycarbonyloxy) succinimide;
THF as tetrahydrofuran;
ClAcOSu as N-(chloroacetoxy)succinimide;
Pd2(dba)3 as Tris(dibenzylideneacetone)dipalladium (0);
SPhos as 2-Dicyclohexylphosphino-2',6'-dimethoxybiphenyl;
EDCl-HCl as 1-(3-Dimethylaminopropyl)-3-ethylcarbodiimide hydrochloride;
Pd (PPh3)4 as Tetrakis(triphenylphosphine)palladium (0);
H-pipzaa (tBu) as tert-butyl 2-(piperazin-1-yl) acetate;
H-PEG4c(tBu) as Tert-butyl 1-amino-3,6,9,12-tetraoxapentadecan-15-oate; and
TEAA as triethylammonium acetate.
Fmoc-F3COO (allyl)-OH (CAS No. 293141-52-1) as (S)-2-(((9H-fluoren-9-ylmethoxy) carbonyl) amino)-3-(3-((allyloxy) carbonyl) phenyl) propanoic acid;
Fmoc-E(allyl)-OH (CAS No. 133464-46-7) as (S)-2-(((9H-fluoren-9-ylmethoxy) carbonyl) amino)-5-(allyloxy)-5-oxopentanoic acid;
Boc-apa-H (CAS No. 75178-96-0) as tert-Butyl (3-aminopropyl) carbamate;
apa (CAS No. 109-76-2) as Propane-1,3-diamine;
Fmoc-MeF4COO (allyl)-OH (CAS No. 2973754-47-9) as (S)-2-((((9H-fluoren-9-ylmethoxy) carbonyl) (methyl)amino)-3-(4-((allyloxy) carbonyl) phenyl) propanoic acid [Example 1]

### Chemical synthesis

All raw materials, building blocks, reagents, acids, bases, solid phase resins, and solvents used in the chemical synthesis in the following examples are either commercially available products used as is, or products that can be synthesized by those skilled in the art using organic chemical methods. Note that for amino acids comprising protective groups, commercially available products were used as is unless otherwise specified.

Elongation of the peptide chain on the solid phase resin was performed using the resin described in each example as a starting raw material normally used under peptide coupling reaction conditions and Fmoc removal reaction conditions. The reaction was performed using Liberty Blue available from CEM, which is an automatic peptide synthesizer, according to the manufacturer's manual. General amino acids used are listed below, with side chain protective groups indicated in parentheses.

Fmoc-Trp(Boc)-OH;Fmoc-Thr(tBu)-OH;Fmoc-N-Me-Gly-OH;Fmoc-Asp(OtBu)-OH;Fmoc-N-Me-Phe-OH;Fmoc-Ala-OH;Fmoc-N-Me-Ala-OH;Fmoc-His(Trt)-OH;Fmoc-Tyr(tBu)-OH;Fmoc-Val-OH;Fmoc-HydPro(tBu)-OH;Fmoc-Cys(Trt)-OH;Fmoc-Lys(Mtt)-OH;Fmoc-Ser(tBu)-OH;Fmoc-N-Me-Ser(tBu)-OH.

Introduction of a chloroacetyl group was performed using the solid phase resin holding the Fmoc-protected peptide obtained in the previous step by removing the Fmoc group of the α-amino group using the above-mentioned method, then adding chloroacetic acid (3 Eq), 3 Eq of an N, N'-diisopropylcarbodiimide-DMF solution (0.5 M), and 3 Eq of a HOAt-DMF solution (0.5 M) to the resin and shaking at room temperature for 40 minutes.

For deprotecting the side chain and cleaving from the solid phase resin, the resin obtained after the chloroacetyl group introduction step was first washed five times each with DMF and methylene chloride then dried under reduced pressure. Next, a reagent cocktail-A (a mixture of TFA/H₂O/TIS/DODT in a volume ratio of 92.5:2.5:2.5:2.5) was added to the reaction vessel containing the solid phase resin, followed by shaking at room temperature for 150 minutes. The reaction liquid was collected by frit filtration. The solid phase resin remaining in the reaction vessel was shaken again with a cleavage cocktail, then the solution component was collected by frit and mixed with the above-mentioned filtrate. When an excess of diethyl ether cooled to 0°C was added to this filtrate, a white cloudy precipitate was produced. This mixture was subjected to centrifugal separation (9000 rpm, 3 mins) and the resulting solution was subjected to decantation. After the obtained solid was washed again with a small amount of diethyl ether cooled to 0°C, the solid thus obtained was used for the subsequent cyclization reaction.

The cyclization reaction of the peptide was performed by dissolving the peptides in DMSO to a final concentration of 5 mM on the basis of the number of moles of the solid phase resin, then adding 6 Eq of triethylamine, followed by stirring at room temperature for about 16 hours. The reaction solution thus obtained was acidified with acetic acid and concentrated under reduced pressure using Biotage (registered trademark) V-10 (Biotage Japan).

As a method for purifying the obtained crudely purified peptides, reverse-phase preparative HPLC was used with the AutoPurification System with a single quadrupole mass spectrometer SQD2 available from Waters to elute the peptides while monitoring m/z ions derived from the target object. It was confirmed that a mass spectrum obtained using the ESI-positive scan mode and a mass spectrum including the multivalent ions calculated from the molecular formula of the target object matched within the error range of the mass spectrometer used. Note that purification conditions including the columns used were indicated in each example.

The structure of the chemically synthesized peptides was confirmed by electrospray ionization mass spectrometry in positive ion mode (ESI-MS (+)) based on the molecular weight calculated by taking into account the amino acids used according to the target sequence and, as necessary, the building blocks employed. The detected masses were reported in units of m/z. Compounds with molecular weights approximately greater than 1000 were frequently detected as doubly or triply charged ions. The analysis was carried out using the following methods (Analysis Conditions A and B), or by the methods described in each respective example.

### (Analysis method)

Analysis conditions A
   Column; Kinetex EVO C18 2.6 µm, 2.1 ID x 150 mm, 100 Å (Phenomenex)
   Column temperature: 60°C
   Mobile phase A: 0.025% TFA in H₂O
   Mobile phase B: 0.025% TFA in CH3CN
   Gradient: 5-45% over 7.2 minutes
   Flow rate: 0.5 mL/min
   Detection: PDA (225 nm)
Analysis conditions B
   Column; Kinetex EVO C18 2.6 µm, 2.1 ID x 150 mm, 100 Å (Phenomenex)
   Column temperature: 60°C
   Mobile phase A: 0.025% TFA in H₂O
   Mobile phase B: 0.025% TFA in CH3CN
   Gradient: 20-60% over 7.2 minutes
   Flow rate: 0.5 mL/min
   Detection: PDA (225 nm)

### [Example 2]

### Chemical synthesis of special cyclic peptides that bind to hTfR

Peptides with binding activity to hTfR and hTfR-binding peptides with a linker were chemically synthesized. The sequences of the synthesized peptides are shown in Tables 1-1 and 1-2, Table 2, and Table 3. The synthesized peptides were analyzed under the analytical conditions described in each example, and the structure was confirmed by ESI-MS (+) in mass spectrometry. The obtained ESI-MS (+) observation and the value of X when expressed as the number of proton additions (M+XH) X+ in such cases are shown in Tables 1-1 and 1-2, Table 2, and Table 3. Note that the N-terminal of all peptides is -NH2.

In the event of any discrepancy between the sequence in the sequence listing and the sequence in the table below, the sequence in the table below is correct.

**[Table 1-1]**

| [Table 1-1-1-1] | | | | | | | | | | | | | | | | |
|---|---|---|---|---|---|---|---|---|---|---|---|---|---|---|---|---|
| SEQ ID No. | Peptide ID | Peptide SEQ | | | | | | | | | | | | | | |
| | | 1 | 2 | 3 | 4 | 5 | 6 | 7 | 8 | 9 | 10 | 11 | 12 | 13 | 14 | 15 |
| 1 | 894_0462 | A | V | MeF3C | V | W7N | N | 3Py6NH2 | F4OMe | I | I | R | R | 4Py | MeY | C |
| 2 | 894_0463 | A | V | MeF3C | V | W7N | N | 3Py6NH2 | F4OMe | I | I | Kmor | R | 4Py | MeY | C |
| 3 | 894_0464 | A | V | MeF3C | V | W7N | N | 3Py6NH2 | F4OMe | I | I | R | Kmor | 4Py | MeY | C |
| 4 | 894_0465 | A | V | MeF3C | V | W7N | N | 3Py6NH2 | F4OMe | I | I | Kmor | Kmor | 4Py | MeY | C |
| 5 | 894_0466 | A | V | MeF3C | V | W7N | N | 3Py6NH2 | F4OMe | I | I | Kmor | KCOpipzaa | 4Py | MeY | C |
| 6 | 894_0467 | A | V | MeF3C | V | W1aa | N | 3Py6NH2 | F4OMe | I | I | Kmor | Kmor | 4Py | MeY | C |
| 7 | 894_0468 | A | V | MeF3C | V | W7N | N | 3Py6NH2 | F4aao | I | I | Kmor | Kmor | 4Py | MeY | C |
| 8 | 894_0469 | A | V | MeF3C | V | Wlaa | N | 3Py6NH2 | F4OMe | I | I | Kmor | KCOpipzaa | 4Py | MeY | C |
| 9 | 894_0470 | A | V | MeF3C | V | W7N | N | 3Py6NH2 | F4aao | I | I | Kmor | KCOpipzaa | 4Py | MeY | C |
| 10 | 894_471 | A | V | MeF3C | V | W7N | N | 3Py6NH2 | F4OMe | I | I | E(Glucamine) | R | 4Py | MeY | C |
| 11 | 894_472 | A | V | MeF3C | V | W7N | N | 3Py6NH2 | F4OMe | I | I | E(PEG8Me) | R | 4Py | MeY | C |
| 12 | 894_473 | A | V | MeF3C | V | W7N | N | 3Py6NH2 | F4aao(Glucamine) | I | I | E(Glucamine) | R | 4Py | MeY | C |
| 13 | 894_474 | A | V | MeF3C | V | W7N | N | 3Py6NH2 | F4aao(Glucamine) | I | I | E(Glucamine) | Kmor | 4Py | MeY | C |
| 14 | 894_479 | A | V | MeF3C | V | W7N | N | 3Py6NH2 | F4aao | I | I | KCOpipzaa | R | 4Py | MeY | C |
| 15 | 894_480 | A | V | MeF3C | V | W7N | N | 3Py6NH2 | F4aao | I | I | KCOpipzaa | Kmor | 4Py | MeY | C |
| 16 | 894_483 | A | V | MeF3C | V | W7N | N | Y | F4aao(pipzaa) | I | I | Q | R | 4Py | MeY | C |
| 17 | 894_485 | A | V | MeF3C | V | W1aa | N | 3Py6NH2 | F4aao(pipzaa) | I | I | KCOpipzaa | Kmor | 4Py | MeY | C |
| 18 | 894_486 | A | V | MeF3C | V | W7N | N | Y | F4aao(pipzaa) | I | I | KCOpipzaa | R | 4Py | MeY | C |
| 19 | 894_487 | A | V | MeF3C | V | W7N | N | Y | F4aao(pipzaa) | I | I | KCOpipzaa | Kmor | 4Py | MeY | C |
| 20 | 894_488 | A | V | MeF3C | V | W7N | N | 3Py6NH2 | F4OMe | I | I | E(Glucamine) | Kmor | 4Py | MeY | C |
| 21 | 894_490 | A | V | MeF3C | V | W7N | N | Y | F4aao(pipzaa) | I | I | E(PEG8Me) | Kmor | F3COO | MeY | C |

**[Table 1-1-1-2]**

| SEQ ID No. | Linker | hTfR SPR pH7.4 | hTfR SPR pH6.0 | dependency koff | dependency KD | m/z | [M+XH]X+ | Analysi -s conc -itions |
|---|---|---|---|---|---|---|---|---|
| | | KD(nM) | KD(nM) | pH6.0/7.4 | pH6.0/7.4 | | | |
| 1 | PEG4c | 1.56 × 10^-9 | 1.82 × 10^-8 | 39.79 | 11.66 | 793.93 | 3 | A |
| 2 | PEG4c | 8.88 × 10^-10 | 1.62 × 10^-8 | 112.65 | 18.23 | 807.95 | 3 | A |
| 3 | PEG4c | 2.63 × 10^-9 | 1.61 × 10^-8 | 51.40 | 6.12 | 807.98 | 3 | A |
| 4 | PEG4c | 2.19 × 10^-9 | 2.97 × 10^-8 | 56.94 | 13.59 | 821.94 | 3 | A |
| 5 | PEG4c | 6.31 × 10^-9 | 1.17 x 10^-7 | 74.33 | 18.53 | 855.34 | 3 | A |
| 6 | PEG4c | 6.56 × 10^-9 | 2.10 × 10^-8 | 38.89 | 3.19 | 840.68 | 3 | B |
| 7 | PEG4c | 1.63 × 10^-8 | 8.98 × 10^-8 | 61.90 | 5.51 | 836.70 | 3 | A |
| 8 | PEG4c | 1.07 × 10^-8 | 1.13 × 10^-7 | 72.99 | 10.55 | 1310.60 | 2 | B |
| 9 | PEG4c | 2.91 × 10^-8 | 7.74 × 10^-7 | 57.29 | 26.64 | 870.00 | 3 | A |
| 10 | PEG4c | 2.50 × 10^-9 | 3.66 × 10^-8 | 41.87 | 14.64 | 839.31 | 3 | A |
| 11 | PEG4c | 7.10 × 10^-9 | 1.10 × 10^-7 | 57.53 | 15.49 | 906.45 | 3 | B |
| 12 | PEG4c | 7.12 × 10^-9 | 1.37 × 10^-7 | 56.55 | 19.24 | 908.38 | 3 | A |
| 13 | PEG4c | 1.14 × 10^-8 | 1.33 × 10^-7 | 62.46 | 11.67 | 922.42 | 3 | A |
| 14 | PEG4c | 3.08 × 10^-8 | 1.08 × 10^-7 | 63.64 | 3.51 | 855.75 | 3 | A |
| 15 | PEG4c | 1.59 × 10^-8 | 1.02 × 10^-7 | 43.85 | 6.42 | 869.77 | 3 | A |
| 16 | PEG4c | 2.94 × 10^-9 | 4.03 × 10^-8 | 28.19 | 13.71 | 841.02 | 3 | B |
| 17 | PEG4c | 4.25 × 10^-9 | 5.01 × 10^-7 | 74.44 | 117.77 | 1395.67 | 2 | A |
| 18 | PEG4c | 1.88 × 10^-9 | 3.00 × 10^-8 | 25.31 | 16.00 | 1346.07 | 2 | A |
| 19 | PEG4c | 4.82 × 10^-9 | 7.92 × 10^-9 | 10.08 | 1.64 | 1367.13 | 2 | A |
| 20 | PEG4c | 4.08 × 10^-9 | 4.13 × 10^-8 | 42.49 | 10.13 | 853.02 | 3 | A |
| 21 | PEG4c | 5.37 × 10^-9 | 1.01 × 10^-7 | 11.57 | 18.80 | 991.43 | 3 | B |

**[Table 1-1-1-2]**

| SEQ ID No. | Peptide ID | Peptide SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 22 | 894_491 | A | V | MeF3C | V | W1aa | N | Y | F4aao(pipzaa) | I | I | E(PEG8Me) | Kmor | 4Py | MeY | C |
| 23 | 894_493 | A | V | MeF3C | V | W7N | N | Y | F4aao(pipzaa) | I | I | Q | Kmor | 4Py | MeY | C |
| 24 | 894_494 | A | V | MeF3C | V | W7N | N | Y | F4aao(pipzaa) | I | I | Q | Kmor | F3COO | MeY | C |
| 25 | 894_495 | A | V | MeF3C | V | W1aa | N | Y | F4aao(pipzaa) | I | I | Q | Kmor | 4Py | MeY | C |
| 26 | 894_0496 | A | V | MeF3C | V | W7N | N | Y | F4aao(Glucamine) | I | I | E(Glucamine) | R | 4Py | MeY | C |
| 27 | 894_0497 | A | V | MeF3C | V | W7N | N | Y | F4aao(Glucamine) | I | I | E(Glucamine) | Kmor | 4Py | MeY | C |
| 28 | 894_0498 | A | V | MeF3C | V | W7N | N | Y | F4aao(Glucamine) | I | I | Q | R | 4Py | MeY | C |
| 29 | 894_0499 | A | V | MeF3C | V | W7N | N | Y | F4aao(Glucamine) | I | I | Q | Kmor | 4Py | MeY | C |
| 30 | 894_0503 | A | V | MeF3C | V | W1aa | N | Y | F4aao(pipzaa) | I | I | E(PEG8Me) | Kmor | F | MeY | C |
| 31 | 894_0504 | A | V | MeF3C | V | W | N | Y | F4aao(pipzaa) | I | I | E(PEG8Me) | | F3COO | MeY | C |
| 32 | 894_0505 | A | V | MeF3C | V | W1aa | N | Y | F4aao(pipzaa) | I | I | Kmor | Kmor | F3COO | MeY | C |
| 33 | 894_0506 | A | V | MeF3C | V | W1aa | N | Y | F4aao(pipzaa) | I | I | KCOpipzaa | Kmor | 4Py | MeY | C |
| 34 | 894_0507 | A | V | MeF3C | V | W1aa | N | Y | F4aao(pipzaa) | I | I | KCOpipzaa | R | 4Py | MeY | C |
| 35 | 894_0508 | A | V | MeF3C | V | W1aa | N | Y | F4aao(pipzaa) | I | I | Q | Kmor | F3COO | MeY | C |
| 36 | 894_0509 | A | V | MeF3C | V | W1aa | N | Y | F4aao(pipzaa) | I | I | Q | R | F3COO | MeY | C |
| 37 | 894_0510 | A | V | MeF3C | V | W | N | Y | F4aao(pipzaa) | I | I | E(Glucamine) | Q | F | MeY | C |
| 38 | 894_0511 | A | V | MeF3C | V | W | N | Y | F4aao(pipzaa) | I | I | KCOpipzaa | Q | F | MeY | C |
| 39 | 894_0512 | A | V | MeF3C | V | W | N | Y | F4aao(Glucamine) | I | I | E(Glucamine) | Q | F | MeY | C |
| 40 | 894_0513 | A | V | MeF3C | V | W1aa | N | Y | F4aao(pipzaa) | I | I | E(Glucamine) | Kmor | 4Py | MeY | C |
| 41 | 894_0514 | A | V | MeF3C | V | W1aa | N | Y | YaeCOpipzaa | I | I | E(PEG8Me) | Kmor | 4Py | MeY | C |
| 42 | 894_0516 | A | V | MeF3C | V | W7N | N | Y | F4aao(pipzaa) | I | I | E(PEG8Me) | R | F3COO | MeY | C |
| 43 | 894_0532 | A | V | MeF3C | V | W7N | N | 3Py6NH2 | F4aao | Eva | I | E(PEG8Me) | R | F3COO | MeY | C |

**[Table 1-1-2-2]**

| SEQ ID No. | Linker | hTfR SPR pH7.4 | hTfR SPR pH6.0 | dependency koff | dependency KD | m/z | [M+XH]X+ | Analysi -s cond -itions |
|---|---|---|---|---|---|---|---|---|
| | | KD(nM) | KD(nM) | pH6.0/7.4 | pH6.0/7.4 | | | |
| 22 | PEG4c | 4.11 × 10^-9 | 2.05 × 10^-7 | 61.58 | 49.93 | 1493.80 | 2 | B |
| 23 | PEG4c | 3.45 × 10^-9 | 3.34 × 10^-8 | 26.28 | 9.67 | 855.36 | 3 | A |
| 24 | PEG4c | 2.01 × 10^-9 | 2.67 × 10^-8 | 21.07 | 13.28 | 869.68 | 3 | A |
| 25 | PEG4c | 3.35 × 10^-9 | 5.79 × 10^-8 | 29.22 | 17.27 | 874.07 | 3 | B |
| 26 | PEG4c | 1.73 × 10^-9 | 1.34 × 10^-8 | 12.10 | 7.76 | 908.40 | 3 | A |
| 27 | PEG4c | 3.03 × 10^-9 | 1.54× 10^-8 | 12.09 | 5.09 | 922.40 | 3 | A |
| 28 | PEG4c | 1.26 × 10^-9 | 1.11 × 10^-8 | 11.37 | 8.81 | 853.71 | 3 | A |
| 29 | PEG4c | 2.17 × 10^-9 | 1.11 × 10^-8 | 13.62 | 5.12 | 867.70 | 3 | A |
| 30 | PEG4c | 1.02 × 10^-8 | 9.56 × 10^-8 | 30.37 | 9.37 | 1493.32 | 2 | B |
| 31 | PEG4c | 2.43 × 10^-9 | 1.71 × 10^-8 | 6.19 | 7.04 | 1486.29 | 2 | B |
| 32 | PEG4c | 2.18 × 10^-9 | 2.70 × 10^-8 | 25.96 | 12.39 | 1367.10 | 2 | B |
| 33 | PEG4c | 8.50 × 10^-9 | 2.21 × 10^-8 | 15.77 | 2.60 | 1395.66 | 2 | B |
| 34 | PEG4c | 5.22 × 10^-9 | 1.49 × 10^-8 | 28.93 | 2.85 | 916.70 | 3 | B |
| 35 | PEG4c | 1.74 × 10^-9 | 3.70 × 10^-8 | 19.32 | 21.26 | 1332.05 | 2 | B |
| 36 | PEG4c | 2.47 × 10^-9 | 4.40× 10^-8 | 25.12 | 17.81 | 1311.00 | 2 | B |
| 37 | PEG4c | 1.54 × 10^-9 | 2.79 × 10^-8 | 33.88 | 18.12 | 1328.06 | 2 | B |
| 38 | PEG4c | 4.75 × 10^-9 | 1.48 × 10^-8 | 12.54 | 3.12 | 887.80 | 3 | B |
| 39 | PEG4c | 1.48 × 10^-9 | 1.24 × 10^-8 | 25.60 | 8.37 | 1346.55 | 2 | B |
| 40 | PEG4c | 4.38 × 10^-8 | 4.26 × 10^-8 | 23.48 | 0.97 | 1392.63 | 2 | B |
| 41 | PEG4c | 1.45 × 10^-8 | 9.14 × 10^-8 | 21.19 | 6.30 | 1005.91 | 3 | B |
| 42 | PEG4c | 5.11 × 10^-9 | 3.44 × 10^-8 | 20.73 | 6.73 | 977.44 | 3 | B |
| 43 | PEG4c | 1.07 × 10^-7 | 6.01 × 10^-7 | 27.60 | 5.62 | 940.20 | 3 | B |

**[Table 1-1-3-1]**

| SEQ ID No. | Peptide ID | Peptide SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 44 | 894_0533 | A | V | MeF3C | V | W7N | N | 3Py6NH2 | F4aao | Eva | I | Q | R | F3CONPEG4c | MeY | C |
| 45 | 894_0535 | A | V | MeF3C | V | W7N | N | 3Py6NH2 | F4aao | Eva | I | Q | R | F3COO | MeF4COO | C |
| 46 | 894_0537 | A | V | MeF3C | V | W7N | N | 3Py6NH2 | F4OMe | Eva | I | Kmor | R | 4Py | MeY | C |
| 47 | 894_0538 | A | V | MeF3C | V | W1aa | N | 3Py6NH2 | F4aao | Eva | I | Q | Kmor | 4Py | MeY | C |
| 48 | 894_0539 | A | V | MeF3C | V | W1aa | N | 3Py6NH2 | F4aao | Eva | I | E(PEG8Me) | R | 4Py | MeY | C |
| 49 | 894_0543 | A | V | MeF3C | V | W1aa | N | 3Py6NH2 | F4aao | Eva | I | Q | Kmor | 4Py | MeF4COO | C |
| 50 | 894_0544 | A | V | MeF3C | V | W1aa | N | 3Py6NH2 | F4aao | Eva | I | E(PEG8Me) | R | 4Py | MeF4COO | C |
| 51 | 894_0547 | A | V | MeF3C | V | W1aa | N | 3Py6NH2 | F4aao(pipzaa) | I | I | Q | Kmor | F3COO | MeF4COO | C |
| 52 | 894_0548 | A | V | MeF3C | V | W1aa | N | 3Py6NH2 | F4aao(pipzaa) | Eva | I | Q | Kmor | F3COO | MeY | C |
| 53 | 894_0552 | A | V | MeF3C | V | 3Imp | N | Y | F4aao | Eva | I | Q | R | F3COO | MeY | C |
| 54 | 894_0574 | A | V | MeF3C | V | W7N | N | Y | F4OMe | Eva | I | Kmor | R | 4Py | MeY | C |
| 55 | 894_0582 | A | V | MeF3C | V | W1aa | N | Y | F4aaopipzaa | Eva | I | Kmor | Kmor | F3COO | MeF4COO | C |
| 56 | 894_0583 | A | V | MeF3C | V | W1aa | N | Y | F4aaopipzaa | Eva | I | Kmor | Kmor | 4Py | MeF4COO | C |
| 57 | 894_0594 | A | V | MeF3C | V | W | N | 3Py6NH2 | F40Me | I | I | K | R | 4Py | MeY | C |
| 58 | 894_0595 | A | V | MeF3C | V | MeW | N | 3Py6NH2 | F4OMe | I | I | K | R | 4Py | MeY | C |
| 59 | 894_0596 | A | V | MeF3C | V | W | N | 3Py6NH2 | F4OMe | I | I | K | R | 4Py | MeF4COO | C |
| 60 | 894_0597 | A | V | MeF3C | V | MeW | N | 3Py6NH2 | F4OMe | I | I | K | R | 4Py | MeF4COO | C |
| 61 | 894_0610 | A | V | MeF3C | V | 3Imp | N | Y | Y | I | I | K | R | Y | MeY | C |
| 62 | 894_0429 | A | V | MeF3C | V | W7N | N | Y | F4aao | I | I | E(PEG8c) | R | 4Py | MeY | C |
| 63 | 894_0434 | A | V | MeF3C | V | W7N | N | Y | F4aao | I | I | E(PEG4c) | R | 4Py | MeY | C |
| 64 | 894_3434 | A | V | MeF | V | W | N | Y | F4aao(PEG8Me) | I | I | R | R | Y | MeY | C |
| 65 | 894_0621 | A | V | MeF3C | V | 31mp | N | 3Py6NH2 | F4OMe | I | I | R | R | 4Py | MeY | C |
| 66 | 894_3427 | A | V | MeF3C | V | W7N | N | 3Py6NH2 | F4OMe | I | I | R | R | 4Py | MeY | C |
| 67 | 894_3426 | A | V | MeF3C | V | 31mp | N | Y | F4aao | Eva | I | Q | R | F3COO | MeY | C |
| 68 | 894 3428 | A | V | MeF3C | V | W7N | N | 3Pv6NH2 | F4OMe | I | I | Kmor | Kmor | 4Py | MeY | C |

**[Table 1--1-3-2]**

| SEQ ID No. | Linker | hTfR SPR pH7.4 | hTfR SPR pH6.0 | dependency koff | dependency KD | m/z | [M+XH]X+ | Analys -s cond -itions |
|---|---|---|---|---|---|---|---|---|
| | | KD(nM) | KD(nM) | pH6.0/7.4 | pH6.0/7.4 | | | |
| 44 | PEG4c | 3.66 × 10^-8 | 3.39 × 10^-7 | 21.28 | 9.26 | 1349.97 | 2 | B |
| 45 | PEG4c | 7.55 × 10^-8 | 8.69 × 10^-7 | 22.41 | 11.51 | 827.40 | 3 | B |
| 46 | PEG4c | 2.65 × 10^-9 | 1.44 × 10^-8 | 18.44 | 5.43 | 812.70 | 3 | A |
| 47 | PEG4c | 1.16 × 10^-7 | 2.49 × 10^-7 | 14.63 | 2.15 | 1254.51 | 2 | B |
| 48 | PEG4c | 1.11 × 10^-7 | 5.36 × 10^-7 | 16.57 | 4.83 | 944.76 | 3 | B |
| 49 | PEG4c | 1.58 × 10^-5 | 5.76 × 10^-7 | 12.22 | 0.04 | 1268.46 | 2 | B |
| 50 | PEG4c | 6.50 × 10^-6 | 1.78 × 10^-6 | 17.69 | 0.27 | 1430.52 | 2 | B |
| 51 | PEG4c | 1.56 × 10^-6 | 6.92 × 10^-7 | 42.98 | 0.44 | 897.36 | 3 | B |
| 52 | PEG4c | 5.82 × 10^-6 | 6.26 × 10^-7 | 32.48 | 0.11 | 892.74 | 3 | B |
| 53 | PEG4c | 2.16 × 10^-8 | 5.30 × 10^-7 | 20.30 | 24.54 | 818.33 | 3 | B |
| 54 | PEG4c | 1.38 × 10^-9 | 9.20 × 10^-9 | 13.66 | 6.67 | 812.37 | 3 | B |
| 55 | PEG4c | 2.64 × 10^-8 | 3.85 × 10^-8 | 13.49 | 1.46 | 925.77 | 3 | B |
| 56 | PEG4c | 1.26 × 10^-8 | 7.20 × 10^-8 | 28.15 | 5.71 | 911.44 | 3 | B |
| 57 | PEG4c | 9.71 × 10^-10 | 5.40 × 10^-9 | 14.31 | 5.56 | 783.91 | 3 | A |
| 58 | PEG4c | 1.34 × 10^-9 | 5.39 × 10^-9 | 8.19 | 4.02 | 788.60 | 3 | A |
| 59 | PEG4c | 1.46 × 10^-9 | 9.57 × 10^-9 | 16.00 | 6.55 | 793.23 | 3 | A |
| 60 | PEG4c | 7.55 × 10^-10 | 4.71 × 10^-9 | 9.02 | 6.24 | 797.88 | 3 | A |
| 61 | PEG4c | 8.61 × 10^-10 | 7.47 × 10^-9 | 15.37 | 8.68 | 1176.36 | 2 | A |
| 62 | PEG4c | 2.85 × 10^-8 | 9.21 × 10^-8 | 13.44 | 3.23 | 940.42 | 3 | B |
| 63 | PEG4c | 2.36 × 10^-8 | 8.77 × 10^-8 | 10.39 | 3.72 | 881.84 | 3 | B |
| 64 | G KN3 | 8.74 × 10^-9 | 3.23 × 10^-8 | 6.94 | 3.70 | 911.32 | 3 | B |
| 65 | PEG4c | 5.43 × 10^-9 | 5.65 × 10^-8 | 14.41 | 10.41 | 793.63 | 3 | A |
| 66 | G KN3 | 2.27 × 10^-9 | 1.61 × 10^-8 | 14.94 | 7.09 | 781.65 | 3 | A |
| 67 | G KN3 | 2.16 × 10^-8 | 4.44 × 10^-7 | 31.32 | 20.56 | 1208.36 | 2 | B |
| 68 | G KN3 | 2.06 × 10^-9 | 1.24 × 10^-8 | 42.54 | 6.02 | 809.62 | 3 | A |

**[Table 1-2]**

| [Table 1-2-1-1] | | | | | | | | | | | | | | | | |
|---|---|---|---|---|---|---|---|---|---|---|---|---|---|---|---|---|
| SEQ ID | Peptide ID | Peptide SEQ | | | | | | | | | | | | | | |
| No. | | 1 | 2 | 3 | 4 | 5 | 6 | 7 | 8 | 9 | 10 | 11 | 12 | 13 | 14 | 15 |
| 69 | 894_0500 | A | V | MeF3C | V | W1aa | N | N Y | F4aao | I | I | Kmor | Kmor | 4Py | MeY | C |
| 70 | 894_0515 | A | V | MeF3C | V | W | N | 3Py6NH2 | F4aao | I | I | E(PEG4Me) | R | F3COO | MeY | C |
| 71 | 894_0517 | A | V | MeF3C | V | W1aa | N | Y | F4aao | I | Har | Q | R | F3COO | MeY | C |
| 72 | 894_0518 | A | V | MeF3C | V | W | N | Y | F4aao | I | I | Q | R | F3COO | MeY | C |
| 73 | 894_0519 | A | V | MeF3C | V | W1EtOH | N | Y | F4aao | I | I | Q | R | F3COO | MeY | C |
| 74 | 894_0520 | A | V | MeF3C | V | W | Nmm | Y | F4aao | I | I | Q | R | F3COO | MeY | C |
| 75 | 894_0521 | A | V | MeF3C | V | W | N | Y | F4aao | Eva | I | Q | R | F3COO | MeY | C |
| 76 | 894_0523 | A | V | MeF3C | V | W1EtOH | N | Y | F4aao | I | T | Q | R | F3COO | MeY | C |
| 77 | 894_0524 | A | T | MeF3C | V | W1EtOH | N | Y | F4aao | Eva | I | Q | R | F3COO | MeY | C |
| 78 | 894_0527 | A | V | MeF3C | V | W1aa | N | Y26dF | F4aao | Eva | I | Q | R | F3COO | MeY | C |
| 79 | 894_0528 | A | V | MeF3C | V | W1Me | N | Y | F4aao | Eva | I | Q | R | F3COO | MeY | C |
| 80 | 894_0530 | A | V | MeF3C | V | W | N | Y | F4OMe | Eva | I | Q | R | F3COO | MeF4COO | C |
| 81 | 894_0531 | A | V | MeF3C | V | W | N | Y | F4OMe | Eva | I | Q | R | F3COO | MeF3COO | C |
| 82 | 894_0536 | A | V | MeF3C | V | W | N | 3Py6NH2 | F4aao | Eva | I | E(PEG8Me) | R | F3COO | MeY | C |
| 83 | 894_0540 | A | V | MeF3C | V | W1aa | N | 3Py6NH2 | F4aao | Eva | I | Kmor | Kmor | F3COO | MeY | C |
| 84 | 894_0541 | A | V | MeF3C | V | W1aa | N | 3Py6NH2 | F4aao | Eva | I | Q | R | F3COO | MeY | C |
| 85 | 894_0542 | A | V | MeF3C | V | W | N | 3Py6NH2 | F4aao | Eva | I | E(PEG8Me) | R | F3COO | MeF4COO | C |
| 86 | 894_0545 | A | V | MeF3C | V | W1aa | N | 3Py6NH2 | F4aao | Eva | I | Kmor | Kmor | F3COO | MeF4COO | C |
| 87 | 894_0546 | A | V | MeF3C | V | W1aa | N | 3Py6NH2 | F4aao | Eva | I | Q | R | F3COO | MeF4COO | C |
| 88 | 894_0549 | A | V | MeF3C | V | W1aa | N | Y | F4aao | Eva | I | Q | R | F3COO | MeY | C |
| 89 | 894_0551 | A | V | MeF3C | V | W1aa | N | Y3Me | F4aao | Eva | I | Q | R | F3COO | MeY | C |
| 90 | 894_0553 | A | V | MeF3C | V | W1aa | N | Y | 3Py6OMe | Eva | I | Q | R | F3COO | MeY | C |
| 91 | 894_0555 | A | V | MeF3C | V | W1aa | N | Y | 3Imp | Eva | I | Q | R | F3COO | MeY | C |
| 92 | 894_0556 | A | V | MeF3C | V | W1aa | N | Y | F4aao | Eva | I | Q | R | F3COO | MeF4C | C |
| 93 | 894_0557 | A | V | MeF3C | V | W1aa | N | Y | F4aao | Eva | I | Q | R | F3COO | MeF4OMe | C |

**[Table 1-2-1-2]**

| SEQ ID No. | Linker | hTfR SPR pH7.4 | hTfR SPR pH6.0 | dependency koff | dependency KD | m/z | [M+XH]X+ | Analysi -s cond -itions |
|---|---|---|---|---|---|---|---|---|
| | | KD(nM) | KD(nM) | pH6.0/7.4 | pH6.0/7.4 | | | |
| 69 | PEG4c | 8.57 × 10^-9 | 3.35 × 10^-8 | 9.63 | 3.91 | 1282.50 | 2 | B |
| 70 | PEG4c | 6.85 × 10^-9 | 4.24 × 10^-8 | 20.00 | 6.19 | 1314.10 | 2 | B |
| 71 | PEG4c | 1.80 × 10^-8 | 7.52 × 10^-8 | 12.78 | 4.18 | 1276.45 | 2 | B |
| 72 | PEG4c | 3.34 × 10^-9 | 6.32 × 10^-9 | 5.72 | 1.89 | 1218.90 | 2 | B |
| 73 | PEG4c | 1.85 × 10^-9 | 4.78 × 10^-9 | 7.54 | 2.58 | 1240.96 | 2 | B |
| 74 | PEG4c | 4.53 × 10^-8 | 7.62 × 10^-8 | 3.53 | 1.68 | 1225.93 | 2 | B |
| 75 | PEG4c | 1.62 × 10^-9 | 6.33 × 10^-9 | 34.44 | 3.91 | 1225.91 | 2 | B |
| 76 | PEG4c | 1.30 × 10^-8 | 1.70 × 10^-8 | 3.37 | 1.31 | 1234.90 | 2 | B |
| 77 | PEG4c | 1.28 × 10^-8 | 2.55 × 10^-8 | 4.46 | 1.99 | 1241.94 | 2 | B |
| 78 | PEG4c | 3.34 × 10^-9 | 4.48 × 10^-9 | 4.38 | 1.34 | 1265.94 | 2 | B |
| 79 | PEG4c | 2.69 × 10^-9 | 3.76 × 10^-9 | 5.31 | 1.40 | 1225.94 | 2 | B |
| 80 | PEG4c | 5.29 × 10^-10 | 5.73 × 10^-9 | 22.20 | 10.83 | 1210.93 | 2 | B |
| 81 | PEG4c | 1.87 × 10^-9 | 9.19 × 10^-9 | 13.49 | 4.91 | 1210.93 | 2 | B |
| 82 | PEG4c | 2.71 × 10^-8 | 2.52 × 10^-7 | 20.39 | 9.30 | 939.85 | 3 | B |
| 83 | PEG4c | 4.37 × 10^-8 | 1.44 × 10^-7 | 32.62 | 3.30 | 1311.05 | 2 | B |
| 84 | PEG4c | 1.91 × 10^-6 | 2.19 × 10^-7 | 17.42 | 0.11 | 1254.86 | 2 | B |
| 85 | PEG4c | 2.34 × 10^-7 | 1.07 × 10^-6 | 14.96 | 4.57 | 949.08 | 3 | B |
| 86 | PEG4c | 1.53 × 10^-6 | 4.06 × 10^-7 | 28.16 | 0.27 | 884.10 | 3 | A |
| 87 | PEG4c | 1.75 × 10^-7 | 4.66 × 10^-7 | 17.51 | 2.66 | 1268.87 | 2 | B |
| 88 | PEG4c | 6.96 × 10^-9 | 5.11 × 10^-8 | 8.60 | 7.34 | 1255.02 | 2 | B |
| 89 | PEG4c | 5.00 × 10^-8 | 2.50 × 10^-7 | 8.08 | 5.00 | 842.01 | 3 | B |
| 90 | PEG4c | 3.94 × 10^-9 | 2.98 × 10^-8 | 8.01 | 7.56 | 823.08 | 3 | B |
| 91 | PEG4c | 5.13 × 10^-9 | 4.49 × 10^-8 | 9.32 | 8.75 | 826.04 | 3 | B |
| 92 | PEG4c | 1.38 × 10^-8 | 4.41 × 10^-8 | 5.47 | 3.20 | 1264.60 | 2 | B |
| 93 | PEG4c | 1.01 × 10^-8 | 4.91 × 10^-8 | 7.99 | 4.86 | 842.02 | 3 | B |

**[Table 1-2-2-1]**

| SEQ ID No. | Peptide ID | Peptide SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 94 | 894_0558 | A | V | MeF3C | V | W1aa | N | Y | F4aao | Eva | I | Q | R | F3COO | MeF3C | C |
| 95 | 894_0559 | A | V | MeF3C | V | W1aa | N | Y | F4aao | Eva | I | Q | R | F3COO | MeF3OMe | C |
| 96 | 894_0560 | A | V | MeF3C | V | W1aa | N | Y | F4aao | Eva | I | Q | R | F3COO | Me4Py | C |
| 97 | 894_0561 | A | V | MeF3C | V | W1aa | N | Y | F4aao | Eva | I | Q | R | F3COO | Me3Py | C |
| 98 | 894_0562 | A | V | MeF3C | V | W1aa | N | Y | F4aao | Eva | I | Q | H | F3COO | MeY | C |
| 99 | 894_0563 | A | V | MeF3C | V | W1aa | N | Y | F4aao | Eva | I | Q | R | Y3Me | MeY | C |
| 100 | 894_0564 | A | V | MeF3C | V | W1aa | N | Y | F4aao | Eva | I | Q | R | 3Py6Me | MeY | C |
| 101 | 894_0565 | A | V | MeF3C | V | W1aa | N | Y | F4aao | Eva | I | Q | R | 3Py6OMe | MeY | C |
| 102 | 894_0566 | A | V | MeF3C | V | W1aa | N | Y | F4aao | Eva | I | Q | R | 3Py6NH2 | MeY | C |
| 103 | 894_0568 | A | V | MeF3C | V | W1aa | N | Y | F4aao | Eva | I | Q | R | 4Py2OMe | MeY | C |
| 104 | 894_0569 | A | V | MeF3C | V | W1aa | N | Y | F4aao | Eva | I | Q | R | 4Py2NH2 | MeY | C |
| 105 | 894_0570 | A | V | MeF3C | V | W1aa | N | Y | F4aao | Eva | I | Q | R | 3Py | MeY | C |
| 106 | 894_0571 | A | V | MeF3C | V | W7N | N | Y | F4aao | Eva | I | QPEG8Me | R | F3COO | MeY | C |
| 107 | 894_0572 | A | V | MeF3C | V | W7N | N | | F4aao | Eva | I | Q | R | F3COO(PEG4c) | MeY | C |
| 108 | 894_0573 | A | V | MeF3C | V | W7N | N | Y | F4aao | Eva | I | Q | R | F3COO | MeF4COO | C |
| 109 | 894_0575 | A | V | MeF3C | V | W1aa | N | Y | F4aao | Eva | I | Kmor | Kmor | F3COO | MeY | C |
| 110 | 894_0576 | A | V | MeF3C | V | W | N | Y | F4aao | Eva | I | Q | R | F3COO(PEG4c) | MeF4COO | C |
| 111 | 894_0577 | A | V | MeF3C | V | W | N | Y | F4OMe | Eva | I | Q | R | F3COO(PEG4c) | MeF4COO | C |
| 112 | 894_0578 | A | V | MeF3C | V | W | N | Y | F4aao | Eva | I | Q | R | F3COO(PEG4c) | MeY | C |
| 113 | 894_0580 | A | V | MeF3C | V | W | N | Y | F4OMe | | I | Q | R | F3COO(PEG4c) | MeF4COO | C |
| 114 | 894_0581 | A | V | MeF3C | V | W7N | N | Y | F4OMe | Eva | I | Kmor | R | 4Py | MeF4COO | C |
| 115 | 894_0584 | A | V | MeF3C | V | W | N | 3Py6NH2 | F4OMe | I | I | Q | R | F3COO(PEG4c) | MeF4COO | C |
| 116 | 894_0585 | A | V | MeF3C | V | W | N | 3Py6NH2 | F4aao | I | I | Q | R | F3COO(PEG4c) | MeF4COO | C |
| 117 | 894_0591 | A | V | MeF | V | MeW | N | Y | Y | Eva | I | K | R | Y | MeY | C |
| 118 | 894_0592 | A | V | MeF | V | MeW | N | Y | Y | I | I | K | R | Y | MeF4COO | C |

**[Table 1-2-2-2]**

| SEQ ID No. | Linker | hTfR SPR pH7.4 KD(nM) | hTfR SPR pH6.0 KD(nM) | dependency koff pH6.0/7.4 | dependency KD pH6.0/7.4 | m/z | [M+XH]X+ | Analysi -s cond -itions |
|---|---|---|---|---|---|---|---|---|
| 94 | PEG4c | 1.20 × 10^-8 | 6.20 × 10^-8 | 7.12 | 5.17 | 1264.21 | 2 | B |
| 95 | PEG4c | 1.06 × 10^-8 | 6.79 × 10^-8 | 9.73 | 6.41 | 1262.38 | 2 | B |
| 96 | PEG4c | 1.82 × 10^-8 | 9.19 × 10^-8 | 7.63 | 5.05 | 1247.48 | 2 | B |
| 97 | PEG4c | 2.26 × 10^-8 | 1.05 × 10^-7 | 7.82 | 4.65 | 831.96 | 3 | B |
| 98 | PEG4c | 2.06 × 10^-8 | 1.45 × 10^-7 | 7.68 | 7.04 | 1245.49 | 2 | B |
| 99 | PEG4c | 9.08 × 10^-9 | 5.62 × 10^-8 | 6.08 | 6.19 | 1247.98 | 2 | B |
| 100 | PEG4c | 1.27 × 10^-8 | 1.13 × 10^-7 | 9.77 | 8.90 | 1240.45 | 2 | B |
| 101 | PEG4c | 8.63 × 10^-9 | 6.67 × 10^-8 | 8.46 | 7.73 | 1248.49 | 2 | B |
| 102 | PEG4c | 9.81 × 10^-9 | 7.64 × 10^-8 | 14.56 | 7.79 | 1240.98 | 2 | B |
| 103 | PEG4c | 1.15 × 10^-8 | 9.50 × 10^-8 | 8.16 | 8.26 | 1248.53 | 2 | B |
| 104 | PEG4c | 1.07 × 10^-8 | 7.82 × 10^-8 | 9.39 | 7.31 | 1240.98 | 2 | B |
| 105 | PEG4c | 1.06 × 10^-8 | 6.79 × 10^-8 | 7.93 | 6.41 | 1233.50 | 2 | B |
| 106 | PEG4c | 2.60 × 10^-8 | 9.01 × 10^-8 | 9.13 | 3.47 | 940.09 | 3 | B |
| 107 | PEG4c | 1.33 × 10^-8 | 3.12 × 10^-8 | 6.37 | 2.35 | 900.38 | 3 | B |
| 108 | PEG4c | 1.39 × 10^-8 | 3.61 × 10^-8 | 5.94 | 2.60 | 827.41 | 3 | B |
| 109 | PEG4c | 6.99 × 10^-9 | 1.11 × 10^-8 | 6.69 | 1.59 | 1311.10 | 2 | B |
| 110 | PEG4c | 7.46 × 10^-9 | 1.28 × 10^-8 | 3.59 | 1.72 | 909.38 | 3 | B |
| 111 | PEG4c | 2.06 × 10^-9 | 3.47 × 10^-9 | 2.68 | 1.68 | 894.74 | 3 | B |
| 112 | PEG4c | 3.53 × 10^-9 | 1.15 × 10^-8 | 5.70 | 3.26 | 900.05 | 3 | B |
| 113 | PEG4c | 7.76 × 10^-10 | 1.43 × 10^-9 | 2.34 | 1.84 | 890.16 | 3 | B |
| 114 | PEG4c | 1.33 × 10^-9 | 1.06 × 10^-8 | 14.51 | 7.97 | 821.70 | 3 | B |
| 115 | PEG4c | 1.84 × 10^-9 | 1.26 × 10^-8 | 16.37 | 6.85 | 890.09 | 3 | B |
| 116 | PEG4c | 8.49 × 10^-9 | 3.52 × 10^-8 | 12.40 | 4.15 | 904.72 | 3 | B |
| 117 | PEG4c | 7.64 × 10^-9 | 1.38 × 10^-8 | 3.29 | 1.81 | 782.12 | 3 | B |
| 118 | PEG4c | 1.11 × 10^-9 | 4.31 × 10^-9 | 4.21 | 3.88 | 786.96 | 3 | B |

**[Table 1-2-3-1]**

| SEQ ID No. | Peptide ID | Peptide SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 119 | 894_0593 | A | V | MeF | V | MeW | N | Y | Y | Eva | I | K | R | Y | MeF4COO | C |
| 120 | 894_0598 | A | V | MeF | V | MeW | N | Y | Y | Eva | I | K | R | F3COO(PEG4c) | MeY | C |
| 121 | 894_0599 | A | V | MeF | V | MeW | N | Y | Y | | I | K | R | F3COO(PEG4c) | MeF4COO | C |
| 122 | 894_0600 | A | V | MeF | V | MeW | N | Y | Y | Eva | I | K | R | F3COO(PEG4c) | MeF4COO | C |
| 123 | 894_0601 | A | V | MeF | V | W | N | 3Py6NH2 | Y | I | I | K | R | Y | MeY | C |
| 124 | 894_0602 | A | V | MeF | V | MeW | N | 3Py6NH2 | Y | I | I | K | R | Y | MeY | C |
| 125 | 894_0603 | A | V | MeF | V | W | N | 3Py6NH2 | Y | I | I | K | R | Y | MeF4COO | C |
| 126 | 894_0604 | A | V | MeF | V | MeW | N | 3Py6NH2 | Y | I | I | K | R | Y | MeF4COO | C |
| 127 | 894_0605 | A | V | MeF3C | V | W1Me | N | Y | Y | Eva | I | K | R | 4Py | MeY | C |
| 128 | 894_0606 | A | V | MeF3C | V | W1Me | N | Y | F4OMe | Eva | I | K | R | 4Py | MeY | C |
| 129 | 894_0607 | A | V | MeF3C | V | W1Me | N | Y | Y | I | I | K | R | 4Py | MeY | C |
| 130 | 894_0608 | A | V | MeF3C | V | W1Me | N | Y | F4OMe | I | I | K | R | 4Py | MeY | C |
| 131 | 894_0611 | A | V | MeF | V | MeW | N | Y | Y | Eva | S | K | R | Y | MeY | C |
| 132 | 894_0612 | A | V | MeF3C | V | MeW | N | Y | Y | Eva | S | K | R | Y | MeY | C |
| 133 | 894_0613 | A | V | MeF | V | MeW | N | 3Py6NH2 | Y | Eva | I | K | R | Y | MeY | C |
| 134 | 894_0614 | A | V | MeF3C | V | MeW | N | 3Py6NH2 | Y | Eva | I | K | R | Y | MeY | C |
| 135 | 894_0615 | A | V | Me3Py | V | MeW | N | Y | Y | I | I | K | R | Y | MeY | C |
| 136 | 894_0616 | A | V | Me3Py | V | MeW | N | Y | Y | Eva | I | K | R | Y | MeY | C |
| 137 | 894_0619 | A | V | MeF | V | MeW | N | 3Py6NH2 | Y | I | s | K | R | Y | MeY | C |
| 138 | 894_0620 | A | V | MeF3C | V | MeW | N | 3Py6NH2 | Y | I | s | K | R | Y | MeY | C |
| 139 | 894_0430 | A | V | MeF3C | V | W7N | N | Y | F4aao | I | I | Q | R | E(PEG8c) | MeY | C |
| 140 | 894_0431 | A | V | MeF3C | V | W7N | N | Y | F4aao | I | I | Q | R | F3COO(PEG8c) | MeY | C |
| 141 | 894_0432 | A | V | MeF3C | V | W7N | N | Y | F4OMe | I | I | E(PEG8c) | R | E(PEG8c) | MeY | C |
| 142 | 894_0433 | A | V | MeF3C | V | W7N | N | Y | F4OMe | I | I | E(PEG8c) | R | F3COO(PEG8c) | MeY | C |
| 143 | 894_0435 | A | V | MeF3C | V | W7N | N | Y | F4aao | I | I | Q | R | E(PEG4c) | MeY | C |
| 144 | 894_0436 | A | V | MeF3C | V | W7N | N | Y | F4aao | I | I | Q | R | F3COO(PEG4c) | MeY | C |
| 145 | 894_0437 | A | V | MeF3C | V | W7N | N | Y | F4OMe | I | I | E(PEG4c) | R | E(PEG4c) | MeY | C |
| 146 | 894_0438 | A | V | MeF3C | V | W7N | N | Y | F4OMe | I | I | E(PEG4c) | R | F3COO(PEG4c) | MeY | C |
| 147 | 894_3435 | A | V | MeF | V | W | N | Y | Y | I | I | E(PEG8Me) | R | Y | MeY | C |
| 148 | 894_3436 | A | V | MeF | V | W | N | Y | Y | I | I | R | R | F3COO(PEG8Me) | MeY | C |
| 149 | 894 3437 | A | V | MeF3C | V | W | N | 3Py6NH2 | F4aao | I | I | Q | R | F3COO | MeY | C |

**[Table 1-2-3-2]**

| SEQ ID No. | Linker | | hTfR SPR pH7.4 KD(nM) | hTfR SPR pH6.0 KD(nM) | dependency koff pH6.0/7.4 | dependency kKD pH6.0/7.4 | m/z | [M+XH]X+ | Analysi -s cond -itions |
|---|---|---|---|---|---|---|---|---|---|
| 119 | PEG4c | | 3.61 × 10^-9 | 9.52 × 10^-9 | 3.72 | 2.64 | 791.61 | 3 | B |
| 120 | PEG4c | | 5.80 × 10^-9 | 1.32 × 10^-8 | 3.40 | 2.28 | 874.04 | 3 | B |
| 121 | PEG4c | | 1.84 × 10^-9 | 7.50 × 10^-9 | 5.54 | 4.08 | 878.60 | 3 | B |
| 122 | PEG4c | | 5.19 × 10^-9 | 1.53 × 10^-8 | 3.83 | 2.95 | 883.26 | 3 | B |
| 123 | PEG4c | | 5.30 × 10^-9 | 1.74 × 10^-8 | 8.89 | 3.28 | 1158.69 | 2 | B |
| 124 | PEG4c | | 6.37 × 10^-9 | 1.89 × 10^-8 | 6.50 | 2.97 | 777.45 | 3 | A |
| 125 | PEG4c | | 7.31 × 10^-9 | 3.50 × 10^-8 | 11.35 | 4.79 | 1172.64 | 2 | A |
| 126 | PEG4c | | 3.51 × 10^-9 | 1.67 × 10^-8 | 7.83 | 4.76 | 786.95 | 3 | A |
| 127 | PEG4c | | 1.57 × 10^-9 | 5.22 × 10^-9 | 7.58 | 3.32 | 1182.36 | 2 | B |
| 128 | PEG4c | | 1.10 × 10^-9 | 3.23 × 10^-9 | 6.68 | 2.94 | 793.43 | 3 | B |
| 129 | PEG4c | | 5.46 × 10^-10 | 2.04 × 10^-9 | 7.49 | 3.74 | 784.05 | 3 | B |
| 130 | PEG4c | | 6.78 × 10^-10 | 1.74 × 10^-9 | 5.96 | 2.57 | 788.77 | 3 | B |
| 131 | PEG4c | | 3.04 × 10^-8 | 6.63 × 10^-8 | 4.83 | 2.18 | 773.66 | 3 | B |
| 132 | PEG4c | | 5.67 × 10^-9 | 1.32 × 10^-8 | 6.71 | 2.33 | 785.07 | 3 | B |
| 133 | PEG4c | | 1.65 × 10^-8 | 2.02 × 10^-8 | 3.75 | 1.22 | 782.31 | 3 | B |
| 134 | PEG4c | | 4.58 × 10^-9 | 3.95 × 10^-9 | 4.01 | 0.86 | 793.64 | 3 | B |
| 135 | PEG4c | | 1.66 × 10^-8 | 1.53 × 10^-8 | 2.87 | 0.92 | 777.77 | 3 | B |
| 136 | PEG4c | | 4.17 × 10^-8 | 5.08 × 10^-8 | 3.41 | 1.22 | 782.50 | 3 | B |
| 137 | PEG4c | | 2.76 × 10^-8 | 1.26 × 10^-7 | 12.22 | 4.57 | 768.78 | 3 | A |
| 138 | PEG4c | | 4.97 × 10^-9 | 1.62 × 10^-8 | 12.52 | 3.26 | 1169.86 | 2 | A |
| 139 | PEG4c | | 1.07 × 10^-7 | 5.24 × 10^-7 | 10.59 | 4.90 | 933.91 | 3 | B |
| 140 | PEG4c | | 5.63 × 10^-9 | 2.21 × 10^-8 | 8.23 | 3.93 | 954.59 | 3 | B |
| 141 | PEG4c | | 1.42 × 10^-7 | 2.12 × 10^-7 | 7.39 | 1.49 | 1060.73 | 3 | B |
| 142 | PEG4c | | 2.15 × 10^-9 | 1.78 × 10^-8 | 15.28 | 8.28 | 1081.40 | 3 | B |
| 143 | PEG4c | | 5.91 × 10^-8 | 2.71 × 10^-7 | 9.77 | 4.59 | 875.15 | 3 | B |
| 144 | PEG4c | | 3.56 × 10^-9 | 1.74 × 10^-8 | 9.40 | 4.89 | 895.81 | 3 | B |
| 145 | PEG4c | | 4.85 × 10^-8 | 1.99 × 10^-7 | 9.27 | 4.10 | 943.24 | 3 | B |
| 146 | PEG4c | | 2.75 × 10^-9 | 1.14 × 10^-8 | 10.23 | 4.15 | 963.85 | 3 | B |
| 147 | G | KN3 | 1.16 × 10^-8 | 4.12 × 10^-8 | 6.89 | 3.55 | 1323.94 | 2 | B |
| 148 | G | KN3 | 2.00 × 10^-9 | 6.72 × 10^-9 | 5.75 | 3.36 | 901.10 | 3 | B |
| 149 | G | KN3 | 3.57 × 10^-9 | 2.81 × 10^-8 | 23.09 | 7.87 | 1200.89 | 2 | B |

**[Table 1-3]**

| [Table 1-3-1-1] | | | | | | | | | | | | | | | | |
|---|---|---|---|---|---|---|---|---|---|---|---|---|---|---|---|---|
| SEQ ID No. | Peptide ID | Peptide SEQ | | | | | | | | | | | | | | |
| | | 1 | 2 | 3 | 4 | 5 | 6 | 7 | 8 | 9 | 10 | 11 | 12 | 13 | 14 | 15 |
| 168 | 894_0636 | A | V | MeF3C | V | MeW | N | Y | F4OMe | I | I | K | R | F | MeY | C |
| 169 | 894_0637 | A | V | MeF3C | V | MeW | N | Y | F4OMe | I | I | Q | R | F | MeY | C |
| 170 | 894_0638 | A | V | MeF3C | V | MeW | N | 3Py6NH2 | F4OMe | I | I | Q | R | F | MeY | C |
| 171 | 894_0639 | A | V | MeF3C | V | MeW | N | Y | F4OMe | I | I | E(apa) | R | F | MeY | C |
| 172 | 894_0640 | A | V | MeF3C | V | MeW | N | 3Py6NH2 | F4OMe | I | I | E(apa) | R | F | MeY | C |
| 173 | 894_0641 | A | V | MeF3C | V | MeW | N | R | F4OMe | I | I | K | R | F | MeY | C |
| 174 | 894_0642 | A | V | MeF3C | V | MeW | N | R | F4OMe | I | I | Q | R | F | MeY | C |
| 175 | 894_0643 | A | V | MeF3C | V | MeW | N | R | F4OMe | I | I | E(apa) | R | F | MeY | C |
| 176 | 894_0644 | A | V | MeF3C | V | MeW | N | Y | F4aao | I | I | K | R | F | MeY | C |
| 177 | 894_0645 | A | V | MeF3C | V | MeW | N | Y | F4aao | I | I | K | R | F3COO | MeY | C |
| 178 | 894_0646 | A | V | MeF3C | V | MeW | N | Y | F4aao | I | I | K | R | F3COO | MeF4COO(PEG4c) | C |
| 179 | 894_0647 | A | V | MeF3C | V | MeW | N | Y | F4aao | I | I | K | R | F | MeF4COO(PEG4c) | C |
| 180 | 894_0648 | A | V | MeF3C | V | MeW | N | Y | F4OMe | I | I | K | R | F3COO | MeF4COO(PEG4c) | C |
| 181 | 894_3489 | A | V | MeF3C | V | 3lmp | N | 3Py6NH2 | F4OMe | Eva | I | R | R | 4Py | MeF4COO | C |
| 182 | 894_3490 | A | V | MeF | V | W | N | Y | Y | I | I | R | R | Y | MeF4COO | C |

**[Table 1-3-1-2]**

| SEQ ID No. | Linker | | | hTfR SPR pH7.4 KD(nM) | hTfR SPR pH6.0 KD(nM) | dependen cy koff - pH6.0/7.4 | dependen -cy KD pH6.0/7.4 | m/z | [M+XH]X+ | Analysi -s conc -itions |
|---|---|---|---|---|---|---|---|---|---|---|
| 168 | PEG4c | NH2 | | 2.26 × 10^-10 | 8.10 × 10^-10 | 5.55 | 3.58 | 1181.87 | 2 | B |
| 169 | PEG4c | NH2 | | 8.59 × 10^-10 | 4.86 × 10^-9 | 9.23 | 5.66 | 1181.86 | 2 | B |
| 170 | PEG4c | NH2 | | 1.34 × 10^-9 | 7.61 × 10^-9 | 12.27 | 5.68 | 788.31 | | B |
| 171 | PEG4c | NH2 | | 1.10 × 10^-10 | 6.78 × 10^-10 | 13.80 | 6.12 | 807.43 | 3 | B |
| 172 | PEG4c | NH2 | | 2.15 × 10^-10 | 2.59 × 10^-9 | 24.82 | 12.05 | 807.44 | 3 | B |
| 173 | PEG4c | NH2 | | 3.81 × 10^-9 | 1.33 × 10^-8 | 3.44 | 3.49 | 1178.39 | 2 | B |
| 174 | PEG4c | NH2 | | 2.06 × 10^-8 | 7.55 × 10^-8 | 4.85 | 3.67 | 785.98 | 3 | B |
| 175 | PEG4c | NH2 | | 3.04 × 10^-9 | 1.22 × 10^-8 | 5.14 | 4.01 | 805.08 | 3 | B |
| 176 | PEG4c | NH2 | | 3.22 × 10^-10 | 7.64 × 10^-10 | 4.92 | 2.37 | 1204.04 | 2 | B |
| 177 | PEG4c | NH2 | | 1.34 × 10^-10 | 2.26 × 10^-9 | 34.80 | 16.79 | 1225.97 | 2 | B |
| 178 | PEG4c | NH2 | | 3.91 × 10^-10 | 3.10 × 10^-9 | 12.99 | 7.93 | 909.45 | 3 | B |
| 179 | PEG4c | NH2 | | 1.42 × 10^-10 | 7.60 × 10^-10 | 9.27 | 5.35 | 894.71 | 3 | B |
| 180 | PEG4c | NH2 | | 2.29 × 10^-10 | 9.57 × 10^-10 | 5.85 | 4.18 | 894.78 | 3 | B |
| 181 | G | KN3 | NH2 | 2.45 × 10^-8 | 1.24 × 10^-7 | 5.23 | 5.06 | 795.68 | 3 | A |
| 182 | G | KN3 | NH2 | 9.21 × 10^-8 | 3.47 × 10^-7 | 2.14 | 3.77 | 777.18 | 3 | A |

**[Table 1-3-2-1]**

| SEQ ID No. | Peptide ID | Peptide SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 183 | 894_3491 | A | V | MeF | V | W | N | R | Y | I | I | R | R | F3COO(PEG4c) | MeF4COO | C |
| 184 | 894_3492 | A | V | MeF3C | V | 3lmp | N | 3Py6NH2 | F4OMe | Eva | I | R | R | 4Py | MeF4COO | C |
| 185 | 894_3497 | A | V | MeF3C | V | 3lmp | N | 3Py6NH2 | F4OMe | I | I | R | R | 4Py | MeY | C |
| 186 | 894_3498 | A | V | MeF | V | MeW | N | 3Py6NH2 | Y | I | I | K | R | Y | MeF4COO | C |
| 187 | 894_3499 | A | V | MeF | V | MeW | N | 3Py6NH2 | Y | Eva | I | K | R | Y | MeF4COO | C |
| 188 | 894_3500 | A | V | MeF | V | MeW | N | 3Py6NH2 | Y | Eva | I | K | R | Y | MeF4COO | C |
| 189 | 894_3503 | A | V | MeF | V | 3lmp | N | 3Py6NH2 | F4OMe | I | I | R | R | 4Py | MeF4COO | C |
| 190 | 894_3504 | A | V | MeF | V | 3lmp | N | 3Py6NH2 | F4OMe | I | I | R | R | 4Py | MeY | C |
| 191 | 894_3447 | A | V | MeF3C | V | W | N | 3Py6NH2 | F4OMe | I | I | R | R | 4Py | MeY | C |
| 192 | 894_3448 | A | V | MeF3C | V | W7N | N | 3Py6NH2 | F4OMe | I | I | Kmor | Kmor | 4Py | MeY | C |
| 193 | 894_3488 | A | V | MeF | V | MeW | N | Y | Y | I | I | K | R | F3COO(PEG4c) | MeF4COO | C |
| 194 | 894_3485 | A | V | MeF | V | MeW | N | Y | Y | Eva | I | K | R | F3COO(PEG4c) | MeF4COO | C |
| 195 | 894_3484 | A | V | MeF | V | MeW | N | Y | Y | Eva | I | K | R | F3COO(PEG4c) | MeY | C |
| 196 | 894_3486 | A | V | MeF | V | MeW | N | 3Py6NH2 | Y | I | I | K | R | Y | MeF4COO | C |
| 197 | 894_3487 | A | V | MeF3C | V | 3lmp | N | 3Py6NH2 | F4OMe | I | I | R | R | 4Pv | MeY | C |

**[Table 1-3-2-2]**

| SEQ ID No. | Linker | | | | | | | | hTfR SPR pH7.4 KD(nM) | hTfR SPR pH6.0 KD(nM) | dependenc -y koff pH6.0/7.4 | dependen -cy KD pH6.0/7.4 | m/z | [M+XH]X+ | Analysi -s cond -itions |
|---|---|---|---|---|---|---|---|---|---|---|---|---|---|---|---|
| 183 | G | KN3 | NH2 | | | | | | 2.14 × 10^-7 | 7.99 × 10^-7 | 2.44 | 3.73 | 868.96 | 3 | A |
| 184 | G | de | de | de | ds | ds | KN3 | NH2 | 1.36 × 10^-7 | 1.21 × 10^-7 | 0.19 | 0.89 | 982.78 | 3 | A |
| 185 | G | de | de | de | ds | ds | KN3 | NH2 | 2.87× 10^-8 | 3.81 × 10^-7 | 15.95 | 13.28 | 1452.72 | 2 | A |
| 186 | G | de | de | de | ds | ds | KN3 | NH2 | 1.88 × 10^-8 | 1.45 × 10^-7 | 10.84 | 7.71 | 1442.46 | 2 | A |
| 187 | G | KN3 | NH2 | | | | | | 0.99 × 10^-8 | 3.07 × 10^-8 | 4.06 | 3.07 | 779.56 | 3 | B |
| 188 | G | de | de | de | ds | ds | KN3 | NH2 | 3.40 × 10^-8 | 1.75 × 10^-7 | 7.17 | 5.15 | 1449.24 | 2 | A |
| 189 | G | KN3 | NH2 | | | | | | 8.12 × 10^-8 | 3.84 × 10^-7 | 5.10 | 4.73 | 779.55 | 3 | A |
| 190 | G | KN3 | NH2 | | | | | | 5.71 × 10^-8 | 1.77 × 10^-7 | 2.30 | 3.10 | 1154.76 | 2 | A |
| 191 | G | G | G | G | S | S | KN3 | NH2 | 8.90 × 10^-10 | 3.82 × 10^-9 | 8.79 | 4.29 | 896.40 | 3 | A |
| 192 | G | G | G | G | S | S | KN3 | NH2 | 1.49 × 10^-9 | 1.05 × 10^-8 | 27.09 | 7.05 | 924.78 | 3 | A |
| 193 | G | KN3 | NH2 | | | | | | 1.60× 10^-9 | 5.15 × 10^-9 | 5.15 | 3.22 | 1299.41 | 2 | B |
| 194 | G | KN3 | NH2 | | | | | | 4.62 × 10^-9 | 1.49 × 10^-8 | 4.44 | 3.23 | 1306.42 | 2 | B |
| 195 | G | KN3 | NH2 | | | | | | 5.77 × 10^-9 | 1.48 × 10^-8 | 4.33 | 2.56 | 1292.43 | 2 | B |
| 196 | G | KN3 | | NH2 | | | | | 2.94 × 10^-9 | 1.39 × 10^-8 | 8.92 | 4.73 | 1161.62 | 2 | B |
| 197 | G | KN3 | NH2 | | | | | | 5.09 × 10^-9 | 4.39 × 10^-8 | 6.70 | 8.62 | 1171.93 | 2 | A |

**[Table 2-1]**

| [Table 2-1-1] | | | | | | | | | | | | | | | | |
|---|---|---|---|---|---|---|---|---|---|---|---|---|---|---|---|---|
| SEQ ID No. | Peptide ID | Amino Acid Sequece | | | | | | | | | | | | | | |
| | | 1 | 2 | 3 | 4 | 5 | 6 | 7 | 8 | 9 | 10 | 11 | 12 | 13 | 14 | 15 |
| 150 | 894_0550 | A | V | MeF3C | | W1aa | N | 3Py6NHaa | F4aao | Eva | I | Q | R | F3COO | MeY | C |
| 151 | 894_0554 | A | V | MeF3C | V | W1aa | N | Y | 3Py6NHaa | Eva | I | Q | R | F3COO | MeY | C |
| 152 | 894_0567 | A | V | MeF3C | V | W1aa | N | Y | F4aao | Eva | I | Q | R | 4Py2Me | MeY | C |
| 153 | 894_475 | A | V | MeF3C | V | W7N | N | 3Py6NH2 | F4aao(pipzaa) | I | I | E(PEG8Me) | R | 4Py | MeY | C |
| 154 | 894_476 | A | V | MeF3C | V | W7N | N | 3Py6NH2 | F4aao(pipzaa) | I | I | E(PEG8Me) | Kmor | 4Py | MeY | C |
| 155 | 894_478 | A | V | MeF3C | V | W1aa | N | 3Py6NH2 | F4aao(pipzaa) | I | I | E(PEG8Me) | Kmor | 4Py | MeY | C |
| 156 | 894_481 | A | V | MeF3C | V | W7N | N | 3Py6NH2 | F4aao(pipzaa) | I | I | KCOpipzaa | R | 4Py | MeY | C |
| 157 | 894_482 | A | V | MeF3C | V | W7N | N | 3Py6NH2 | F4aao(pipzaa) | I | I | KCOpipzaa | Kmor | 4Py | MeY | C |
| 158 | 894_484 | A | V | MeF3C | V | W1aa | N | 3Py6NH2 | F4aao(pipzaa) | I | I | KCOpipzaa | R | 4Py | MeY | C |
| 159 | 894_489 | A | V | MeF3C | V | W7N | N | 3Py6NH2 | F4aao(pipzaa) | I | I | E(PEG8Me) | R | F3COO | MeY | C |
| 160 | 894_492 | A | V | MeF3C | V | W1aa | N | 3Py6NH2 | F4aao(pipzaa) | I | I | E(PEG8Me) | R | 4Py | MeY | C |
| 161 | 894_0534 | A | V | MeF3C | V | W7N | N | 3Pv6NH2 | F4aao | Eva | I | E(PEG8Me) | R | F3COO | MeF4COO | C |

**[Table 2-1-2]**

| SEQ ID No. | Linker | hTfR SPR pH7.4 | hTfR SPR pH6.0 | m/z | [M+XH]X+ | Analysis conditions |
|---|---|---|---|---|---|---|
| | | KD(n M) | KD(nM) | | | |
| 150 | PEG4c | LS | 5.89 × 10^-8 | 856.67 | 3 | B |
| 151 | PEG4c | LS | 4.82 × 10^-8 | 837.39 | 3 | B |
| 152 | PEG4c | LS | 1.16 × 10^-7 | 1240.48 | 2 | B |
| 153 | PEG4c | 4.43 × 10^-8 | LS | 722.65 | 4 | A |
| 154 | PEG4c | 3.26 × 10^-8 | LS | 977.25 | 3 | A |
| 155 | PEG4c | 2.84 × 10^-8 | LS | 996.13 | 3 | B |
| 156 | PEG4c | 1.60 × 10^-8 | LS | 897.74 | 3 | A |
| 157 | PEG4c | 1.50 × 10^-8 | LS | 1367.13 | 2 | A |
| 158 | PEG4c | 2.02 × 10^-8 | LS | 1374.65 | 2 | A |
| 159 | PEG4c | 6.55 × 10^-9 | LS | 977.47 | 3 | B |
| 160 | PEG4c | 2.08 × 10^-8 | LS | 982.19 | 3 | B |
| 161 | PEG4c | 1.22 × 10^-5 | LS | 949.79 | 3 | A |

**[Table 2-2]**

| SEQ ID No. | Peptide ID | Peptide SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 198 | 894_3493 | A | V | MeF | V | W | N | R | Y | I | I | R | R | Y | MeF4COO | C |
| 199 | 894_3495 | A | V | MeF | V | W | N | R | Y | I | I | R | R | Y | MeY | C |
| 200 | 894_3496 | A | V | MeF3C | V | 3lmp | N | 3Py6NH2 | F4OMe | I | I | R | R | 4Py | MeF4COO | C |
| 201 | 894_3501 | A | V | MeF | V | 3lmp | N | 3Py6NH2 | F4OMe | I | I | R | R | 4Py | MeF4COO | C |
| 202 | 894_3502 | A | V | MeF | V | 3lmp | N | 3Py6NH2 | F4OMe | I | I | R | R | 4Py | MeY | C |

| SEQ ID No. | Linker | | | | | | | | _hTfR SPR pH7.4 | hTfR SPR pH6.0 | m/z | [M+XH]X+ | Analys -is con -ditions |
|---|---|---|---|---|---|---|---|---|---|---|---|---|---|
| | | | | | | | | | KD(nM) | KD(nM) | | | |
| 198 | G | de | de | de | ds | ds | KN3 | NH2 | 1.04 × 10^-6 | LS | 1446.04 | 2 | A |
| 199 | G | de | de | de | ds | ds | KN3 | NH2 | 5.31 × 10^-7 | LS | 954.93 | 3 | A |
| 200 | G | de | de | de | ds | ds | KN3 | NH2 | 8.16 × 10^-8 | LS | 1466.74 | 2 | A |
| 201 | G | de | de | de | ds | ds | KN3 | NH2 | 2.12 × 10^-7 | LS | 966.62 | 3 | A |
| 202 | G | de | de | de | ds | ds | KN3 | NH2 | 5.15 × 10^-7 | LS | 1435.46 | 2 | A |

**[Table 3]**

| SEQ ID No. | Peptide ID | Amino Acid Sequece | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 162 | 894_477 | A | V | MeF3C | V | W7N | N | 3Py6NH2 | F4aao(pipzaa) | I | I | E(PEG8Me) | Kmor | F3COO | MeY | C |
| 163 | 894_0522 | A | V | MeF3C | V | W1aa | N | Y | F4aao | I | Kmor | Q | R | F3COO | MeY | C |
| 164 | 894_0525 | A | V | MeF3C | V | W1aa | N | Y | F4aao | I | Mor | Q | R | F3COO | MeY | C |
| 165 | 894_0526 | A | V | MeF3C | V | W1aa | N | Y | F4aao | I | Hpr | Q | R | F3COO | MeY | C |
| 166 | 894_0529 | A | Kmor | MeF3C | V | W | N | Y | F4aao | I | Kmor | Q | R | F3COO | MeY | C |
| 167 | 894_0579 | A | V | MeF3C V | | W | N | Y | F4aao | I | I | Q | R | F3COO(PEG4c) | MeF4COO | C |

| SEQ ID No. | Linker | hTfR SPR pH7.4 KD(nM) | hTfR SPR pH6.0 KD(nM) | m/z | [M+XH]X+ | Analysis conditions |
|---|---|---|---|---|---|---|
| 162 | PEG4c | LS | ND | 991.43 | 3 | B |
| 163 | PEG4c | LS | ND | 860.68 | 3 | B |
| 164 | PEG4c | LS | ND | 1248.00 | 2 | B |
| 165 | PEG4c | LS | ND | 1246.95 | 2 | B |
| 166 | PEG4c | LS | ND | 874.79 | 3 | A |
| 167 | PEG4c | 4.51×10^-9 | 2.51 × 10^-9 | 904.69 | 3 | B |

### [Example 3]

### Evaluation of Molecular Interactions Between Transferrin Receptor (hTfR) and Peptides by Surface Plasmon Resonance (SPR)

Molecular interactions between the synthesized peptides and the transferrin receptor (hTfR) were evaluated by surface plasmon resonance (SPR) using the following method. The specific assay procedure is described below.

### [SPR measurement]

### SPR measurement under pH 6.0 conditions

An NTA sensor chip (Cytiva) was inserted in a Biacore T200 (Cytiva), a priming operation was performed 3 times with a running buffer: HBS-P+ pH 6.0 (Cytiva), and an equilibrium state was created at a flow rate of 30 µL/min. A ligand immobilization operation was carried out at a flow rate of 10 µL/min.

The surface of the NTA sensor chip was washed with 350 mM EDTA solution, nickel ions were loaded into the carbomethyldextran chains of the sensor chip with a 500 µM aqueous nickel chloride solution, and then washing was performed with 3 mM EDTA solution. After mixing 50 µL of each of 60 mM EDC solution (Cytiva) and 650 mM NHS solution (Cytiva), the mixture was reacted for 420 seconds at a flow rate of 10 µL/min. 150 µL of 0.2 µM hTfR solution was prepared by diluting with 10 mM acetic acid solution (pH 5.0), and reacted for 420 seconds at a flow rate of 10 µL/min to immobilize hTfR on the NTA sensor chip. For the human transferrin receptor (hTfR), the recombinant hTfR described in Example 2 of WO2018/124121 was used. After immobilization, capping was performed by reacting with 1.0 M ethanolamine aqueous solution (Cytiva) at a flow rate of 10 µL/min for 420 seconds. Peptide lysate prepared in DMSO to 10 mM was diluted with the running buffer to a final concentration of 10 µM peptide lysate, and then peptide solutions of 250 nM, 125 nM, 62.5 nM, 25 nM, and 12.5 nM were prepared. The kinetics of the peptide against hTfR was obtained by SPR measurement using the above samples.

Or, SPR measurement under pH 7.4 conditions was performed by the following method.

An CM3 sensor chip (Cytiva) was inserted into BiacoreT200 (Cytiva), then a priming operation was performed three times by using a running buffer: HBS-P+, pH7.4 (Cytiva), and an equilibrium state was created at a flow rate of 30 µm/min. Ligand immobilization was performed at a flow rate of 10 µL/min. After mixing 50 µL each of 60 mM EDC solution (Cytiva) and 650 mM NHS solution (Cytiva), the mixture was reacted at a flow rate of 10 µL/min for 420 seconds. 150 µL of 20 µg/mg THE His Tag Antibody, mAb, Mouse (GenScript) was prepared by diluting with 10 mM acetic acid solution (pH 5.0), and reacted at a flow rate of 10 µL/ min for 420 seconds. Then, THE His Tag Antibody, mAb, Mouse (GenScript) was immobilized as a capture molecule on the CM3 sensor chip. After immobilization, capping was performed by reacting with 1.0 M ethanolamine solution (Cytiva) at a flow rate of 10 µL/min for 420 seconds. 300 nM hTfR was captured at a flow rate of 10 µL/min, a peptide solution prepared at 10 mM in DMSO was diluted with running buffer to achieve a final concentration of 10 µM, and peptide solutions at 250 nM, 125 nM, 62.5 nM, 25 nM, and 12.5 nM were prepared. . The peptide samples were reacted at a flow late of 30 µL/min for 120 seconds, followed by dissociation for 600 seconds. The kinetics of the peptide against hTfR was obtained by SPR measurement for the above samples.

### Measurement of koff and KD (nM)

The kinetic evaluation model was single-cycle kinetics, and curve fitting was performed using Biacore T200 Evaluation Software Version 3.0 (Cytiva). The binding of the peptide to hTfR was evaluated by performing curve fitting on the obtained sensorgram by the least squares method and obtaining the kon value, the koff value, and the KD value. The KD values thus obtained are shown in Tables 1-1, 1-2, 1-3, Table 2-1, 2-2, and Table 3. In the tables, LS stands for Low Signal, indicating that TfR biding activity was low at equal to or less than the measurement threshold value, and ND stands for No Data.

The results show that the synthesized special cyclic peptides listed in Tables 1-1, 1-2, 1-3, and Table 2-1, 2-2 (and Table 3) have binding ability to hTfR.

### Evaluation of pH dependence

The ratio of the calculated koff or KD values under pH 6.0 conditions to those under pH 7.4 conditions was calculated to check pH dependency. The koff comparison values (pH 6.017.4) and KD comparison values (pH 6.0/7.4) obtained in this way are shown in Tables 1-1 and 1-2.

The results show that the synthesized special cyclic peptides listed in Tables 1-1 1-2 and 1-3 bind to hTfR in a pH-dependent manner. Further, the special cyclic peptides listed in Tables 2-1 and 2-2 showed TfR binding activity only under pH 6.0 conditions or pH 7.4 conditions, indicating that the special cyclic peptides in Tables 2-1 and 2-2 also have pH dependence.

### [Example 4]

The following peptides or peptides with a linker were synthesized.

### [Example 4-1]

### Synthesis of 894_0462 (SEQ ID NO: 1)

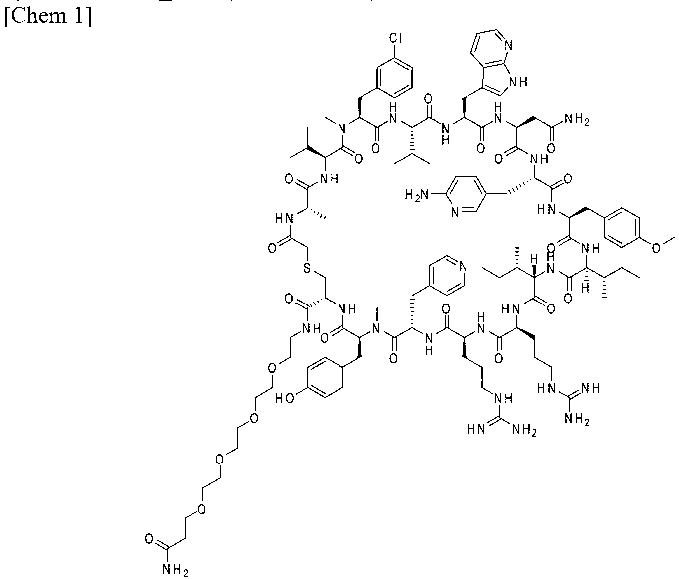

The target peptide was synthesized using Sieber amide resin (Watanabe Chemical Industries, 0.48 mmol/g, 0.10 g), starting with the removal of the Fmoc group by the general method described above. In this process, Liberty Blue from CEM was used as a solid phase synthesizer, and the synthesis was performed according to the manufacturer's manual. For the introduction of each residue, elongation from the 16th residue to the 13th residue was performed using Fmoc-AA/DIPCI/Oxyma Pure (4.2 equivalents/8 equivalents/4 equivalents) per 1 equivalent of resin by reacting once for 10 min at 75°C in DMF. Elongation from the 12th residue to the 1st residue was performed using Fmoc-AA/DIPCI/Oxyma Pure (5.3 equivalents/15 equivalents/7.5 equivalents) by reacting once for 3 min at 90°C in DMF. However, the 3rd residue was reacted twice at 75°C for 15 min. The 11th and 12th residues were reacted twice at 50°C for 15 min. The 13th residue was reacted twice at 75°C for 10 min. The 15th residue was reacted once at 50°C for 15 min. Fmoc removal from the 16th residue to the 13th residue was performed by reacting with 20% piperidine in DMF at 75°C for 3 min. Fmoc removal from the 12th residue to the 1st residue was performed by reacting with 10% pyrrolidine in DMF at 90°C for 1 min. However, Fmoc removal of the 2nd residue and the 13th residue was performed by reacting at 25°C for 1 min followed by reacting for 1 min. Introduction of the chloroacetyl group was performed by removing the Fmoc group of the α-amino group from the solid phase resin holding the Fmoc-protected peptide obtained in the previous step by the method described above, then adding ClAcOSu (0.2 M) prepared by shaking chloroacetic acid (about 10 equivalents), DIPCI (about 10 equivalents), and HOSu (about 10 equivalents) in DCM for 60 min and then adding DMF in an equivalent amount to the DCM, and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (3 mL, mixture of TFA/H₂O/TIS/DODT in a volume ratio of 92.5/2.5/2.5/2.5) was added to the reaction vessel containing the solid phase resin, and the vessel was shaken at room temperature for 90 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water/acetonitrile (1/1/1) to a final peptide concentration of 5 mM based on the number of moles of the solid phase resin, and then 10 equivalents of triethylamine were added and stirred at room temperature for 5 hours. The reaction mixture was quenched with acetic acid and then concentrated under reduced pressure using a Genevac EZ-2 Elite.

The obtained crude product was purified under the following conditions (column: Waters Xbridge (registered trademark) C18 5 µm 30 x 150 mm; mobile phase: A = 0.1% TFA in H₂O, B = 0.1% TFA in MeCN; temperature: 50°C; gradient (%B): 5-24% over 3 minutes, then 24-29% over 8 minutes, and then 29-60% over 1 minute; flow rate: 45 mL/min).

The purity of the target product was calculated from the area ratio of the LC/MS (UV wavelength: 225 nm) chromatogram of the analysis conditions, and the result was 95.5%.

Analysis conditions: retention time = 4.88 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 5-45% over 7.15 minutes, then 45-95% over 0.3 minutes, and then 95-95% for 1.55 minutes; flow rate: 0.5 mL/min.
ESI-MS (+) measured value m/z = 793.93 (M+3H)³⁺

### [Example 4-2]

### Synthesis of 894_0465 (SEQ ID NO: 4)

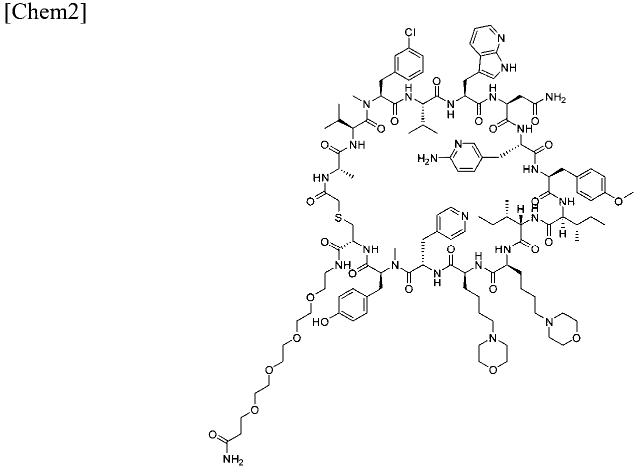

The target peptide was synthesized using Sieber amide resin (Watanabe Chemical Industries, 0.48 mmol/g, 0.10 g), starting with the removal of the Fmoc group by the general method described above. In this process, Liberty Blue from CEM was used as the solid phase synthesizer, and the synthesis was performed according to the manufacturer's manual in the same manner as for 894_0462. In the solid phase synthesis, a reaction with Fmoc-Kmor-OH was performed using Fmoc-Kmor-OH/HATU/Oxyma Pure (5.3 equivalents/5 equivalents/15 equivalents) for 10 min at 75°C in DMF.

The obtained crude product was purified under the following conditions (column: Waters Xbridge (registered trademark) C18 5 µm 30 x 150 mm; mobile phase: A = 0.1% TFA in H₂O, B = 0.1% TFA in MeCN; temperature: 50°C; gradient (%B): 5-24% over 3 minutes, then 24-29% over 8 minutes, and then 29-60% over 1 minute; flow rate: 45 mL/min).

The purity of the target product was calculated from the area ratio of the LC/MS (UV wavelength: 225 nm) chromatogram of the analysis conditions, and the result was 90.1%.

Analysis conditions: retention time = 4.98 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 5-45% over 7.15 minutes, then 45-95% over 0.3 minutes, and then 95-95% for 1.55 minutes; flow rate: 0.5 mL/min.
ESI-MS (+) measured value m/z = 821.94 (M+3H)³⁺

### [Example 4-3]

### Synthesis of 894_471 (SEQ ID NO: 10)

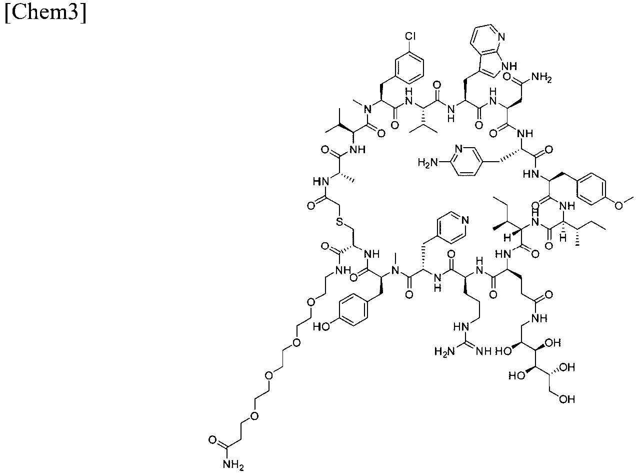

The target peptide was synthesized using Sieber amide resin (Watanabe Chemical Industries, 0.48 mmol/g, 0.10 g), starting with the removal of the Fmoc group by the general method described above. In this process, Liberty Prime from CEM was used as a solid phase synthesizer, and the synthesis was performed according to the manufacturer's manual. For the introduction of each residue, Fmoc-AA/DIPCI/Oxyma Pure (5.3 equivalents/20 equivalents/7.5 equivalents) was used per 1 equivalent of resin, and the reaction was performed once for 1.5 min at 105°C in DMF. However, the 2nd residue was reacted twice at 75°C for 30 min. The 12th residue was reacted twice at 50°C for 15 min. The 13th residue was reacted once at 90°C for 10 min. The 15th residue was reacted once at 50°C for 15 min. Fmoc removal was performed by reacting with 25% pyrrolidine in DMF at 110°C for 1 min. However, Fmoc removal of the 2nd residue and the 13th residue was performed by reacting at 25°C for 1.5 min followed by reacting for 1.5 min. Introduction of the chloroacetyl group was performed by removing the Fmoc group of the α-amino group from the solid phase resin holding the Fmoc-protected peptide obtained in the previous step by the method described above, then adding ClAcOSu (0.1 M) prepared by shaking chloroacetic acid (about 5 equivalents), DIPCI (about 5 equivalents), and HOSu (about 5 equivalents) in DCM for 60 min and then adding DMF in an equivalent amount to the DCM, and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (2 mL, mixture of TFA/H₂O/TIS/DODT in a volume ratio of 92.5/2.5/2.5/2.5) was added to the reaction vessel containing the solid phase resin, and the vessel was shaken at room temperature for 90 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water/isopropanol (90/5/5) to a final peptide concentration of 5 mM based on the number of moles of the solid phase resin, and then 10 equivalents of triethylamine were added and stirred at room temperature for 5 hours. The reaction mixture was concentrated under reduced pressure using a Genevac EZ-2 Elite. The mixture obtained was dissolved in DMSO to a final peptide concentration of 12 mM based on the number of moles of the solid phase resin, then 1.1 equivalents of D-glucamine, 4 equivalents of DIEA, and 2 equivalents of PyAOP were added and stirred at room temperature for 60 min, and the reaction mixture was quenched with acetic acid.

The obtained mixture was purified under the following conditions (column: Waters Xbridge (registered trademark) C18 5 µm 30 x 150 mm; mobile phase: A = 0.1% TFA in H₂O, B = 0.1% TFA in MeCN; temperature: 50°C; gradient (%B): 5-24% over 3 minutes, then 24-29% over 8 minutes, and then 29-60% over 1 minute; flow rate: 45 mL/min).

The purity of the target product was calculated from the area ratio of the LC/MS (UV wavelength: 225 nm) chromatogram of the analysis conditions, and the result was 88.3%.

Analysis conditions: retention time = 5.12 minutes; column: Kinetex EVO C18 2.6 µm, 2.1 x 150 mm, 100Å; mobile phase: A = 0.025% TFA in H₂O, B = 0.025% TFA in MeCN; temperature: 60°C; gradient (%B conc): 5-45% over 7.15 minutes, then 45-95% over 0.3 minutes, and then 95-95% for 1.55 minutes; flow rate: 0.5 mL/min.
ESI-MS (+) measured value m/z = 839.31 (M+3H)³⁺

### [Example 4-4]

### Synthesis of 894_473 (SEQ ID NO: 12)

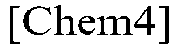

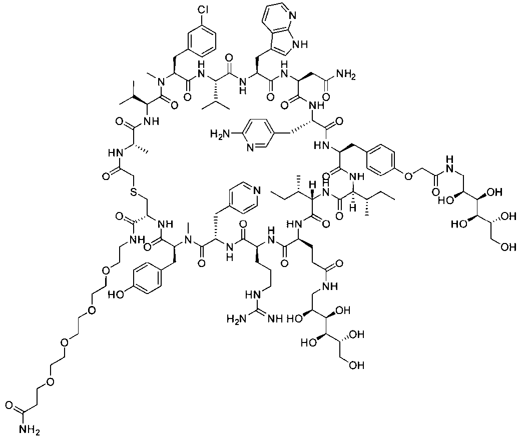

The target peptide was synthesized using Sieber amide resin (Watanabe Chemical Industries, 0.48 mmol/g, 0.10 g), starting with the removal of the Fmoc group by the general method described above. In this process, Liberty Prime from CEM was used as the solid phase synthesizer, and the synthesis was performed according to the manufacturer's manual in the same manner as for 894_471. Condensation of the 8th and 11th residue side chain carboxylic acids with D-glucamine was performed using 2.2 equivalents of D-glucamine, 8 equivalents of DIEA, and 4 equivalents of PyAOP.

The obtained crude product was purified under the following conditions (column: Waters Xbridge (registered trademark) C18 5 µm 30 x 150 mm; mobile phase: A = 0.1% TFA in H₂O, B = 0.1% TFA in MeCN; temperature: 50°C; gradient (%B): 5-19% over 3 minutes, then 19-24% over 8 minutes, and then 24-60% over 1 minute; flow rate: 45 mL/min).

The purity of the target product was calculated from the area ratio of the LC/MS (UV wavelength: 225 nm) chromatogram of the analysis conditions, and the result was 90.6%.

Analysis conditions: retention time = 4.48 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 5-45% over 7.15 minutes, then 45-95% over 0.3 minutes, and then 95-95% for 1.55 minutes; flow rate: 0.5 mL/min.
ESI-MS (+) measured value m/z = 908.38 (M+3H)³⁺

### [Example 4-5]

### Synthesis of 894_491 (SEQ ID NO: 22)

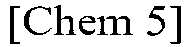

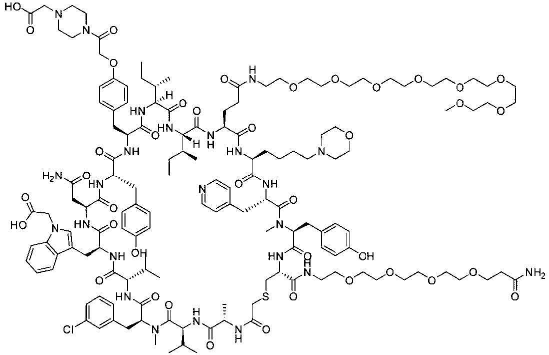

The target peptide was synthesized using Sieber amide resin (Watanabe Chemical Industries, 0.59 mmol/g, 0.11 g), starting with the removal of the Fmoc group by the general method described above. In this process, Liberty Blue HT from CEM was used as a solid phase synthesizer, and the synthesis was performed according to the manufacturer's manual. For the introduction of each residue, elongation from the 16th residue to the 14th residue was performed using Fmoc-AA/DIPCI/Oxyma Pure (4.2 equivalents/8 equivalents/4 equivalents) per 1 equivalent of resin by reacting once for 10 min at 75°C in DMF. Elongation from the 13th residue to the 1st residue was performed using Fmoc-AA/DIPCI/Oxyma Pure (4.2 equivalents/12 equivalents/6 equivalents) by reacting once for 3 min at 90°C in DMF. For the reaction at the 11th residue, Fmoc-E(allyl)-OH was used, and for the reaction at the 8th residue, Fmoc-F4aao (allyl)-OH was used. However, the 2nd residue was reacted twice at 75°C for 30 min. The 13th residue was reacted twice at 90°C for 3 min. The 15th residue was reacted once at 50°C for 15 min. Fmoc removal from the 16th residue to the 14th residue was performed by reacting with 20% piperidine in DMF at 75°C for 3 min. Fmoc removal from the 13th residue to the 1st residue was performed by reacting with 10% pyrrolidine in DMF at 90°C for 1 min. However, Fmoc removal of the 2nd residue and the 13th residue was performed by reacting at 25°C for 1 min followed by reacting for 1 min. After the above elongation up to the 11th residue in the solid phase synthesis, the solid phase resin was removed from the solid phase synthesizer, and the solid phase resin was then shaken with a solution of 5 equivalents of Fmoc-OSu in DMF per 1 equivalent of solid phase resin for 1 hour and washed with DMF. The solid phase resin was suspended in DCM, 0.2 equivalents of tetrakis (triphenylphosphine) palladium (0) and 10 equivalents of phenylsilane were added to 1 equivalent of resin, the mixture was shaken for 1 hour, and washed with DCM and DMF. The resin was suspended in DMF and reacted with HPEG8Me/DIPCI/Oxyma Pure (4 equivalents/8 equivalents/4 equivalents) to 1 equivalent of resin at 75°C for 20 min. The obtained solid phase resin was washed with DMF, and the peptide was elongated using a solid phase synthesizer according to the method described above. After the elongation up to the 8th residue, the solid phase resin was removed from the solid phase synthesizer, and the solid phase resin was then shaken with a solution of 5 equivalents of Fmoc-OSu in DMF per 1 equivalent of solid phase resin for 1 hour and washed with DMF. The solid phase resin was suspended in DCM, 0.2 equivalents of tetrakis(triphenylphosphine) palladium (0) and 10 equivalents of phenylsilane were added to 1 equivalent of solid phase resin, the mixture was shaken for 1 hour, and washed with DCM and DMF. The solid phase resin was suspended in DMF and reacted with H-Pipzaa (t-Bu)/DIPCI/Oxyma Pure (4 equivalents/8 equivalents/4 equivalents) to 1 equivalent of resin at 25°C for 60 min. The obtained solid phase resin was washed with DMF, and the peptide was elongated using a solid phase synthesizer according to the method described above. Introduction of the chloroacetyl group was performed by removing the Fmoc group of the α-amino group from the solid phase resin holding the Fmoc-protected peptide obtained in the previous step by reacting with 10% pyrrolidine in DMF at 90°C for 1 minute, then washing with DMF, then adding ClAcOSu (0.31 M) prepared by shaking chloroacetic acid (about 10 equivalents), DIPCI (about 10 equivalents), and HOSu (about 10 equivalents) in DCM for 60 min and then adding DMF in an equivalent amount to the DCM, and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (3 mL, mixture of TFA/H₂O/TIS/DODT in a volume ratio of 92.5/2.5/2.5/2.5) was added to the reaction vessel containing the solid phase resin, and the vessel was shaken at room temperature for 90 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in acetonitrile/water (1/1) to a final peptide concentration of 5 mM based on the number of moles of the solid phase resin, and then 10 equivalents of triethylamine were added and stirred at room temperature for 2 hours. The reaction mixture was quenched with acetic acid and then concentrated under reduced pressure using a Genevac EZ-2 Elite.

The obtained crude product was purified under the following conditions (column: Waters Xbridge(registered trademark) C18 5 µm 30 × 150 mm; mobile phase: A = 0.1% TFA in H₂O, B = 0.1% TFA in MeCN; temperature: 50°C; gradient (%B): 5-30% over 3 minutes, then 30-35% over 8 minutes, and then 35-60% over 1 minute; flow rate: 45 mL/min).

The purity of the target product was calculated from the area ratio of the LC/MS chromatogram (UV wavelength: 225 nm) under the analysis conditions and was found to be 92.7%.

Analysis conditions: retention time = 3.88 minutes; column: Kinetex EVO C18 2.6 µm, 2.1 × 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value: m/z = 1493.80 (M+2H)²⁺

### [Example 4-6]

### Synthesis of 894_0552 (SEQ ID NO: 53)

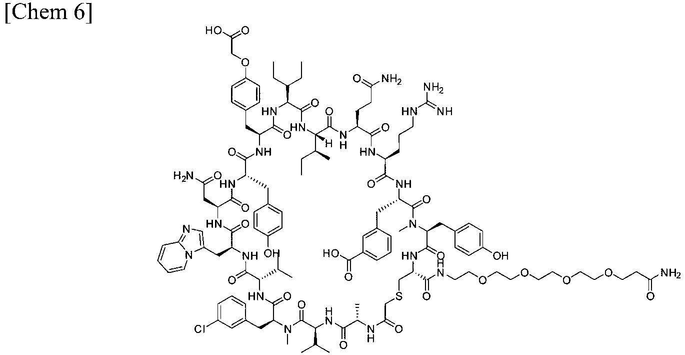

The target peptide was synthesized using Sieber amide resin (Watanabe Chemical Industries, 0.48 mmol/g, 0.10 g), starting with the removal of the Fmoc group by the general method described above. In this process, Liberty Prime from CEM was used as a solid phase synthesizer, and the synthesis was performed according to the manufacturer's manual. For the introduction of each residue, Fmoc-AA/DIPCI/Oxyma Pure (4.2 equivalents/16 equivalents/6 equivalents) was used per 1 equivalent of resin, and the reaction was performed once for 1.5 min at 105°C in DMF. However, the 2nd residue was reacted twice at 75°C for 30 min. The 12th residue was reacted twice at 50°C for 15 min. The 13th residue was reacted twice at 90°C for 10 min. The 15th residue was reacted once at 50°C for 15 min. The 5th residue was reacted by removing the solid phase resin from the solid phase synthesizer and shaking with Fmoc-AA/HATU/DIEA (4 equivalents/4 equivalents/8 equivalents) for 60 min at 25°C. After washing the solid phase resin with DMF, peptide elongation was continued using the solid phase synthesizer. Fmoc removal was performed by reacting with 4% pyrrolidine and 83 mM Oxyma Pure in DMF at 110°C for 1 min. However, Fmoc removal of the 2nd residue and the 13th residue was performed by reacting with 10% pyrrolidine in DMF at 25°C for 1.5 min followed by reacting for 1.5 min. Introduction of the chloroacetyl group was performed by removing the Fmoc group of the α-amino group from the solid phase resin holding the Fmoc-protected peptide obtained in the previous step by the method described above, then adding N- (chloroacetoxy) succinimide (about 5 equivalents) in DMF, and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. Aa reactant cocktail-A (3 mL, mixture of TFA/H₂O/TIS/DODT in a volume ratio of 92.5/2.5/2.5/2.5) was added to the reaction vessel containing the solid phase resin, and the vessel was shaken at room temperature for 90 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diisopropyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in acetonitrile/water (1/1) to a final peptide concentration of 2.5 mM based on the number of moles of the solid phase resin, then 10 equivalents of triethylamine were added and stirred at room temperature for 16 hours, and the reaction mixture was quenched with acetic acid. The reaction mixture was concentrated under reduced pressure using a Genevac EZ-2 Elite.

The obtained crude product was purified under the following conditions (column: Waters Xbridge (registered trademark) C18 5 µm 30 x 150 mm; mobile phase: A = 0.1% TFA in H₂O, B = 0.1% TFA in MeCN; temperature: 50°C; gradient (%B): 5-29% over 3 minutes, then 29-34% over 8 minutes, and then 34-60% over 1 minute; flow rate: 45 mL/min).

The purity of the target product was calculated from the area ratio of the LC/MS (UV wavelength: 225 nm) chromatogram of the analysis conditions, and the result was 92.1%.

Analysis conditions: retention time = 3.23 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value m/z = 818.33 (M+3H)³⁺

### [Example 4-7]

### Synthesis of 894_0436 (SEQ ID NO: 144)

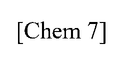

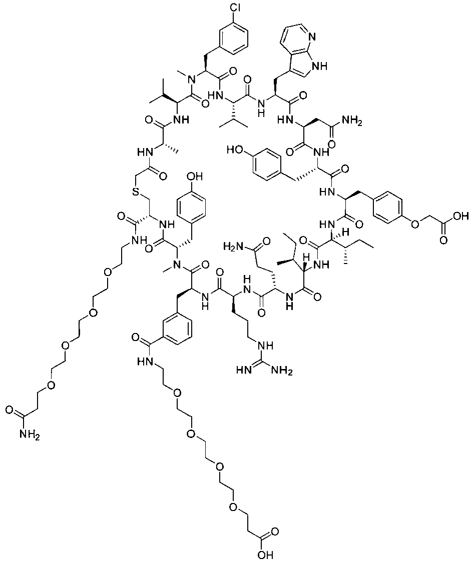

The target peptide was synthesized using Sieber amide resin (Watanabe Chemical Industries, 0.48 mmol/g, 0.21 g), starting with the removal of the Fmoc group by the general method described above. In this process, Liberty Blue from CEM was used as a solid phase synthesizer, and the synthesis was performed according to the manufacturer's manual. For the introduction of each residue, Fmoc-AA/DIPCI/Oxyma Pure (5.3 equivalents/10 equivalents/5 equivalents) was used per 1 equivalent of resin, and the reaction was performed once for 10 min at 90°C in DMF. For the reaction at the 13th residue, Fmoc-F3COO (allyl)-OH was used. However, the 2nd residue was reacted twice at 75°C for 30 min. The 5th, 6th, and 7th residues were reacted at 90°C for 3 min. The 12th residue was reacted twice at 50°C for 15 min. The 13th residue was reacted twice at 90°C for 3 min. The 14th residue and 16th residue were reacted at 90°C for 3 min. The 15th residue was reacted at 50°C for 15 min. Fmoc removal was performed up to the 2nd residue by reacting with 10% pyrrolidine in DMF at 90°C for 1 min. However, Fmoc removal of the 2nd residue and the 13th residue was performed by reacting at 25°C for 1 min followed by reacting for 1 min. The obtained solid phase resin was shaken with a solution of 5 equivalents of Fmoc-OSu in DMF per 1 equivalent of solid phase resin for 1 hour and washed with DMF. The solid phase resin was suspended in DCM, 0.2 equivalents of tetrakis (triphenylphosphine) palladium (0) and 10 equivalents of phenylsilane were added to 1 equivalent of resin, the mixture was shaken for 1 hour, and washed with DCM and DMF. The obtained solid phase resin was suspended in DMF and reacted with H-PEG4c(t-Bu)/DIPCI/Oxyma Pure (4 equivalents/8 equivalents/4 equivalents) to 1 equivalent of resin at 75°C for 30 min, and washed with DMF. Introduction of the chloroacetyl group was performed by removing the Fmoc group of the α-amino group from the solid phase resin holding the Fmoc-protected peptide obtained in the previous step by reacting twice with 20% piperidine in DMF at 25°C for 5 minutes, then washing with DMF, then adding ClAcOSu (0.05 M) prepared by shaking chloroacetic acid (about 5 equivalents), DIPCI (about 5 equivalents), and HOSu (about 5 equivalents) in DCM for 60 min and then adding DMF in an equivalent amount to the DCM, and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (4 mL, mixture of TFA/H₂O/TIS/DODT in a volume ratio of 92.5/2.5/2.5/2.5) was added to the reaction vessel containing the solid phase resin, and the vessel was shaken at room temperature for 60 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diisopropyl ether/hexane (1/1) mixed solvent cooled to 0°C, a cloudy precipitate was formed. The mixture was centrifuged (9000 rpm, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water (9/1) to a final peptide concentration of 5 mM based on the number of moles of the solid phase resin, and then 10 equivalents of triethylamine were added and stirred at room temperature for 15 hours. The reaction mixture was quenched with acetic acid and then concentrated under reduced pressure using a Genevac HT-12.

The obtained crude product was purified under the following conditions (column: Waters Xbridge (registered trademark) C18 5 µm 50 x 250 mm; mobile phase: A = 0.1% TFA in H₂O, B = 0.1% TFA in MeCN; temperature: 50°C; gradient (%B): 0-0% over 4.9 minutes, then 0-4.2% over 2 minutes, then 4.2-29.7% over 3 minutes, then 29.7-34.7% over 15 minutes, and then 34.7-60% over 3 minutes; flow rate: 18-18mL/min for 4.9 minutes, then 18-118 mL/min over 2 minutes, then 118 mL/min).

The purity of the target product was calculated from the area ratio of the LC/MS (UV wavelength: 225 nm) chromatogram of the analysis conditions, and the result was 97.8%.

Analysis conditions: retention time = 3.91 minutes; column: Kinetex EVO C18 2.6 µm, 2.1 x 150 mm, 100Å; mobile phase: A = 0.025% TFA in H₂O, B = 0.025% TFA in MeCN; temperature: 60°C; gradient (%B conc): 20-60% over 7.15 minutes, then 60-95% over 3 minutes, and then 95-95% for 1.55 minutes; flow rate: 0.5 mL/min.
ESI-MS (+) measured value m/z = 895.81 (M+3H)³⁺

### [Example 4-8]

### Synthesis of 894_0438 (SEQ ID NO: 146)

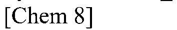

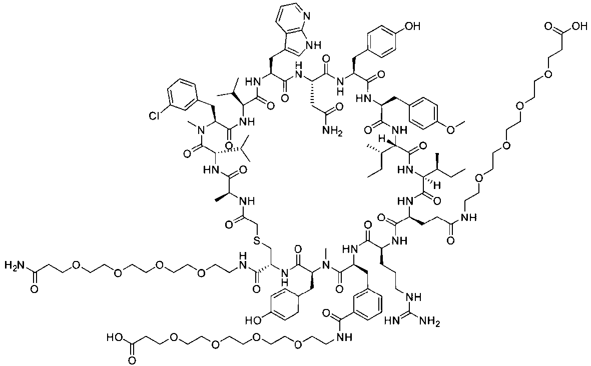

The target peptide was synthesized using Sieber amide resin (Watanabe Chemical Industries, 0.48 mmol/g, 0.21 g), starting with the removal of the Fmoc group by the general method described above. In this process, Liberty Blue from CEM was used as the solid phase synthesizer, and the synthesis was performed according to the manufacturer's manual in the same manner as for the synthesis of 894_0436. In the solid phase synthesis, Fmoc-E(allyl)-OH was used for the reaction of the 11th residue, and Fmoc-F3COO (allyl)-OH was used for the reaction of the 13th residue. Condensation of the 11th and 13th residue side chain carboxylic acids with H-PEG4c (tBu) was performed using H-PEG4c (tBu)/DIPCI/Oxyma Pure (8 equivalents/16 equivalents/8 equivalents).

The obtained crude product was purified under the following conditions (column: Waters Xbridge (registered trademark) C18 5 µm 30 x 150 mm; mobile phase: A = 0.1% TFA in H₂O, B = 0.1% TFA in MeCN; temperature: 50°C; gradient (%B): 2.2-2.2% for 4.9 minutes, then 2.2-6.0% over 1 minute, then 6.0-31.6% over 3 minutes, then 31.6-36.7% over 9 minutes, and then 36.7-60% over 1 minute; flow rate: 9-9mL/min for 4.9 minutes, then 9-44mL/min over 1 minute, then 44 mL/min).

The purity of the target product was calculated from the area ratio of the LC/MS (UV wavelength: 225 nm) chromatogram of the analysis conditions, and the result was 95.7%.

Analysis conditions: retention time = 4.28 minutes; column: Kinetex EVO C18 2.6 µm, 2.1 x 150 mm, 100Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value m/z = 963.85 (M+3H)³⁺

### [Example 4-9]

### Synthesis of 894_3426 (SEQ ID NO: 67)

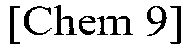

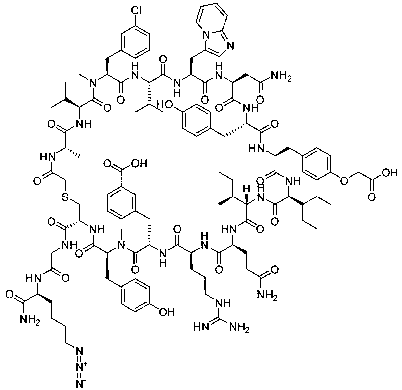

The target peptide was synthesized using Sieber amide resin (Watanabe Chemical Industries, 0.54 mmol/g, 0.93 g), starting with the removal of the Fmoc group by the general method described above. In this process, Liberty Blue from CEM was used as a solid phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was performed once for 10 min at 75°C in DMF. However, the 2nd residue was reacted twice at 75°C for 30 min. The 8th, 9th, and 13th residues were reacted twice at 75°C for 10 min. The 12th residue was reacted twice at 50°C for 15 min. The 15th residue was reacted once at 50°C for 15 min. The 5th residue was reacted by removing the solid phase resin from the solid phase synthesizer and shaking with Fmoc-AA/HATU/DIEA (5 equivalents/5 equivalents/10 equivalents) for 60 min at 40°C. After washing the solid phase resin with DMF, peptide elongation was continued using the solid phase synthesizer. Fmoc removal was performed by reacting with 10% pyrrolidine in DMF at 75°C for 3 min. However, Fmoc removal of the 2nd residue and the 13th residue was performed by reacting at 25°C for 5 min followed by reacting for 5 min. Introduction of the chloroacetyl group was performed by removing the Fmoc group of the α-amino group from the solid phase resin holding the Fmoc-protected peptide obtained in the previous step by the method described above, then adding N-(chloroacetoxy) succinimide (about 10 equivalents) in DMF, and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (30 mL, mixture of TFA/H₂O/TIS/DODT in a volume ratio of 92.5/2.5/2.5/2.5) was added to the reaction vessel containing the solid phase resin, and the vessel was shaken at room temperature for 90 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water (9/1) to a final peptide concentration of 5 mM based on the number of moles of the solid phase resin, and then 10 equivalents of triethylamine were added and stirred at room temperature for 2 hours. The reaction mixture was quenched with acetic acid, and then concentrated under reduced pressure using a Genevac HT-12.

The obtained crude product was purified under the following conditions (column: Waters Xselect CSH Prep C18 5 µm 50 x 250 mm; mobile phase: A = 20 mM TEAA in H₂O, B = 0 mM TEAA in MeCN, C = 200 mM TEAA in H₂O, D = MeCN; temperature: 50°C; gradient (%A): 0.1-0.1% for 5 minutes, then 0.1-71.1% over 0.1 minutes, and then (100-%B), gradient (%B): 0-0% for 5 minutes, then 0-28.9% over 0.1 minutes, then 28.9-30.7% over 1.9 minutes, then 30.7-30.7% for 3 minutes, then 30.7-35.8% over 15.5 minutes, then 35.8-60% over 1.5 minutes, and then 60-90% over 4 minutes, gradient (%C): 71.0-71.0% for 5 minutes, 71.0-0% over 0.1 minutes, then 0%, gradient (%D): 28.9-28.9% for 5 minutes, then 28.9-0% over 0.1 minutes, then 0%; flow rate: 18-18 mL/min for 5.1 minutes, then 18-118 mL/min over 1.9 minutes, then 118 mL/min).

The purity of the target product was calculated from the area ratio of the LC/MS (UV wavelength: 225 nm) chromatogram of the analysis conditions, and the result was 95.7%.

Analysis conditions: retention time = 3.58 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value m/z = 1208.36 (M+2H)²⁺

### [Example 4-10]

### Synthesis of 894_3427 (SEQ ID NO: 66)

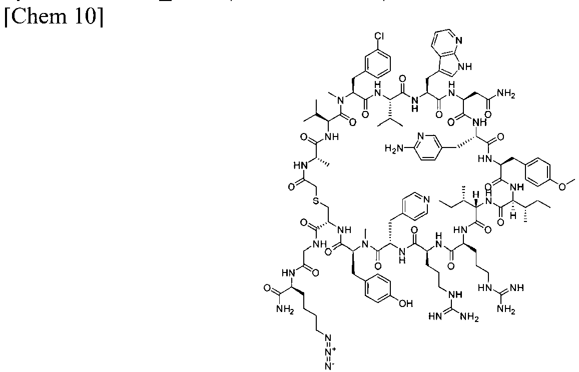

The target peptide was synthesized using Sieber amide resin (Watanabe Chemical Industries, 0.54 mmol/g, 0.46 × 2 g), starting with the removal of the Fmoc group by the general method described above. In this process, Liberty Blue from CEM was used as a solid phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was performed once for 3 min at 90°C in DMF. However, the 15th residue was reacted once at 50°C for 15 min. The 13th residue was reacted twice at 90°C for 3 min. The 11th and 12 residues were reacted twice at 50°C for 15 min. The 9th residue was reacted twice at 90°C for 10 min. The 8th residue was reacted once at 90°C for 10 min. The 2nd residue was reacted twice at 75°C for 30 min. Fmoc removal was performed by reacting with 10% pyrrolidine in DMF at 90°C for 1 min. However, Fmoc removal of the 2nd residue and the 13th residue was performed by reacting at 25°C for 1 min followed by reacting for 1 min. Introduction of the chloroacetyl group was performed by removing the Fmoc group of the α-amino group from the solid phase resin holding the Fmoc-protected peptide obtained in the previous step by the method described above, then adding N- (chloroacetoxy) succinimide (about 10 equivalents), and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (33 mL, mixture of TFA/H₂O/TIS/DODT in a volume ratio of 92.5/2.5/2.5/2.5) was added to the reaction vessel containing the solid phase resin, and the vessel was shaken at room temperature for 90 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water (9/1) to a final peptide concentration of 5 mM based on the number of moles of the solid phase resin, and then 10 equivalents of triethylamine were added and stirred at room temperature for 2 hours. The reaction mixture was quenched with 15 equivalents of acetic acid, and then concentrated under reduced pressure using a Genevac HT-12.

The obtained crude product was purified under the following conditions (column: Waters Xbridge (registered trademark) C18 5 µm 50 x 150 mm; mobile phase: A = 0.1% TFA in H₂O, B = 0.1% TFA in MeCN; temperature: 40°C; gradient (%B): 5-5% for 2 minutes, then 5-23% over 1 minute, then 23-28% over 8 minutes, and then 28-60% over 1 minute; flow rate: 20-20 mL/min for 1 min, then 20-120 mL/min over 1 min, and then 120 mL/min).

The purity of the target product was calculated from the area ratio of the LC/MS (UV wavelength: 225 nm) chromatogram of the analysis conditions, and the result was 93.3%.

Analysis conditions: retention time = 5.11 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 5-45% over 7.15 minutes, then 45-95% over 0.3 minutes, and then 95-95% for 1.55 minutes; flow rate: 0.5 mL/min.
ESI-MS (+) measured value m/z = 781.65 (M+3H)³⁺

### [Example 4-11]

### Synthesis of 894_3428 (SEQ ID NO: 68)

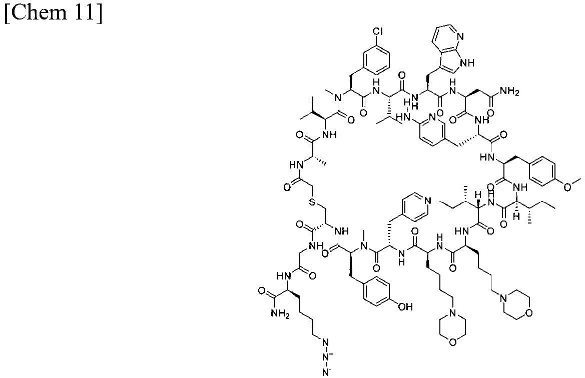

The target peptide was synthesized using Sieber amide resin (Watanabe Chemical Industries, 0.54 mmol/g, 0.46 g × 2), starting with the removal of the Fmoc group by the general method described above. In this process, Liberty Blue from CEM was used as a solid phase synthesizer, and the synthesis was performed according to the manufacturer's manual. For the introduction of each residue, Fmoc-AA/DIC/Oxyma Pure (4.2 equivalents/ 8 equivalents/ 4 equivalents) was used per 1 equivalent of resin, and the reaction was performed once for 3 min at 90°C in DMF. However, the 15th residue was reacted once at 50°C for 15 min. The 13th residue was reacted twice at 90°C for 3 min. The 1st, 3rd, 4th, 5th, 6th, 7th, 8th, 9th, 10th, and 11th residues were reacted once at 40°C for 30 min. The 2nd residue was reacted twice at 75°C for 30 min. Fmoc removal was performed by reacting with 10% pyrrolidine in DMF at 90°C for 1 min. However, Fmoc removal of the 1st, 2nd, 3rd, 4th, 5th, 6th, 7th, 8th, 9th, 10th, 11th, 12th, and 13th residues was performed by reacting at 25°C for 1 min followed by reacting for 1 min. Introduction of the chloroacetyl group was performed by removing the Fmoc group of the α-amino group from the solid phase resin holding the Fmoc-protected peptide obtained in the previous step by the method described above, then adding N-(chloroacetoxy) succinimide (about 10 equivalents), and shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (30 mL, mixture of TFA/H₂O/TIS/DODT in a volume ratio of 92.5/2.5/2.5/2.5) was added to the reaction vessel containing the solid phase resin, and the vessel was shaken at room temperature for 90 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water (9/1) to a final peptide concentration of 5 mM based on the number of moles of the solid phase resin, and then 10 equivalents of triethylamine were added and stirred at room temperature for 2 hours. The reaction mixture was quenched with 15 equivalents of acetic acid, and then concentrated under reduced pressure using a Genevac HT-12.

The obtained crude product was purified under the following conditions (column: Waters Xbridge (registered trademark) C18 5 µm 50 x 150 mm; mobile phase: A = 0.1% TFA in H₂O, B = 0.1% TFA in MeCN; temperature: 40°C; gradient (%B): 5-5% for 2 minutes, then 5-23% over 1 minute, then 23-28% over 8 minutes, and then 28-60% over 1 minute; flow rate: 20-20 mL/min for 1 min, then 20-120 mL/min over 1 min, and then 120 mL/min).

The purity of the target product was calculated from the area ratio of the LC/MS (UV wavelength: 225 nm) chromatogram of the analysis conditions, and the result was 97.3%.

Analysis conditions: retention time = 5.15 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 5-45% over 7.15 minutes, then 45-95% over 0.3 minutes, and then 95-95% for 1.55 minutes; flow rate: 0.5 mL/min.
ESI-MS (+) measured value m/z = 809.62 (M+3H)³⁺

### [Example 4-12]

### Synthesis of 894_3484 (SEQ ID NO: 195)

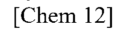

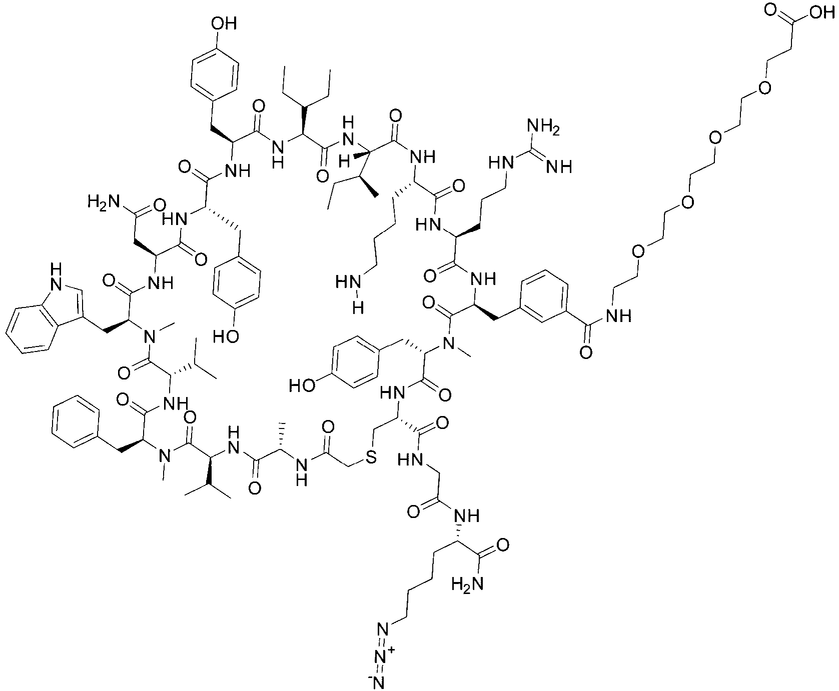

The target peptide was synthesized using Sieber amide resin (Watanabe Chemical Industries, 0.60 mmol/g, 0.83 g), starting with the removal of the Fmoc group by the general method described above. In this process, Liberty Blue HT from CEM was used as a solid phase synthesizer, and the synthesis was performed according to the manufacturer's manual. For the introduction of each residue, using Fmoc-AA/DIPCI/Oxyma Pure (4.2 equivalents/8 equivalents/4 equivalents) per 1 equivalent of resin, the reaction was performed once for 10 minutes at 75°C in DMF. However, the 2nd residue and 4th residue were reacted twice at 75°C for 30 minutes. Fmoc-F3COO (allyl)-OH was used for the reaction of the 13th residue. The 15th residue was reacted once at 50°C for 15 minutes. Fmoc removal was performed by reacting twice with 10% piperidine DMF at room temperature for 5 minutes. However, Fmoc removal of the 3rd residue and the 5th residue were performed by reacting at 25°C for 5 minutes followed by reacting for 10 minutes. Fmoc removal of the 14th residue, the 15th residue, the 16th residue, and 17th residue were performed by reacting at 75°C for 3 minutes. The solid-phase resin obtained by removing the solid-phase resin from the solid phase synthesizer was suspended in DCM/HFIP (99/1), 0.2 equivalents of tetrakis (triphenylphosphine) palladium (0) and 10 equivalents of phenylsilane were added to 1 equivalent of resin, the mixture was shaken for 1 hour, and washed with DCM and DMF. The obtained solid phase resin was suspended in DMF and reacted with H-PEG4c (tBu) /HATU/DIEA (4.2 equivalents/4 equivalents/8 equivalents) to 1 equivalent of resin at room temperature for 30 minutes. Introduction of the chloroacetyl group was carried out by first removing the Fmoc group from the α-amino group of the Fmoc-protected peptide retained on the solid-phase resin obtained from the previous step, by treating with a 10% piperidine solution in DMF at room temperature for 2 minutes, followed by washing with DMF. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (25 mL, mixture of TFA/H₂O/TIS/DODT in a volume ratio of 92.5/2.5/2.5/2.5) was added to the reaction vessel containing the solid phase resin, and the vessel was shaken at room temperature for 40 minutes. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diisopropyl ether/hexane (1/1) mixed solvent cooled to 0°C, a cloudy precipitate was formed. The mixture was centrifuged (9000 rpm, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/acetonitrile/water (1/1/1) to a final peptide concentration of 5 mM based on the number of moles of the solid phase resin, and then 10 equivalents of triethylamine were added and stirred at room temperature for 2 hours. The reaction mixture was quenched with acetic acid and then concentrated under reduced pressure using a Genevac EZ-2 Elite.

The obtained crude product was purified under the following conditions (column: Waters Xselect CSH (registered trademark) C18 5 µm 50 x 250 mm; mobile phase: A = 0.1% TFA in H₂O, B = 0.1% TFA in MeCN; temperature: 50°C; gradient (%B): 1.1% over 5 minutes, then 1.1-5.2% over 2 minute, then 5.2-30.7% over 3 minutes, then 30.7-35.8% over 15.5 minutes, and then 35.8-60% over 1.5 minutes; flow rate: 18 mL/min over 8 minutes, then 18-118mL/min over 2 minutes, and then 118 mL/min).

The purity of the target product was calculated from the area ratio of the LC/MS (UV wavelength: 225 nm) chromatogram of the analysis conditions, and the result was 96.4%.

Analysis conditions: retention time = 4.10 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value m/z = 1292.43 (M+2H)²⁺

### [Example 4-13]

### Synthesis of 894_3486 (SEQ ID NO: 196)

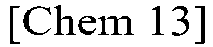

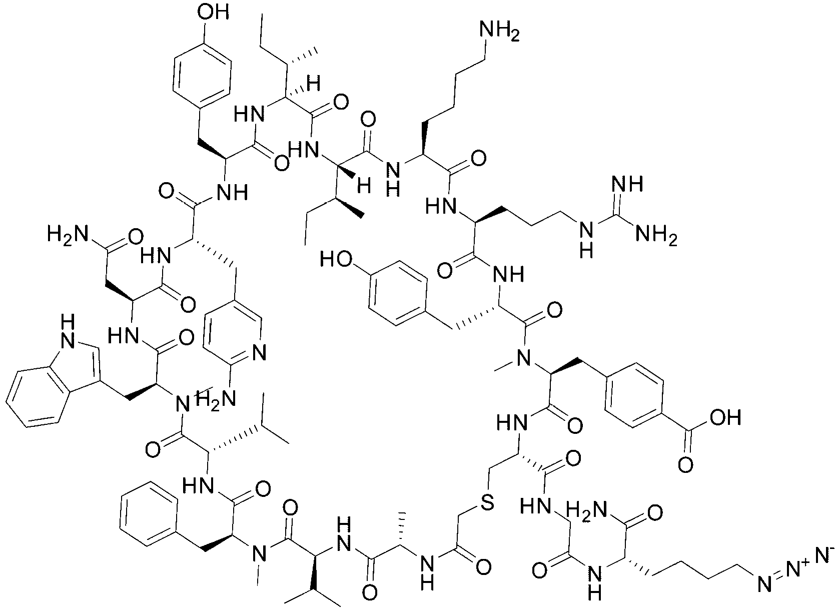

The target peptide was synthesized using Sieber amide resin (Watanabe Chemical Industries, 0.60 mmol/g, 0.41 g × 2 batches), starting with the removal of the Fmoc group by the general method above. In this process, Liberty Blue HT from CEM was used as a solid phase synthesizer, and the synthesis was performed according to the manufacturer's manual. For the introduction of each residue, using Fmoc-AA/DIPCI/Oxyma Pure (4.2 equivalents/8 equivalents/4 equivalents) per 1 equivalent of resin, the reaction was performed once for 3 minutes at 90°C in DMF. However, the 2nd residue was reacted twice at 75°C for 30 minutes.

For the introduction of the 7th residue, using Fmoc-AA/HATU/DIEA (4.2 equivalents/8 equivalents/4 equivalents), the reaction was performed once for 30 minutes at 40°C. The 9th residue was reacted twice at 90°C for 10 minutes. The 10th residue was reacted once at 90°C for 10 minutes. The 12th residue was reacted twice at 50°C for 15 minutes. The 13th residue was reacted twice at 90°C for 10 minutes. The 15th residue was reacted once at 50°C for 15 minutes. Fmoc removal was performed by reacting once with 10% pyrrolidine in DMF at 90°C for 1 minute. However, Fmoc removal of the 2nd residue, the 4th residue, the 9th residue, and the 13th residue were performed by reacting at 25°C for 1 minute followed by reacting for 1 minute. Introduction of the chloroacetyl group was carried out by first mixing the solid-phase resin retaining the Fmoc-protected peptide obtained in the previous step, by removing the Fmoc group from the α-amino group using the method described above, and then adding a DMF solution of N-(chloroacetoxy) succinimide (approximately 5 equivalents) followed by shaking at 25°C for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (25 mL, mixture of TFA/H₂O/TIS/DODT in a volume ratio of 92.5/2.5/2.5/2.5) was added to the reaction vessel containing the solid phase resin, and the vessel was shaken at room temperature for 60 minutes. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diisopropyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water (9/1) to a final peptide concentration of 2.8 mM based on the number of moles of the solid phase resin, and then 10 equivalents of triethylamine were added and stirred at room temperature for 3 hours. The reaction mixture was quenched with acetic acid and then concentrated under reduced pressure using a Genevac HT-12.

The obtained crude product was purified under the following conditions (column: Waters Xselect CSH (registered trademark) C18 5 µm 50 x 250 mm; mobile phase: A = 0.1% TFA in H₂O, B = 0.1% TFA in MeCN; temperature: 50°C; gradient (%B): 0% over 5 minutes, then 0-4.2% over 2 minute, then 4.2-25.6% over 3 minutes, then 25.6-30.7% over 15.5 minutes, and then 30.7-60% over 1.5 minutes; flow rate: 18 mL/min over 8 minutes, then 18-118mL/min over 2 minutes, and then 118 mL/min).

The purity of the target product was calculated from the area ratio of the LC/MS (UV wavelength: 225 nm) chromatogram of the analysis conditions, and the result was 94.9%.

Analysis conditions: retention time = 3.02 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value m/z = 1161.72 (M+2H)²⁺

### [Example 4-14]

### Synthesis of 894_3488 (SEQ ID NO: 193)

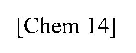

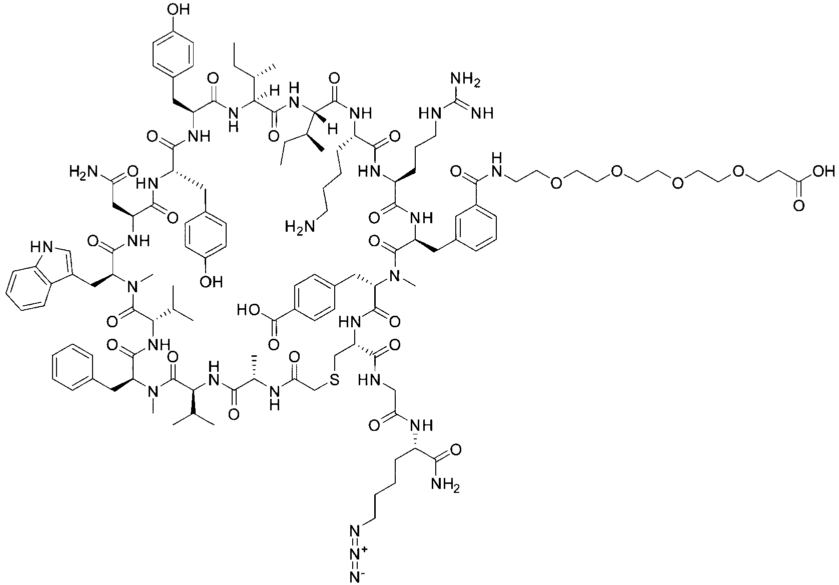

The target peptide was synthesized using Sieber amide resin (Watanabe Chemical Industries, 0.60 mmol/g, 0.83 g), starting with the removal of the Fmoc group by the general method described above. In this process, Liberty Blue HT from CEM was used as a solid phase synthesizer, and the synthesis was performed according to the manufacturer's manual. For the introduction of each residue, using Fmoc-AA/DIPCI/Oxyma Pure (4.2 equivalents/8 equivalents/4 equivalents) per 1 equivalent of resin, the reaction was performed once for 10 minutes at 75°C in DMF. However, the 2nd residue and 4th residue were reacted twice at 75°C for 30 minutes. The 12th residue was reacted twice at 50°C for 20 minutes Fmoc-F3COO (allyl)-OH was used for the reaction of the 13th residue. The 15th residue was reacted once at 50°C for 20 minutes. Fmoc removal was performed by reacting twice with 10% pyrrolidine in DMF at 75°C for 3 minutes. However, Fmoc removal of the 2nd residue, the 4th residue, and the 13th residue were performed by reacting at room temperature for 5 minutes followed by reacting for 5 minutes. The obtained solid phase resin was shaken with a solution of 5 equivalents of Fmoc-OSu in DMF per 1 equivalent of solid phase resin for 1 hour and washed with DMF. The solid-phase resin obtained by removing the solid-phase resin from the solid phase synthesizer was suspended in DCM, 0.2 equivalents of tetrakis (triphenylphosphine)palladium (0) and 10 equivalents of phenylsilane were added to 1 equivalent of resin, the mixture was shaken for 1 hour, and washed with DCM and DMF. The obtained solid phase resin was suspended in DMF and reacted with H-PEG4c (tBu)/DIC/Oxyma Pure (4 equivalents/8 equivalents/4 equivalents) to 1 equivalent of resin at 75°C temperature for 30 minutes. Introduction of the chloroacetyl group was performed by removing the Fmoc group of the α-amino group from the solid phase resin holding the Fmoc-protected peptide obtained in the previous step by reacting with 10% pyrrolidine in DMF at room temperature for 5 minutes, then adding DMF/DCM (1:1) solution of N-(chloroacetoxy) succinimide (about 5 equivalents), and shaking for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (20 mL, mixture of TFA/H₂O/TIS/DODT in a volume ratio of 90/2.5/2.5/5.0) was added to the reaction vessel containing the solid phase resin, and the vessel was shaken at room temperature for 60 minutes. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether /hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in acetonitrile/water containing 1% triethylamine (1/1) to a final peptide concentration of 5 mM based on the number of moles of the solid phase resin, and then 10 equivalents of triethylamine was added and stirred at room temperature for 1 hour. The reaction mixture was quenched with acetic acid and then concentrated under reduced pressure using a Genevac HT-12.

The obtained crude product was purified under the following conditions (column: Waters Xselect CSH (registered trademark) C18 5 µm 50 x 250 mm; mobile phase: A = 0.1% TFA in H₂O, B = 0.1% TFA in MeCN; temperature: 50°C; gradient (%B): 1.1% over 5 minutes, then 1.1-5.2% over 2 minutes, then 5.2-30.7% over 3 minutes, then 30.7-35.8% over 15.5 minutes, and then 35.8-60% over 1.5 minutes; flow rate: 18 mL/min over 8 minutes, then 18-118mL/min over 2 minutes, and then 118 mL/min).

The purity of the target product was calculated from the area ratio of the LC/MS (UV wavelength: 225 nm) chromatogram of the analysis conditions, and the result was 94.0%.

Analysis conditions: retention time = 4.05 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value m/z = 1299.41 (M+2H)²⁺

### [Example 4-15]

### Synthesis of 894_0640 (SEQ ID NO: 172)

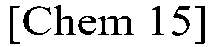

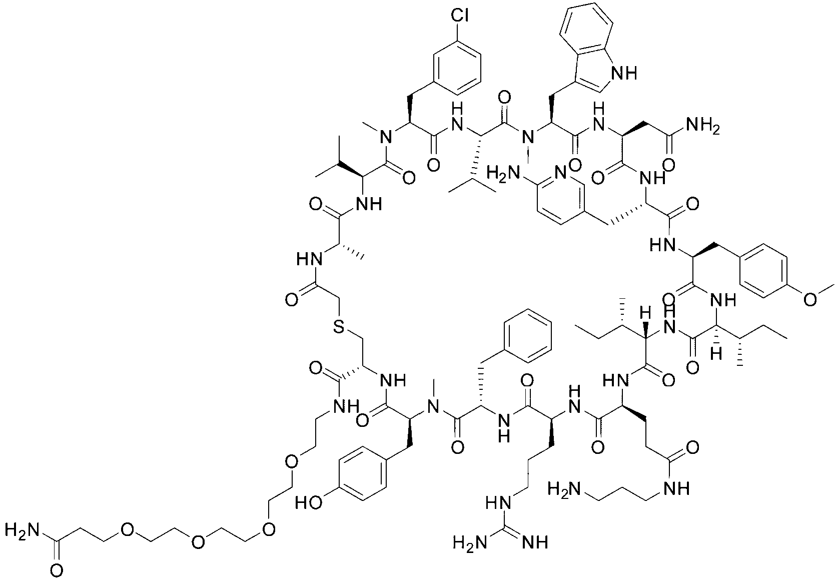

The target peptide was synthesized using Sieber amide resin (Watanabe Chemical Industries, 0.60 mmol/g, 1.67 g), starting with the removal of the Fmoc group by the general method described above. In this process, Liberty Blue HT from CEM was used as a solid phase synthesizer, and the synthesis was performed according to the manufacturer's manual. For the introduction of each residue, using Fmoc-AA/DIPCI/Oxyma Pure (4.2 equivalents/8 equivalents/4 equivalents) per 1 equivalent of resin, the reaction was performed once for 10 minutes at 90°C in DMF, elongating the peptide to the 12th residue. The 12th residue was reacted twice at 50°C for 20 minutes. The 13th residue was reacted twice at 75°C for 30 minutes. The 14th residue was reacted once at 75°C for 30 minutes. The 15th residue was reacted once for 20 minutes. The 16th residue was reacted once at 75°C for 10 minutes. Fmoc removal was performed by reacting with 10% pyrrolidine in DMF at 75°C for 3 minutes. However, the 12th residue, the 13th residue, and the 16th residue were reacted once at 25°C for 1 minute followed by reacting for 1 minute. For the introduction of the 11th residue, the reaction was performed using Fmoc-E(allyl)-OH/HATU/DIEA (5 equivalents/5 equivalents/10 equivalents) per 1 equivalent of resin by reacting once for 60 minutes at room temperature.

The solid-phase resin obtained by removing the solid-phase resin from the solid phase synthesizer was suspended in DCM, 0.2 equivalents of tetrakis (triphenylphosphine)palladium (0) and 10 equivalents of phenylsilane were added to 1 equivalent of resin, the mixture was shaken for 1 hour, and washed with DCM and DMF. The obtained solid phase resin was suspended in DMF and reacted with Boc-apa-H/PyAOP/DIEA (10 equivalents/10 equivalents/10 equivalents) to 1 equivalent of resin at 25°C for 120 minutes, and washed with DMF. For the following introduction of each residue, elongation was performed using Fmoc-AA/DIPCI/Oxyma Pure (5.3 equivalents/10 equivalents/5 equivalents) per 1 equivalent of resin once for 10 minutes at 90°C in DMF. However, the 1st residue, the 6th residue, and the 8th residue were reacted once at 90°C for 3 minutes. The 2nd residue and the 4th residue were reacted twice at 90°C for 10 minutes. Fmoc removal was performed by reacting twice with 10% pyrrolidine in DMF at 90°C for 1 minute followed by reacting for 1 minute.

However, Fmoc removal of 2nd residue and the 4th residue was performed by reacting twice with 10% pyrrolidine in DMF at 25°C for 1 minute followed by reacting for 1 minute. Introduction of the chloroacetyl group was carried out by first removing the Fmoc group from the α-amino group of the Fmoc-protected peptide retained on the solid-phase resin obtained from the previous step, by treating twice with a 10% piperidine solution in DMF at 25°C for 1 minute, followed by washing with DMF, then shaking with DMF solution of N-(chloroacetoxy) succinimide (about 10 equivalents) for 60 minutes. After the obtained resin was washed five times with DMF and three times with methylene chloride, followed by washing with diethyl ether, the resin was dried under reduced pressure. Then, deprotection of the side chain and cleavage from the solid phase resin was performed. A reactant cocktail-A (20 mL, mixture of TFA/H₂O/TIS/DODT in a volume ratio of 92.5/2.5/2.5/2.5) was added to the reaction vessel containing the solid phase resin, and the vessel was shaken at room temperature for 60 min. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diisopropyl ether/hexane (1/1) mixed solvent cooled to 0°C, a cloudy precipitate was formed. The mixture was centrifuged (9000 rpm, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water/acetonitrile (2/1/1) to a final peptide concentration of 5 mM based on the number of moles of the solid phase resin, and then 10 equivalents of triethylamine were added and stirred at room temperature for 1 hour. The reaction mixture was quenched with acetic acid and then concentrated under reduced pressure using a Genevac EZ-2 Elite.

The obtained crude product was purified under the following conditions (column: Waters Xbridge (registered trademark) C18 5 µm 50 x 150 mm; mobile phase: A = 0.1% TFA in H₂O, B = 0.1% TFA in MeCN; temperature: 40°C; gradient (%B): 5% over 2 minutes, then 5-28% over 1 minute, then 28-33% over 8 minutes, and then 33-60% over 1 minute; flow rate: 20 mL/min over 1 minute, then 20-120mL/min over 1 minute, and then 120 mL/min over 1 minute).

The purity of the target product was calculated from the area ratio of the LC/MS (UV wavelength: 225 nm) chromatogram of the analysis conditions, and the result was 90.9%.

Analysis conditions: retention time = 3.51 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (+) measured value m/z = 807.44 (M+3H)³⁺

### [Example 4-16]

### Synthesis of 894 0646 (SEQ ID NO: 178)

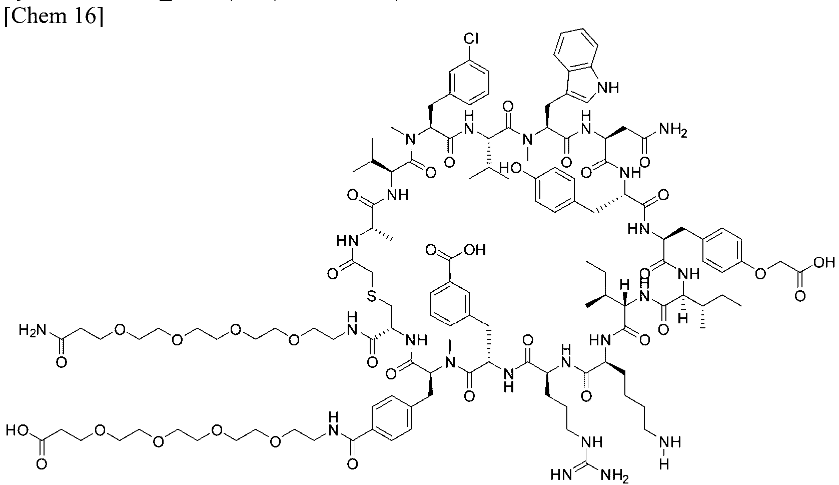

The target peptide was synthesized using Sieber amide resin (Watanabe Chemical Industries, 0.60 mmol/g, 0.21 g), starting with the removal of the Fmoc group by the general method described above. In this process, Liberty Blue from CEM was used as a solid phase synthesizer, and the synthesis was performed according to the manufacturer's manual. For the introduction of each residue, reaction was performed using Fmoc-AA/DIPCI/Oxyma Pure (4.2 equivalents/8 equivalents/4 equivalents) per 1 equivalent of resin by reacting twice for 3 minutes at 90°C in DMF. However, the 2nd residue and the 4th residue were reacted twice at 75°C for 30 minutes. The 12th residue was reacted once at 75°C for 10 minutes. The 13th residue was reacted twice at 90°C for 10 minutes. The 14th residue was performed using Fmoc-MeF4COO (allyl)-OH by reacting once for 3 minutes at 90°C in DMF. The 15th residue was performed by reacting once for 15 minutes at 50°C in DMF. Fmoc removal was performed by reacting once with 10% piperidine in DMF at 90°C for 1 minute. However, Fmoc removal of the 4th residue and the 13th residue were performed by reacting for 1 min followed by reacting for 1 minute. Fmoc removal of the 1st residue was performed by reacting after the reaction between the MeF4COO side chain and H-PEG4c-OtBu. The solid-phase resin obtained by removing the solid-phase resin from the solid phase synthesizer was suspended in DCM/ acetic acid (99/1), 0.2 equivalents of tetrakis (triphenylphosphine) palladium (0) and 10 equivalents of phenylsilane were added to 1 equivalent of resin, the mixture was shaken for 1 hour, and washed with DCM and DMF. The resin was suspended in DMF and reacted with H-PEG4c (tBu)/HATU/DIEA (4.2 equivalents/4 equivalents/8 equivalents) to 1 equivalent of resin at room temperature for 10 minutes. Introduction of the chloroacetyl group was carried out by first removing the Fmoc group from the α-amino group of the Fmoc-protected peptide retained on the solid-phase resin obtained from the previous step, by treating twice with a 10% piperidine solution in DMF at room temperature for 5 minutes, followed by washing with DMF, then shaking with DMF solution of chloroacetic acid/HATU/DIEA (5 equivalents/5 equivalents/10 equivalents) for 30 minutes. After the obtained resin was washed five times with DMF and three times with methylene chloride, followed by washing with diethyl ether, the resin was dried under reduced pressure. Then, deprotection of the side chain and cleavage from the solid phase resin was performed. Aa reactant cocktail-A (5 mL, mixture of TFA/H₂O/TIS/DODT in a volume ratio of 92.5/2.5/2.5/2.5) was added to the reaction vessel containing the solid phase resin, and the vessel was shaken at room temperature for 90 minutes. The reaction liquid was collected by frit filtration. The solid phase resin remaining in the reaction vessel was shaken again with the cleavage cocktail, and the solution component was collected from the frit and mixed with the above-mentioned filtrate. After the filtrate was added to an excess of a diethyl ether/hexane (1/1) mixed solvent cooled to 0°C, a cloudy precipitate was formed. The mixture was centrifuged (9000 rpm, 0°C, 2 min) and the solution was decanted. The resulting solid was washed again with a small amount of diethyl ether cooled to 0°C, and dried under reduced pressure. The solid obtained was used for the next cyclization reaction. For the cyclization reaction of the peptide, the peptide was dissolved in DMSO/water (95/5) to a final peptide concentration of 5 mM based on the number of moles of the solid phase resin, and then 10 equivalents of triethylamine were added and stirred at room temperature for 1 hours. The reaction mixture was quenched with acetic acid and then concentrated under reduced pressure using a Genevac EZ-2 Elite.

The obtained crude product was purified under the following conditions (column: Waters Xbridge (registered trademark) C18 5 µm 50 x 150 mm; mobile phase: A = 0.1% TFA in H₂O, B = 0.1% TFA in MeCN; temperature: 40°C; gradient (%B): 6% over 2 minutes, then 6-31% over 1 minute, then 31-36% over 8 minutes, and then 36-60% over 1 minute; flow rate: 20 mL/min over 1 minute, then 20-120mL/min over 1 minute, and then 20 mL/min over 1 minute).

The purity of the target product was calculated from the area ratio of the LC/MS (UV wavelength: 225 nm) chromatogram of the analysis conditions, and the result was 91.8%.

Analysis conditions: retention time = 3.95 minutes; column: Kinetex EVO C18 2.6 µm, 2.1 x 150 mm, 100 Å; mobile phase: A = 0.025% TFA in H₂O, B = 0.025% TFA in MeCN; temperature: 60°C; gradient (%B conc): 20-60% over 7.15 minutes, then 60-95% over 0.3 minutes, and then 95-95% for 1.55 minutes; flow rate: 0.5 mL/min.
ESI-MS (⁺) measured value m/z = 909.45 (M+3H)³⁺

### Industrial Applicability

This invention can be used in pharmaceutical industries.

## Claims

1. A peptide, or a pharmaceutically acceptable salt thereof, consisting of an amino acid sequence set forth in a following amino acid sequence:
Ala-Val-MeF3C-Val-W7N-Asn-3Py6NH2-F4OMe-Ile-Ile-Arg-Arg-4Py-MeTyr-Cys (SEQ ID NO: 1); or
a peptide, or a pharmaceutically acceptable salt thereof, consisting of the amino acid sequence set forth in SEQ ID NO: 1 which includes one or more substitutions selected from following groups:
(I) substitution of valine residue at position 2 of SEQ ID NO: 1 for a polar amino acid;
(II) substitution of MeF3C residue at position 3 of SEQ ID NO: 1 for an N-methyl amino acid having an aromatic ring or a heterocyclic ring in a side chain;
(III) substitution of W7N residue at position 5 of SEQ ID NO: 1 for tryptophan optionally having a substituent, in which a C on the indole ring is optionally substituted for N, or N-methyltryptophan;
(IV) substitution of asparagine residue at position 6 of SEQ ID NO: 1 for Nmm;
(V) substitution of 3Py6NH2 residue at position 7 of SEQ ID NO: 1 for an amino acid having in a side chain an aromatic ring or a heterocyclic ring optionally having a substituent;
(VI) substitution of F4OMe residue at position 8 of SEQ ID NO: 1 for an amino acid having in a side chain an aromatic ring or a heterocyclic ring optionally having a substituent;
(VII) substitution of isoleucine residue at position 9 of SEQ ID NO: 1 for Eva;
(VIII) substitution of isoleucine residue at position 10 of SEQ ID NO: 1 for any amino acid;
(IX) substitution of arginine residue at position 11 of SEQ ID NO: 1 for lysine optionally having a substituent in a side chain or glutamine optionally having a substituent in a side chain;
(X) substitution of the arginine residue at position 12 of SEQ ID NO: 1 for lysine, histidine, or glutamine optionally having a substituent in a side chain.
(XI) substitution of the 4Py residue at position 13 of SEQ ID NO: 1 for an amino acid having in a side chain an aromatic ring or a heterocyclic ring optionally having a substituent, or glutamine optionally having a substituent in a side chain; and
(XII) substitution of MeTyr residue at position 14 of SEQ ID NO: 1 for an N-methyl amino acid having in a side chain an aromatic ring or a heterocyclic ring optionally having a substituent.

2. The peptide according to claim 1, or a pharmaceutically acceptable salt thereof, which is:
a peptide, or a pharmaceutically acceptable salt thereof, consisting of the amino acid sequence set forth in SEQ ID NO: 1; or
a peptide, or a pharmaceutically acceptable salt thereof, consisting of the amino acid sequence set forth in SEQ ID NO: 1 which includes one or more substitutions selected from following groups:
(I) substitution of valine residue at position 2 of SEQ ID NO: 1 for one amino acid selected from a group consisting of T and Kmor;
(II) substitution of MeF3C residue at position 3 of SEQ ID NO: 1 for one amino acid selected from a group consisting of MeF and Me3Py;
(III) substitution of W7N residue at position 5 of SEQ ID NO: 1 for one amino acid selected from a group consisting of W1aa, W, W1EtOH, W1Me, 3Imp, and MeW;
(IV) substitution of 3Py6NH2 residue at position 7 of SEQ ID NO: 1 for one amino acid selected from a group consisting of Y, Y26dF, 3Py6NHaa, Y3Me, and R;
(V) substitution of F4OMe residue at position 8 of SEQ ID NO: 1 for one amino acid selected from a group consisting of F4aao, F4aao (Glucamine), F4aao (pipzaa), YaeCOpipzaa, 3Py6OMe, 3Py6NHaa, 3Imp, Y, and F4aao (PEG8Me);
(VI) substitution of isoleucine residue at position 10 of SEQ ID NO: 1 for one amino acid selected from a group consisting of Har, Kmor, T, Mor, Hpr, and S;
(VII) substitution of arginine residue at position 11 of SEQ ID NO: 1 for one amino acid selected from a group consisting of Kmor, KCOpipzaa, K, E (Glucamine), Q, a Q optionally having a linker in a side chain, and E and E (apa) optionally having a linker in a side chain;
(VIII) substitution of arginine residue at position 12 of SEQ ID NO: 1 for one amino acid selected from a group consisting of Kmor, KCOpipzaa, Q, and H;
(IX) substitution of 4Py residue at position 13 of SEQ ID NO: 1 for one amino acid selected from a group consisting of F3COO, F, F3CONPEG4c, Y3Me, 3Py6Me, 3Py6OMe, 3Py6NH2, 4Py2Me, 4Py2OMe, 4Py2NH2, 3Py, Y, F3COO optionally having a linker, and E optionally having a linker in a side chain; and
(X) substitution of MeTyr residue at position 14 of SEQ ID NO: 1 for one amino acid selected from a group consisting of MeF4COO, MeF3COO, MeF4C, MeF4OMe, MeF3C, MeF3OMe, Me4Py, Me3Py, and MeF4COO (PEG4c).

3. The peptide according to claim 1 or 2, or a pharmaceutically acceptable salt thereof, consisting of an amino acid sequence that satisfies at least one of:
(I) a residue at position 1 of SEQ ID NO: 1 is an alanine residue;
(II) a residue at position 4 of SEQ ID NO: 1 is a valine residue;
(III) a residue at position 6 of SEQ ID NO: 1 is an asparagine residue;
(IV) a residue at position 7 of SEQ ID NO: 1 is a tyrosine residue or a 3Py6NH2 residue; and
(V) a residue at position 9 of SEQ ID NO: 1 is an isoleucine residue.

4. The peptide according to claim 3, or a pharmaceutically acceptable salt thereof, consisting of at least either:
A: an amino acid sequence that satisfies at least two of:
(a) a residue at position 5 of SEQ ID NO: 1 is a W7N residue or a 3Imp residue;
(b) a residue at position 7 of SEQ ID NO: 1 is a 3Py6NH2 residue;
(c) a residue at position 8 of SEQ ID NO: 1 is a F4aao (pipzaa) residue; and
(d) a residue at position 13 of SEQ ID NO: 1 is a 4Py residue,
or
B: an amino acid sequence in which (i) a residue at position 8 of SEQ ID NO: 1 is an F4aao(pipzaa) residue, an F4aao (Glucamine) residue, an F4aao (PEG8Me) residue, or a YaeCOpipzaa residue.

5. The peptide according to claim 1, or a pharmaceutically acceptable salt thereof, consisting of an amino acid sequence set forth in any of SEQ ID Nos: 1 to 202 or a conjugate of an amino acid sequence set forth in any of SEQ ID Nos: 1 to 202 and a linker, wherein an amino acid sequence site of the amino acid sequence of from residues 1 to 15 has a cyclic structure.

6. The peptide according to claim 1, or a pharmaceutically acceptable salt thereof, which is a cyclic peptide.

7. The peptide according to claim 3, or a pharmaceutically acceptable salt thereof, which has an hTfR binding activity.

8. The peptide according to claim 4, or a pharmaceutically acceptable salt thereof, which has an hTfR binding activity in a pH-dependent manner.

9. The peptide according to claim 1, or a pharmaceutically acceptable salt thereof, wherein a linker is bound to an amino acid at positions 8, 11, 13, or 15 of SEQ ID NO: 1.

10. The peptide according to claim 9, or a pharmaceutically acceptable salt thereof, which is set forth in any of SEQ ID Nos: 1 to 167.

11. The peptide according to claim 9, or a pharmaceutically acceptable salt thereof, wherein the linker is a polyethylene glycol (PEG) linker or a GKN3 linker.

12. A composite comprising the peptide according to claim 9 or a pharmaceutically acceptable salt thereof and a substance bound to the linker.

13. The composite according to claim 12, wherein the linker is a polyethylene glycol (PEG) linker or a GKN3 linker.

14. A composition comprising the peptide according to claim 1 or a pharmaceutically acceptable salt thereof.

15. A pharmaceutical or diagnostic composition comprising the peptide according to claim 1 or a pharmaceutically acceptable salt thereof.

16. A composition comprising the composite according to claim 12 or 13.

17. A pharmaceutical or diagnostic composition comprising the composite according to claim 12 or 13.

18. A method for producing a pharmaceutical or diagnostic composition, comprising a step of obtaining the composite of claim 12 or 13.

19. The method according to claim 18, wherein the linker is a polyethylene glycol (PEG) linker or a GKN3 linker.

20. A method of testing a peptide, or a pharmaceutically acceptable salt thereof, comprising testing the peptide or pharmaceutically acceptable salt thereof for at least one of:
a) solubility in a solvent;
b) binding ability to hTfR;
c) toxicity to a cell and/or a tissue; and
d) toxicity to an experimental animal,
wherein the peptide or pharmaceutically acceptable salt thereof has an amino acid sequence in which from 1 to 3 amino acid residues have been deleted, substituted, inserted and/or added in the amino acid sequence of the peptide according to claim 1 or a pharmaceutically acceptable salt thereof.
